# EUROPEAN PATENT SPECIFICATION

(11) **EP 4 049 325 B1**
(45) Date of publication and mention of the grant of the patent: **05.08.2026**
(21) Application number: 20790321.2
(22) Date of filing: 20.10.2020
(51) Int. Cl.: H10K 50/11, H10K 85/60

(54) **MATERIALS FOR ORGANIC ELECTROLUMINESCENT DEVICES**
MATERIALIEN FÜR ORGANISCHE ELEKTROLUMINESZENTE VORRICHTUNGEN
MATÉRIAUX POUR DISPOSITIFS ÉLECTROLUMINESCENTS ORGANIQUES

(30) Priority: 22.10.2019 EP 19204493
(43) Date of publication of application: 31.08.2022
(73) Proprietor: UDC Ireland Limited, Ballycoolin, Dublin 15 (IE)
(72) Inventor: PARHAM, Amir, 60486 Frankfurt am Main (DE); EHRENREICH, Christian, 64285 Darmstadt (DE); ENGELHART, Jens, 64285 Darmstadt (DE)
(74) Representative: Maiwald GmbH
(86) International application number: PCT/EP2020/079444
(87) International publication number: WO 2021/078710

(56) References cited:
- CN-A- 108 148 016
- KR-B1- 101 834 228
- YAN-NA MA ET AL: "Synthesis of dibenzothiazines from sulfides by one-pot N , O -transfer and intramolecular C-H amination", GREEN CHEMISTRY, vol. 20, no. 13, 14 May 2018 (2018-05-14), pages 2953 - 2958, XP055761299, ISSN: 1463-9262, DOI: 10.1039/C8GC01057A

## Description

The present invention relates to organic electroluminescent devices comprising benzothiazine derivatives, and to specific benzothiazine derivatives for use in electronic devices, in particular in organic electroluminescent devices.

The structure of organic electroluminescent devices (OLEDs) in which organic semiconductors are employed as functional materials is described, for example in US 4539507. The emitting materials employed here are very often organometallic complexes which exhibit phosphorescence. For quantum-mechanical reasons, an up to four-fold increase in efficiency is possible using phosphorescent instead of fluorescent emitters. In general, however, there is still a need for improvement in the case of OLEDs, in particular also in the case of OLEDs which exhibit triplet emission (phosphorescence), for example with respect to efficiency, operating voltage and lifetime.

The properties of phosphorescent OLEDs are not only determined by the triplet emitters but also by the other materials used together with triplet emitters in OLEDs, such as matrix materials, also called host materials. Improvements in these materials and their charge-transport properties can thus also result in significant improvements in the OLED properties.

Thus, the choice of the matrix material in an emission layer comprising a phosphorescent emitter has a great influence on OLEDs properties, especially in terms of efficiency. The matrix material limits the quenching of excited states of emitter molecules by energy transfer.

The object of the present invention is the provision of OLEDs and materials, which are suitable for use in an OLED. More particularly, the object of the present invention is the provision of compounds, which are particularly suitable as matrix material for phosphorescent emitters in an OLED, but also as hole-transport material (HTM), electron-blocking material (EBM), electron-transport material (ETM), hole-blocking material (HBM) depending on the specific structure and radicals present in the compound. A further object of the present invention is to provide further organic semiconductors for organic electroluminescent devices to provide the person skilled in the art with a greater possible choice of materials for the production of OLEDs.

Surprisingly, it has now been found that OLEDs comprising dibenzothiazine derivatives, as described in greater detail below, exhibit excellent properties, particularly when the dibenzothiazine derivatives are employed as matrix material for red phosphorescent emitters. Indeed, these compounds lead to OLEDs exhibiting very good properties in terms of lifetime and/or efficiency and/or electroluminescent emission. The present invention therefore relates to OLEDs comprising dibenzothiazine derivatives, to specific dibenzothiazine derivatives and to electronic devices, in particular organic electroluminescent devices, which comprise these specific dibenzothiazine derivatives. The present invention also relates to mixtures and formulations comprising these compounds. Dibenzothiazine derivatives and their synthesis are known from the prior art (for example in Ma et. al, J. Org. Chem. 2019, 84, 450-457, Hanchate et al., J. Org. Chem. 2019, 84, 8248-8255, KR10-1834228 or Yongpruksa et al., Org. Biomol.

Chem., 2011, 9,7979). Dibenzothiazine derivatives and their application in a charge transport layer of an organic light emitting device is disclosed document CN 108 148 016 A.

The present invention therefore relates to an organic electroluminescent device comprising an anode, a cathode and at least one organic layer between the cathode and the anode, characterized in that the at least one organic layer comprises a compound of the formula (1), 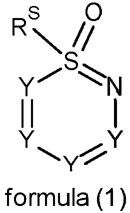 where the following applies to the symbols and indices used:
Y stands for CR^{Y} or N; with the proviso that at least two adjacent groups Y form a condensed aromatic or heteroaromatic ring system with the benzothiazine ring of formula (1);
R^{S}, R^{Y} stand on each occurrence, identically or differently, for H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R, where in each case one or more non-adjacent CH₂ groups may be replaced by RC=CR, C=C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 60 aromatic ring atoms, which may be substituted by one or more radicals R;
   - where the radical R^{S} may form an aliphatic, aromatic or heteroaromatic ring system ring with a radical R^{Y} or with the adjacent ring formed by two groups Y, which may be substituted by one or more radicals R; and
   - where two adjacent radicals R^{Y} may form an aliphatic, aromatic or heteroaromatic ring system together, which may be substituted by one or more radicals R;
R stands on each occurrence, identically or differently, for H, D, F, Cl, Br, I, CHO, CN, C(=O)Ar, P(=O)(Ar)₂, S(=O)Ar, S(=O)₂Ar, N(R')₂, N(Ar)₂, NO₂, Si(R')₃, B(OR')₂, OSO₂R', a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R', where in each case one or more non-adjacent CH₂ groups may be replaced by R'C=CR', C=C, Si(R')₂, Ge(R')₂, Sn(R')₂, C=O, C=S, C=Se, P(=O)(R'), SO, SO₂, O, S or CONR' and where one or more H atoms may be replaced by D, F, Cl, Br, I, CN or NO₂, an aromatic or heteroaromatic ring systems having 5 to 60 aromatic ring atoms, which may in each case be substituted by one or more radicals R', or an aryloxy group having 5 to 60 aromatic ring atoms, which may be substituted by one or more radicals R'; where two adjacent substituents R may form an aliphatic or aromatic ring system together, which may be substituted by one or more radicals R';
Ar is, on each occurrence, identically or differently, an aromatic or heteroaromatic ring system having 5 to 60 aromatic ring atoms, which may in each case also be substituted by one or more radicals R'; and
R' stands on each occurrence, identically or differently, for H, D, F, Cl, Br, I, CN, a straight-chain alkyl, alkoxy or thioalkyl group having 1 to 20 C atoms or abranched or cyclic alkyl, alkoxy or thioalkyl group having 3 to 20 C atoms, where in each case one or more non-adjacent CH₂ groups may be replaced by SO, SO₂, O, S and where one or more H atoms may be replaced by D, F, Cl, Br or I, or an aromatic or heteroaromatic ring system having 5 to 24 aromatic ring atoms.
Furthermore, the following definitions of chemical groups apply for the purposes of the present application:
   Adjacent radicals in the sense of the present invention are radicals which are bonded to atoms which are linked directly to one another or which are bonded to the same atom.

An aryl group in the sense of this invention contains 6 to 60 aromatic ring atoms; a heteroaryl group in the sense of this invention contains 5 to 60 aromatic ring atoms, at least one of which is a heteroatom. The hetero atoms are preferably selected from N, O and S. This represents the basic definition. If other preferences are indicated in the description of the present invention, for example with respect to the number of aromatic ring atoms or the heteroatoms present, these apply.

An aryl group or heteroaryl group here is taken to mean either a simple aromatic ring, i.e. benzene, or a simple heteroaromatic ring, for example pyridine, pyrimidine or thiophene, or a condensed (annellated) aromatic or heteroaromatic polycycle, for example naphthalene, phenanthrene, quinoline or carbazole. A condensed (annellated) aromatic or heteroaromatic polycycle in the sense of the present application consists of two or more simple aromatic or heteroaromatic rings condensed with one another.

An aryl or heteroaryl group, which may in each case be substituted by the above-mentioned radicals and which may be linked to the aromatic or heteroaromatic ring system via any desired positions, is taken to mean, in particular, groups derived from benzene, naphthalene, anthracene, phenanthrene, pyrene, dihydropyrene, chrysene, perylene, fluoranthene, benzanthracene, benzophenanthrene, tetracene, pentacene, benzopyrene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, pyrazine, phenazine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

An aryloxy group in accordance with the definition of the present invention is taken to mean an aryl group, as defined above, which is bonded via an oxygen atom. An analogous definition applies to heteroaryloxy groups.

An aromatic ring system in the sense of this invention contains 6 to 60 C atoms in the ring system. A heteroaromatic ring system in the sense of this invention contains 5 to 60 aromatic ring atoms, at least one of which is a heteroatom. The heteroatoms are preferably selected from N, O and/or S. An aromatic or heteroaromatic ring system in the sense of this invention is intended to be taken to mean a system which does not necessarily contain only aryl or heteroaryl groups, but instead in which, in addition, a plurality of aryl or heteroaryl groups may be connected by a non-aromatic unit (preferably less than 10% of the atoms other than H), such as, for example, an sp³-hybridised C, Si, N or O atom, an sp²-hybridised C or N atom or an sp-hybridised C atom. Thus, for example, systems such as 9,9'-spirobifluorene, 9,9'-diarylfluorene, triarylamine, diaryl ether, stilbene, etc., are also intended to be taken to be aromatic ring systems in the sense of this invention, as are systems in which two or more aryl groups are connected, for example, by a linear or cyclic alkyl, alkenyl or alkynyl group or by a silyl group. Furthermore, systems in which two or more aryl or heteroaryl groups are linked to one another via single bonds are also taken to be aromatic or heteroaromatic ring systems in the sense of this invention, such as, for example, systems such as biphenyl, terphenyl or diphenyltriazine.

An aromatic or heteroaromatic ring system having 5 - 60 aromatic ring atoms, which may in each case also be substituted by radicals as defined above and which may be linked to the aromatic or heteroaromatic group via any desired positions, is taken to mean, in particular, groups derived from benzene, naphthalene, anthracene, benzanthracene, phenanthrene, benzophenanthrene, pyrene, chrysene, perylene, fluoranthene, naphthacene, pentacene, benzopyrene, biphenyl, biphenylene, terphenyl, terphenylene, quaterphenyl, fluorene, spirobifluorene, dihydrophenanthrene, dihydropyrene, tetrahydropyrene, cis- or trans-indenofluorene, truxene, isotruxene, spiro-truxene, spiroisotruxene, furan, benzofuran, isobenzofuran, dibenzofuran, thiophene, benzothiophene, isobenzothiophene, dibenzothiophene, pyrrole, indole, isoindole, carbazole, indolocarbazole, indenocarbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, 1,5-diazaanthracene, 2,7-diazapyrene, 2,3-diazapyrene, 1,6-diazapyrene, 1,8-diazapyrene, 4,5-diazapyrene, 4,5,9,10-tetraazaperylene, pyrazine, phenazine, phenoxazine, phenothiazine, fluorubin, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole, or combinations of these groups.

For the purposes of the present invention, a straight-chain alkyl group having 1 to 40 C atoms or a branched or cyclic alkyl group having 3 to 40 C atoms or an alkenyl or alkynyl group having 2 to 40 C atoms, in which, in addition, individual H atoms or CH₂ groups may be substituted by the groups mentioned above under the definition of the radicals, is preferably taken to mean the radicals methyl, ethyl, n-propyl, i-propyl, n-butyl, i-butyl, s-butyl, t-butyl, 2-methylbutyl, n-pentyl, s-pentyl, cyclopentyl, neopentyl, n-hexyl, cyclohexyl, neohexyl, n-heptyl, cycloheptyl, n-octyl, cyclooctyl, 2-ethylhexyl, trifluoromethyl, pentafluoroethyl, 2,2,2-trifluoroethyl, ethenyl, propenyl, butenyl, pentenyl, cyclopentenyl, hexenyl, cyclohexenyl, heptenyl, cycloheptenyl, octenyl, cyclooctenyl, ethynyl, propynyl, butynyl, pentynyl, hexynyl or octynyl. An alkoxy or thioalkyl group having 1 to 40 C atoms is preferably taken to mean methoxy, trifluoro-methoxy, ethoxy, n-propoxy, i-propoxy, n-butoxy, i-butoxy, s-butoxy, t-butoxy, n-pentoxy, s-pentoxy, 2-methylbutoxy, n-hexoxy, cyclohexyloxy, n-heptoxy, cycloheptyloxy, n-octyloxy, cyclooctyloxy, 2-ethylhexyloxy, pentafluoroethoxy, 2,2,2-trifluoroethoxy, methylthio, ethylthio, n-propylthio, i-propylthio, n-butylthio, i-butylthio, s-butylthio, t-butylthio, n-pentylthio, s-pentylthio, n-hexylthio, cyclohexylthio, n-heptylthio, cycloheptylthio, n-octylthio, cyclooctylthio, 2-ethyl-hexylthio, trifluoromethylthio, pentafluoroethylthio, 2,2,2-trifluoroethylthio, ethenylthio, propenylthio, butenylthio, pentenylthio, cyclopentenylthio, hexenylthio, cyclohexenylthio, heptenylthio, cycloheptenylthio, octenylthio, cyclooctenylthio, ethynylthio, propynylthio, butynylthio, pentynylthio, hexynylthio, heptynylthio or octynylthio.

The formulation that two radicals may form a ring with one another is, for the purposes of the present application, intended to be taken to mean, inter alia, that the two radicals are linked to one another by a chemical bond. This is illustrated by the following schemes:

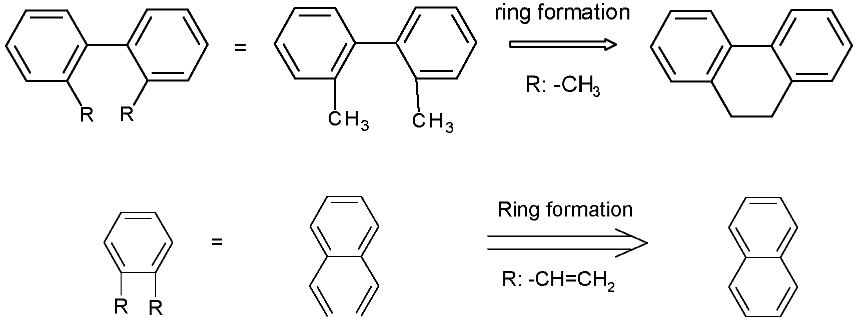

Furthermore, the above-mentioned formulation is also intended to be taken to mean that, in the case where one of the two radicals represents hydrogen, the second radical is bonded at the position to which the hydrogen atom was bonded, with formation of a ring. This is illustrated by the following scheme:

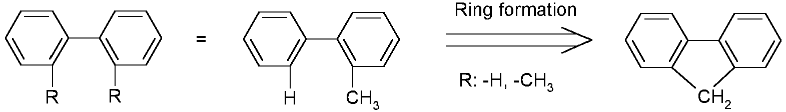

In accordance with a preferred embodiment, the compound of formula (1) is selected from the compounds of formulae (2), (3) and (4); 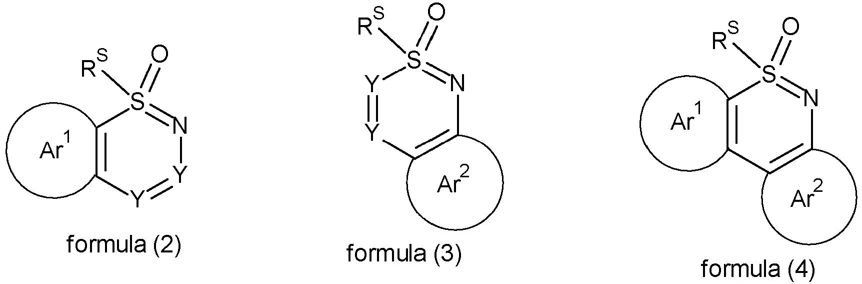 where the rings Ar¹ and Ar² are aromatic or heteroaromatic ring systems condensed with the benzothiazine ring, which have 5 to 30 aromatic ring atoms and which may be substituted by at least one radical R; and where the symbols Y and R^{S} have the same meaning as above.

Preferably, the rings Ar¹ and Ar² are selected from the rings of the formula (Ar-1) or (Ar-2), 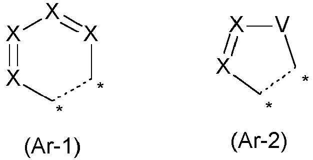 where the dashed bonds between the signs "*" correspond to the common bond of the group of formula (Ar-1) or (Ar-2) with the benzothiazine ring, and where:
V stands for C(R⁰)₂, NR^{N}, O or S; preferably for NR^{N}, O or S, more preferably for NR^{N};
X stands, identically or differently on each occurrence, for CR^{X} or N; or two adjacent groups X stand for a group of the formula (Ar-3) or (Ar-4), 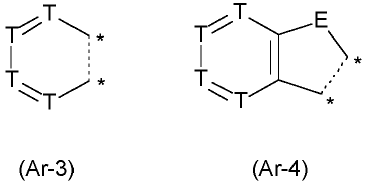 where the dashed bonds between the signs "*" correspond to the common bond of the group of formula (Ar-3) or (Ar-4) with the group of formulae (Ar-1) or (Ar-2), and furthermore:
   - E stands for C(R⁰)₂, C=O, NR^{N}, O or S; preferably for NR^{N}, O or S, more preferably for NR^{N};
   - T stands, identically or differently on each occurrence, for CR^{T} or N;
   - R^{X}, R^{T} stand on each occurrence, identically or differently, for H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R, where in each case one or more non-adjacent CH₂ groups may be replaced by RC=CR, C=C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 60 aromatic ring atoms, which may be substituted by one or more radicals R; and where two adjacent substituents R^{T} may form an aliphatic, aromatic or heteroaromatic ring system together, which may be substituted by one or more radicals R; and where two adjacent substituents R^{X} may form an aliphatic, aromatic or heteroaromatic ring system together, which may be substituted by one or more radicals R;
R⁰, R^{N} are on each occurrence, identically or differently, for H, D, F, Cl, Br, I, CN, Si(R)₃, a straight-chain alkyl, alkoxy or thioalkyl group having 1 to 40, preferably 1 to 20, more preferably 1 to 10 C atoms or a branched or a cyclic alkyl, alkoxy or thioalkyl group having 3 to 40, preferably 3 to 20, more preferably 3 to 10 C atoms, each of which may be substituted by one or more radicals R, where in each case one or more non-adjacent CH₂ groups may be replaced by RC=CR, C=C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60, preferably 5 to 40, more preferably 5 to 30, particularly preferably 5 to 24, very particularly preferably 5 to 18 aromatic ring atoms, which may in each case be substituted by one or more radicals R, or an aryloxy group having 5 to 60, preferably 5 to 40, more preferably 5 to 30, particularly preferably 5 to 24, very particularly preferably 5 to 18 aromatic ring atoms, which may be substituted by one or more radicals R, where two adjacent substituents R⁰ may form an aliphatic, aromatic or heteroaromatic ring system together, which may be substituted by one or more radicals R.

More preferably, R⁰, R^{N} are on each occurrence, identically or differently, for H, D, F, a straight-chain alkyl or alkoxy group having 1 to 20, more preferably 1 to 10 C atoms or branched or a cyclic alkyl or alkoxy group having 3 to 20, more preferably 3 to 10 C atoms, each of which may be substituted by one or more radicals R, where in each case one or more non-adjacent CH₂ groups may be replaced by RC=CR, C=C, O or S, and where one or more H atoms may be replaced by D, F, Cl, Br, I, CN or NO₂, an aromatic or heteroaromatic ring systems having 5 to 60, preferably 5 to 40, more preferably 5 to 30, particularly preferably 5 to 24, very particularly preferably 5 to 18 aromatic ring atoms, which may in each case be substituted by one or more radicals R, where two adjacent substituents R⁰ may form an aliphatic, aromatic or heteroaromatic ring system together, which may be substituted by one or more radicals R.

Preferably, in formula (2), the ring Ar¹ corresponds to a ring of formula (Ar-1).

Preferably, in formula (3), the ring Ar² corresponds to a ring of formula (Ar-1).

Preferably, in formula (4), one ring of the rings Ar¹ and Ar² is a ring of formula (Ar-1), and the other ring is either a ring of formula (Ar-1) or a ring of formula (Ar-2).

In accordance with a very preferred embodiment, the compound of formula (1) is selected from the compounds of formulae (2-1) to (4-5) as depicted below: 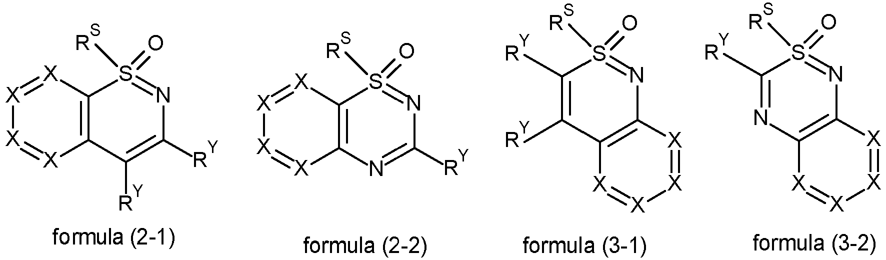 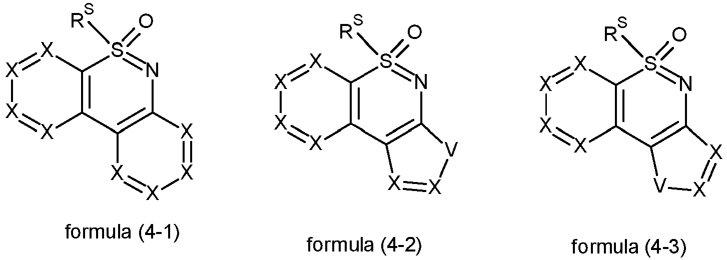 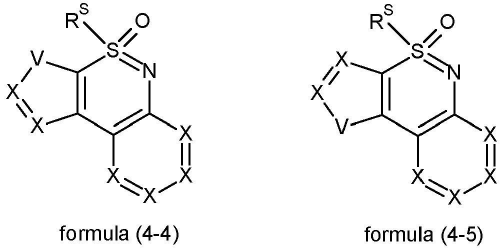 where the symbols X, R^{S}, R^{Y} and V have the same meaning as above.

In accordance with a particularly preferred embodiment the compound of formula (1) is selected from the compounds of formulae (2-1-1) to (4-5-3) as depicted below: 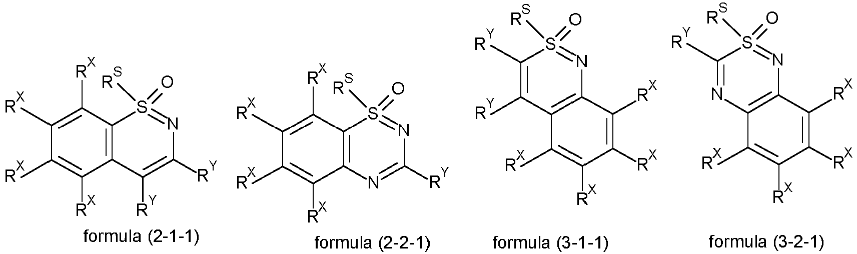 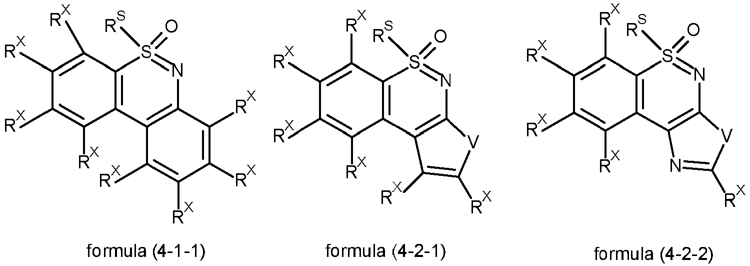 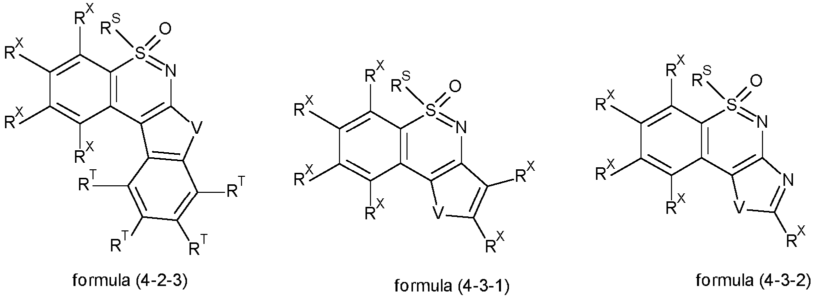 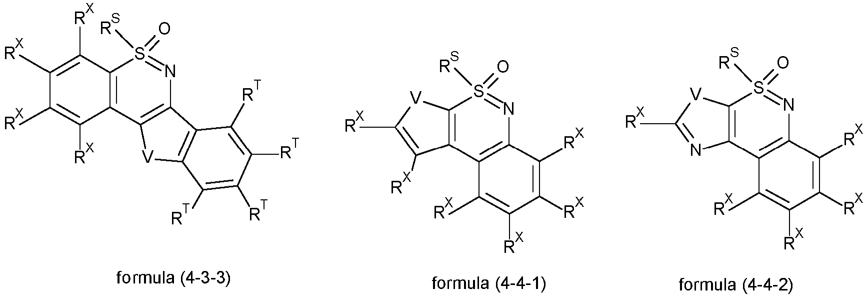 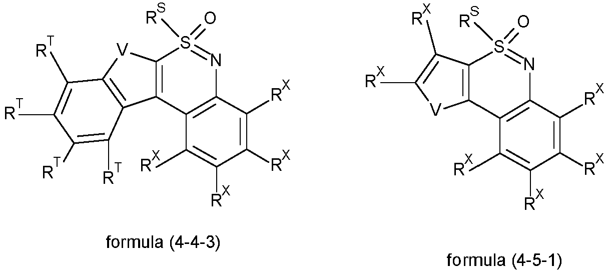 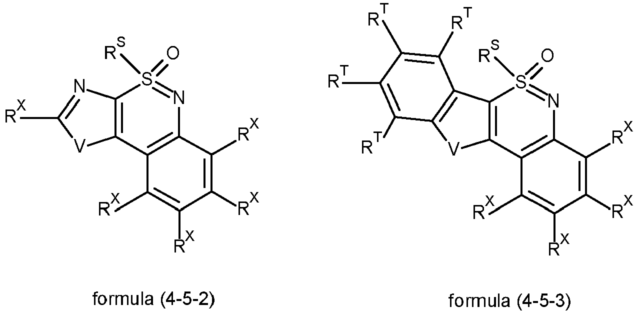 where the symbols R^{X}, R^{Y}, R^{S}, R^{T} and V have the same meaning as above.

More preferably, R^{S} stands on each occurrence, identically or differently, for H, D, F, Cl, Br, I, a straight-chain alkyl, alkoxy or thioalkyl group having 1 to 40, preferably 1 to 20, more preferably 1 to 10 C atoms or branched or a cyclic alkyl, alkoxy or thioalkyl group having 3 to 40, preferably 3 to 20, more preferably 3 to 10 C atoms, each of which may be substituted by one or more radicals R, where in each case one or more non-adjacent CH₂ groups may be replaced by RC=CR, C=C, O or S, and where one or more H atoms may be replaced by D, F, Cl, Br, I, CN or NO₂, an aromatic or heteroaromatic ring systems having 5 to 60, preferably 5 to 40, more preferably 5 to 30, particularly preferably 5 to 24, very particularly preferably 5 to 18 aromatic ring atoms, which may in each case be substituted by one or more radicals R. Even more preferably, R^{S} stands on each occurrence, identically or differently, for a straight-chain alkyl group having 1 to 20, more preferably 1 to 10 C atoms or branched or a cyclic alkyl group having 3 to 20, more preferably 3 to 10 C atoms, each of which may be substituted by one or more radicals R, or an aromatic or heteroaromatic ring systems having 5 to 40, more preferably 5 to 30, particularly preferably 5 to 24, very particularly preferably 5 to 18 aromatic ring atoms, which may in each case be substituted by one or more radicals R. Particularly preferably, R^{S} stands on each occurrence, identically or differently, for an aromatic or heteroaromatic ring systems having 5 to 40, more preferably 5 to 30, particularly preferably 5 to 24, very particularly preferably 5 to 18 aromatic ring atoms, which may in each case be substituted by one or more radicals R.

More preferably, R^{X}, R^{Y} and R^{T} stand on each occurrence, identically or differently, for H, D, F, Cl, Br, I, CN, N(R)₂, N(Ar)₂, a straight-chain alkyl, alkoxy or thioalkyl group having 1 to 40, preferably 1 to 20, more preferably 1 to 10 C atoms or a branched or a cyclic alkyl, alkoxy or thioalkyl group having 3 to 40, preferably 3 to 20, more preferably 3 to 10 C atoms, each of which may be substituted by one or more radicals R, where in each case one or more non-adjacent CH₂ groups may be replaced by RC=CR, C=C, O or S, and where one or more H atoms may be replaced by D, F, Cl, Br, I, CN or NO₂, an aromatic or heteroaromatic ring systems having 5 to 60, preferably 5 to 40, more preferably 5 to 30, particularly preferably 5 to 24, very particularly preferably 5 to 18 aromatic ring atoms, which may in each case be substituted by one or more radicals R;
- where two adjacent radicals R^{X} may form an aliphatic, aromatic or heteroaromatic ring system together, which may be substituted by one or more radicals R;
- where two adjacent radicals R^{T} may form an aliphatic, aromatic or heteroaromatic ring system together, which may be substituted by one or more radicals R.

Even more preferably, R^{X}, R^{Y} and R^{T} stand on each occurrence, identically or differently, for H, a straight-chain alkyl or alkoxy group having 1 to 20, more preferably 1 to 10 C atoms or a branched or a cyclic alkyl or alkoxy group having 3 to 20, more preferably 3 to 10 C atoms, each of which may be substituted by one or more radicals R, or an aromatic or heteroaromatic ring systems having 5 to 60, preferably 5 to 40, more preferably 5 to 30, particularly preferably 5 to 24, very particularly preferably 5 to 18 aromatic ring atoms, which may in each case be substituted by one or more radicals R.

In accordance with a preferred embodiment, the compound of formula (1) comprises at least one radical, preferably one radical R^{S}, R^{Y} or R, more preferably one radical R^{Y} or R, which corresponds to an aromatic or heteroaromatic ring system having 5 to 60 aromatic ring atoms, which may in each case be substituted by one or more radicals R. More preferably, the compounds of formula (1) comprise at least one radical, preferably one radical R^{S}, R^{Y} or R, more preferably one radical R^{Y} or R, which stands for an aromatic or heteroaromatic ring system having 5 to 60 aromatic ring atoms selected from the groups of formula (R-1), 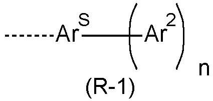 where
Ar^{S} stands for a single bond or for an aromatic or heteroaromatic ring system having 5 to 30 aromatic ring atoms, which may be substituted by one or more radicals R; and
Ar² stands for phenyl, biphenyl, terphenyl, quaterphenyl, fluorene, spirobifluorene, naphthalene, anthracene, phenanthrene, triphenylene, fluoranthene, indole, benzofuran, benzothiophene, dibenzofuran, dibenzothiophene, carbazole, indenocarbazole, indolocarbazole, phenanthroline, pyridine, pyrimidine, pyrazine, pyridazine, triazine, quinolone, benzopyridine, benzopyridazine, benzopyrimidine, quinazoline, benzimidazole, or a combination of two or three of these groups, each of which may be substituted by one or more radicals R; and
n is an integer equal to 1, 2, 3 or 4, preferably 1 or 2, more preferably 1.

When Ar^{S} is a single bond, the group (R-1) corresponds to (R-1sb): 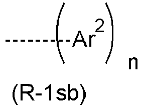 where Ar² and the index n have the same meaning as above.

More specifically, it is preferred that the compounds of formulae (2-1-1), (2-2-1), (3-1-1) and (3-2-1) comprise at least one radical R^{S}, R^{X}, R^{Y} or R, preferably R^{X}, R^{Y} or R, more preferably R^{X} or R^{Y}, which corresponds to an aromatic or heteroaromatic ring system having 5 to 60 aromatic ring atoms, which may in each case be substituted by one or more radicals R. More preferably, the compounds of formulae (2-1-1), (2-2-1), (3-1-1) and (3-2-1) comprise at least one radical R^{S}, R^{X}, R^{Y} or R, preferably R^{X}, R^{Y} or R, more preferably R^{X} or R^{Y}, which stands for an aromatic or heteroaromatic ring system having 5 to 60 aromatic ring atoms selected from the groups of formula (R-1) as defined above.

It is also preferred that the compounds of formulae (4-1-1), (4-2-1), (4-2-2), (4-3-1), (4-3-2), (4-4-1), (4-4-2), (4-5-1) and (4-5-2) comprise at least one radical R^{S}, R^{X} or R, preferably R^{X} or R, more preferably R^{X}, which corresponds to an aromatic or heteroaromatic ring system having 5 to 60 aromatic ring atoms, which may in each case be substituted by one or more radicals R. More preferably, the compounds of formulae (4-1-1), (4-2-1), (4-2-2), (4-3-1), (4-3-2), (4-4-1), (4-4-2), (4-5-1) and (4-5-2) comprise at least one radical R^{S}, R^{X} or R, preferably R^{X} or R, more preferably R^{X}, which stands for an aromatic or heteroaromatic ring system having 5 to 60 aromatic ring atoms selected from the groups of formula (R-1) as defined above.

It is also preferred that the compounds of formulae (4-2-3), (4-3-3), (4-4-3) and (4-5-3) comprise at least one radical R^{S}, R^{X}, R^{T} or R, preferably R^{X}, R^{T} or R, more preferably R^{X} or R^{T}, which corresponds to an aromatic or heteroaromatic ring system having 5 to 60 aromatic ring atoms, which may in each case be substituted by one or more radicals R. More preferably, the compounds of formulae (4-2-3), (4-3-3), (4-4-3) and (4-5-3) comprise at least one radical R^{S}, R^{X}, R^{T} or R, preferably R^{X}, R^{T} or R, more preferably R^{X} or R^{T}, which stands for an aromatic or heteroaromatic ring system having 5 to 60 aromatic ring atoms selected from the groups of formula (R-1) as defined above.

Preferably the group of formula (R-1) is selected from a group of formula (R-2), 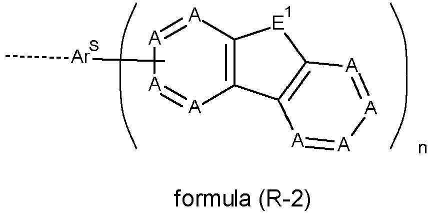 where the dashed bond indicates the bond to the structure,
where n has the same meaning as above, and where:
A stands, identically or differently on each occurrence, for CR^{A} or N; or two adjacent groups A stand for a group of the formula (Ar-5) or (Ar-6), 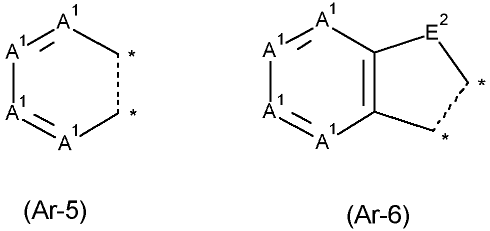
where the dashed bonds between the signs "*" correspond to the common bond of the group of formula (Ar-5) or (Ar-6) with the group of formula (R-2), and
where:
   E¹, E² stands for C(R⁰)₂, C=O, NR^{N}, O or S, where R⁰ and R^{N} have the same definition as above; it is preferred that E¹ stands for NR^{N};
   A¹ stands, identically or differently on each occurrence, for CR^{A1} or N; R^{A}, R^{A1} are on each occurrence, identically or differently, for H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R, where in each case one or more non-adjacent CH₂ groups may be replaced by RC=CR, C=C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 60 aromatic ring atoms, which may be substituted by one or more radicals R; where two adjacent substituents R^{A} may form an aliphatic, aromatic or heteroaromatic ring system together, which may be substituted by one or more radicals R and where two adjacent substituents R^{A1} may form an aliphatic, aromatic or heteroaromatic ring system together, which may be substituted by one or more radicals R.

When Ar^{S} is a single bond, the group (R-2) corresponds to the group (R-2sb), 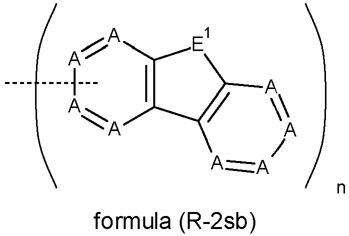 where the symbols have the same meaning as above.

In accordance with a preferred embodiment, the group Ar^{S} is a single bond.

In accordance with another preferred embodiment, the group Ar^{S} is an aromatic or heteroaromatic ring system having 5 to 24 aromatic ring atoms, which may in each case also be substituted by one or more radicals R. More preferably, the group Ar^{S} stands on each occurrence, identically or differently, for phenyl, biphenyl, fluorene, spirobifluorene, naphthalene, phenanthrene, anthracene, dibenzofuran, dibenzothiophene, carbazole, pyridine, pyrimidine, pyrazine, pyridazine, triazine, benzopyridine, benzopyridazine, benzopyrimidine and quinazoline, each of which may be substituted by one or more radicals R. Particularly preferably, the group Ar^{S} stands on each occurrence, identically or differently, for phenyl, biphenyl, fluorene, dibenzofuran, dibenzothiophene and carbazole, each of which may be substituted by one or more radicals R.

Examples of suitable groups Ar^{S}, when Ar^{S} is not a single bond, are the groups (Ar^{S}-1) to (Ar^{S}-22) depicted in the table below:

| | | |
|---|---|---|
| 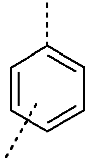 | 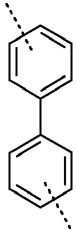 | 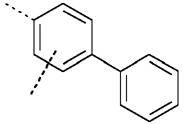 |
| Ar^{S}-1 | Ar^{S}-2 | Ar^{S}-3 |
| 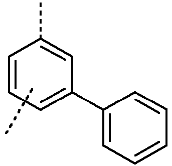 | 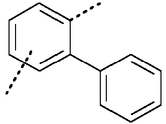 | 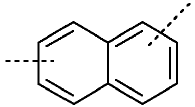 |
| Ar^{S}-4 | Ar^{S}-5 | Ar^{S}-6 |
| 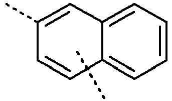 | 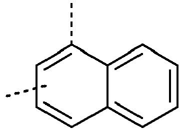 | 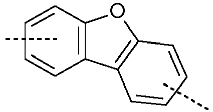 |
| Ar^{S}-7 | Ar^{S}-8 | Ar^{S}-9 |
| 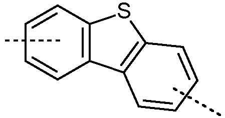 | 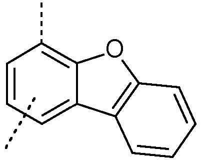 | 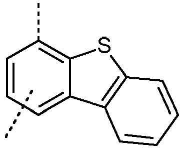 |
| Ar^{S}-10 | Ar^{S}-11 | Ar^{S}-12 |
| 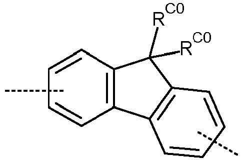 | 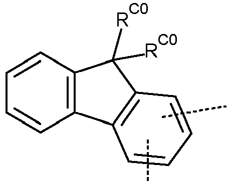 | 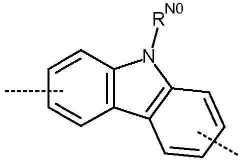 |
| Ar^{S}-13 | Ar^{S}-14 | Ar^{S}-15 |
| 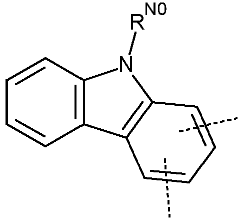 | 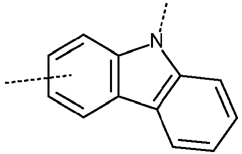 | 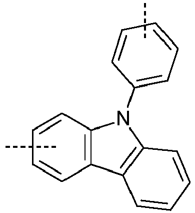 |
| Ar^{S}-16 | Ar^{S}-17 | Ar^{S}-18 |
| 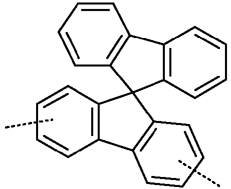 | 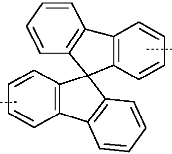 | 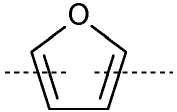 |
| Ar^{S}-19 | Ar^{S}-20 | Ar^{S}-21 |
| 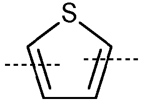 | | |
| Ar^{S}-22 | | |

where the dashed bonds indicate the bonds to the structure of formula (1), and where the groups (Ar^{S}-1) to (Ar^{S}-22) may be substituted at each free position by a radical R and where:
R^{N0}, R^{C0} are on each occurrence, identically or differently, H, D, F, Cl, Br, I, CN, a straight-chain alkyl, alkoxy or thioalkoxy group having 1 to 40, preferably 1 to 20, more preferably 1 to 10 C atoms or cyclic alkyl, alkoxy or thioalkoxy group having 3 to 40, preferably 3 to 20, more preferably 3 to 10 C atoms, each of which may be substituted by one or more radicals R, where one or more non-adjacent CH₂ groups may be replaced by (R)C=C(R), C=C, O or S and where one or more H atoms may be replaced by D, F, Cl, Br, I, CN or NO₂, or an aromatic or heteroaromatic ring system having 5 to 60, preferably 5 to 40, more preferably 5 to 30, very more preferably 5 to 18 aromatic ring atoms, which may in each case be substituted by one or more radicals R, where optionally two adjacent radicals R^{C0} can form a mono- or polycyclic, aliphatic, aromatic or heteroaromatic ring system with one another.

Examples of very suitable groups Ar^{S}, when Ar^{S} is not a single bond, are the groups (Ar^{S}-23) to (Ar^{S}-67) depicted in the table below:

| | | |
|---|---|---|
| 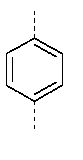 | 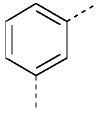 | 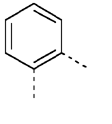 |
| Ar^{S}-23 | Ar^{S}-24 | Ar^{S}-25 |
| 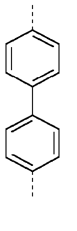 | 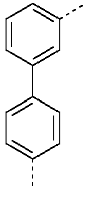 | 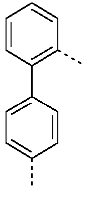 |
| Ar^{S}-26 | Ar^{S}-27 | Ar^{S}-28 |
| 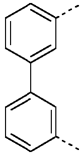 | 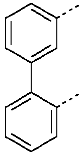 | 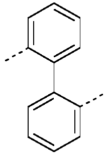 |
| Ar^{S}-29 | Ar^{S}-30 | Ar^{S}-31 |
| 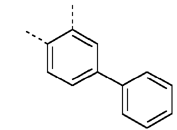 | 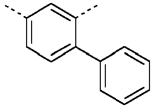 | 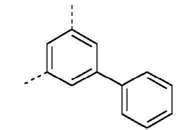 |
| Ar^{S}-32 | Ar^{S}-33 | Ar^{S}-34 |
| 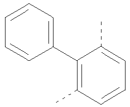 | 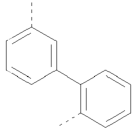 | 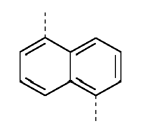 |
| Ar^{S}-35 | Ar^{S}-36 | Ar^{S}-37 |
| 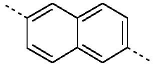 | 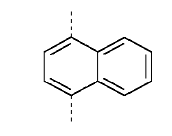 | 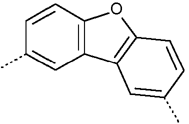 |
| Ar^{S}-38 | Ar^{S}-39 | Ar^{S}-40 |
| 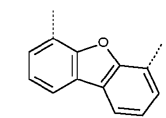 | 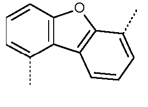 | 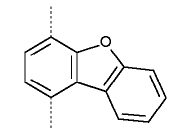 |
| Ar^{S}-41 | Ar^{S}-42 | Ar^{S}-43 |
| 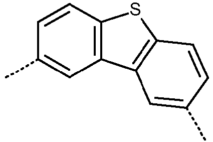 | 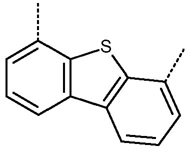 | 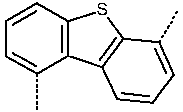 |
| Ar^{S}-44 | Ar^{S}-45 | Ar^{S}-46 |
| 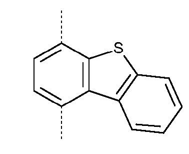 | 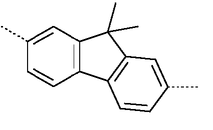 | 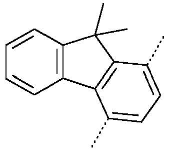 |
| Ar^{S}-47 | Ar^{S}-48 | Ar^{S}-49 |
| 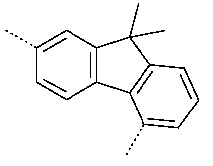 | 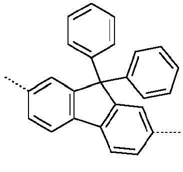 | 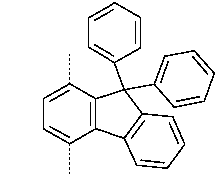 |
| Ar^{S}-50 | Ar^{S}-51 | Ar^{S}-52 |
| 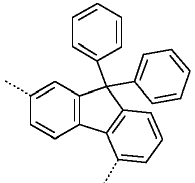 | 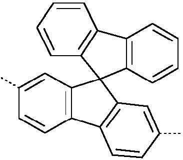 | 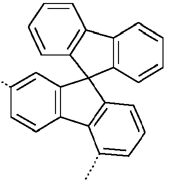 |
| Ar^{S}-53 | Ar^{S}-54 | Ar^{S}-55 |
| 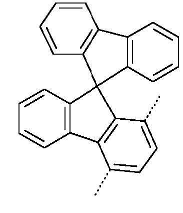 | 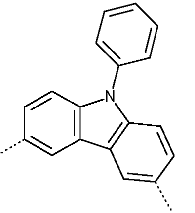 | 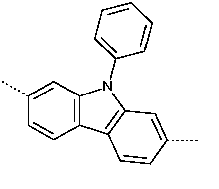 |
| Ar^{S}-56 | Ar^{S}-57 | Ar^{S}-58 |
| 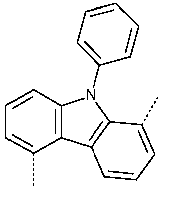 | 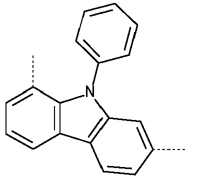 | 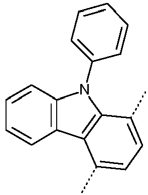 |
| Ar^{S}-59 | Ar^{S}-60 | Ar^{S}-61 |
| 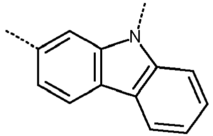 | 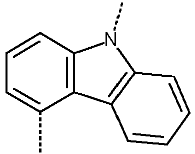 | 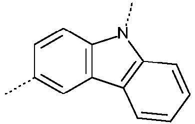 |
| Ar^{S}-62 | Ar^{S}-63 | Ar^{S}-64 |
| 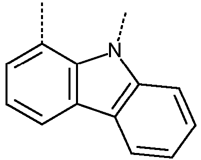 | 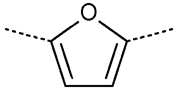 | 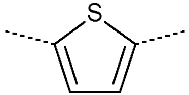 |
| Ar^{S}-65 | Ar^{S}-66 | Ar^{S}-67 |

where the dashed bonds indicate the bonds to the structure of formula (1) and where the groups (Ar^{S}-23) to (Ar^{S}-67) may be substituted at each free position by a radical R.

Preferably, R stands on each occurrence, identically or differently, for H, D, F, Cl, Br, I, CN, N(R')₂, N(Ar)₂, a straight-chain alkyl, alkoxy or thioalkyl group having 1 to 40, preferably 1 to 20, more preferably 1 to 10 C atoms or a branched or a cyclic alkyl, alkoxy or thioalkyl group having 3 to 40, preferably 3 to 20, more preferably 3 to 10 C atoms, each of which may be substituted by one or more radicals R', where in each case one or more non-adjacent CH₂ groups may be replaced by R'C=CR', C=C, O or S and where one or more H atoms may be replaced by D, F, Cl, Br, I, CN or NO₂, an aromatic or heteroaromatic ring system having 5 to 60, preferably 5 to 40, more preferably 5 to 30, particularly preferably 5 to 24, very particularly preferably 5 to 18 aromatic ring atoms, which may in each case be substituted by one or more radicals R'; where two adjacent substituents R may form an aliphatic or aromatic ring system together, which may be substituted by one or more radicals R';

Preferably, Ar is, on each occurrence, identically or differently, an aromatic or heteroaromatic ring system having 5 to 40, preferably 4 to 30, more preferably 5 to 24, particularly preferably 5 to 18 aromatic ring atoms, which may in each case also be substituted by one or more radicals R'; and

Preferably, R stands on each occurrence, identically or differently, for H, D, F, Cl, Br, I, CN, a straight-chain alkyl or alkoxy group having 1 to 20, preferably 1 to C atoms or a branched or cyclic alkyl or alkoxy group having 3 to 20, preferably 3 to 10 C atoms, or an aromatic or heteroaromatic ring system having 5 to 24, preferably 5 to 18 aromatic ring atoms.

Examples of suitable compounds of formula (1) are the structures shown in the table below:

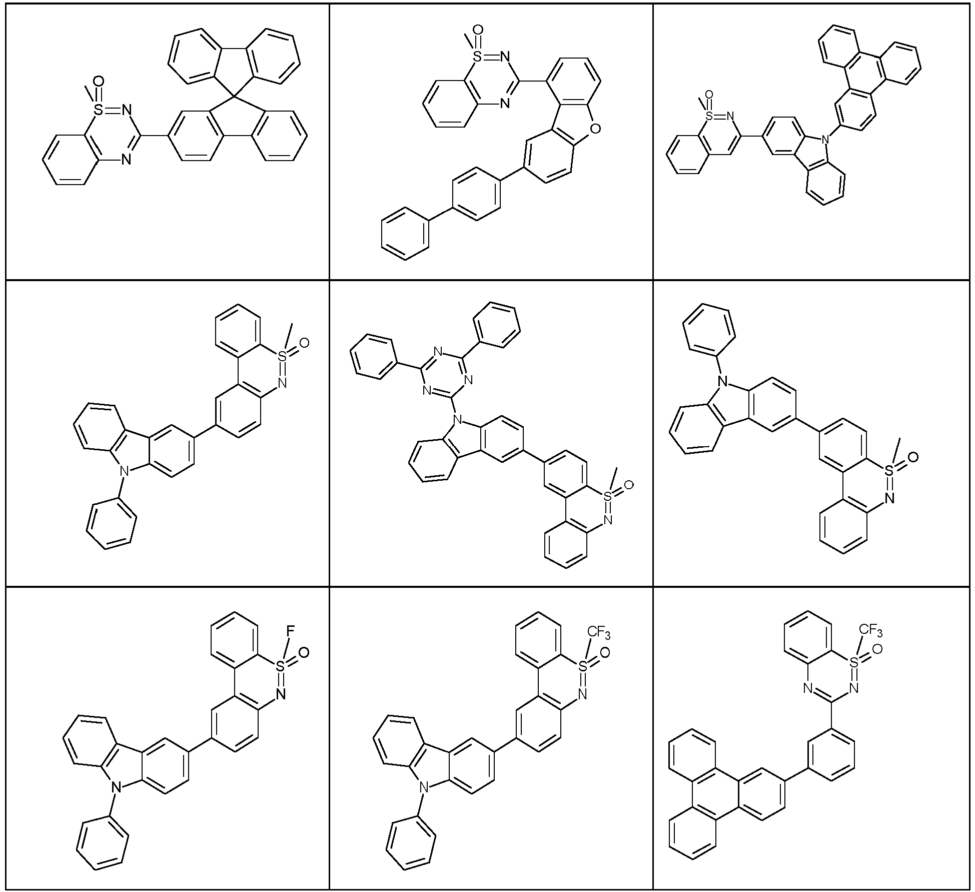

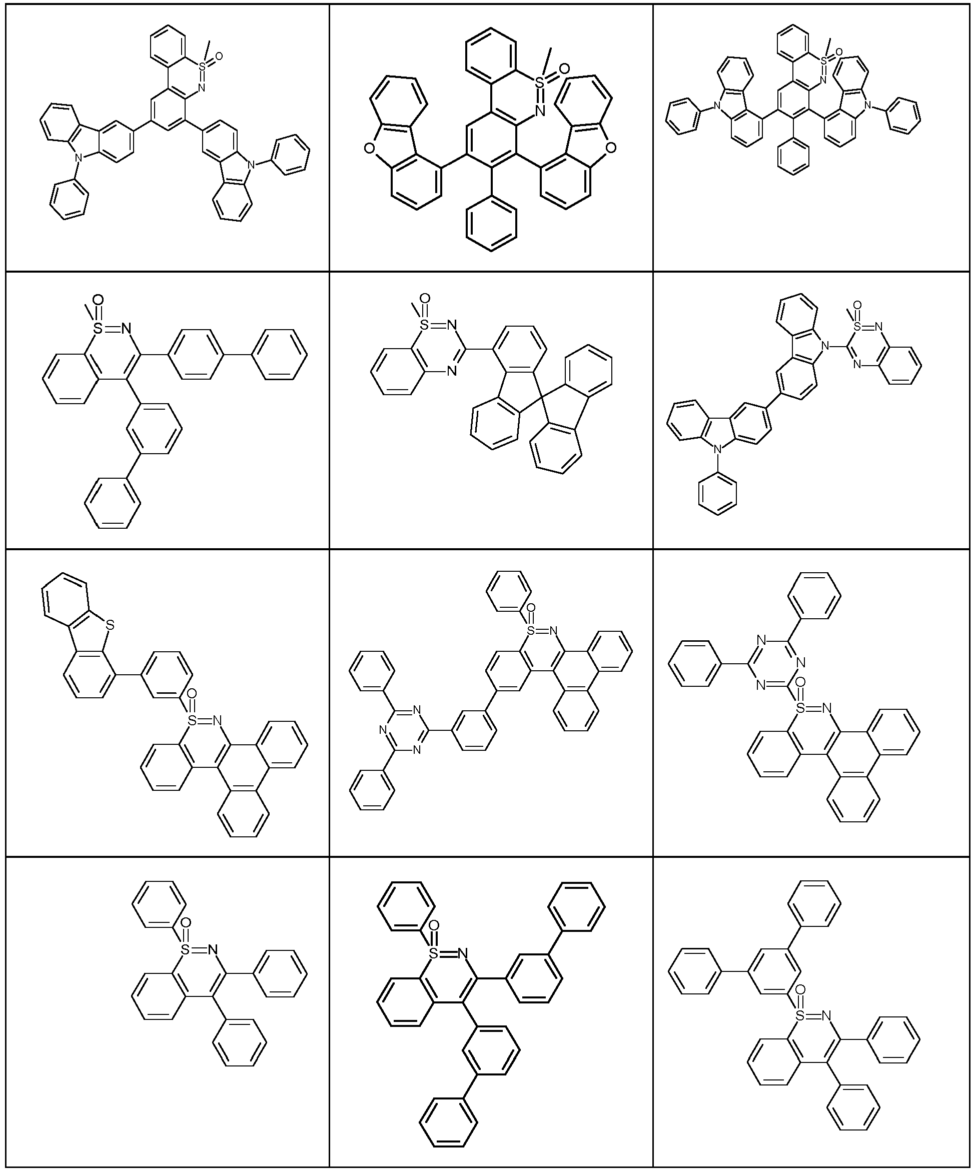

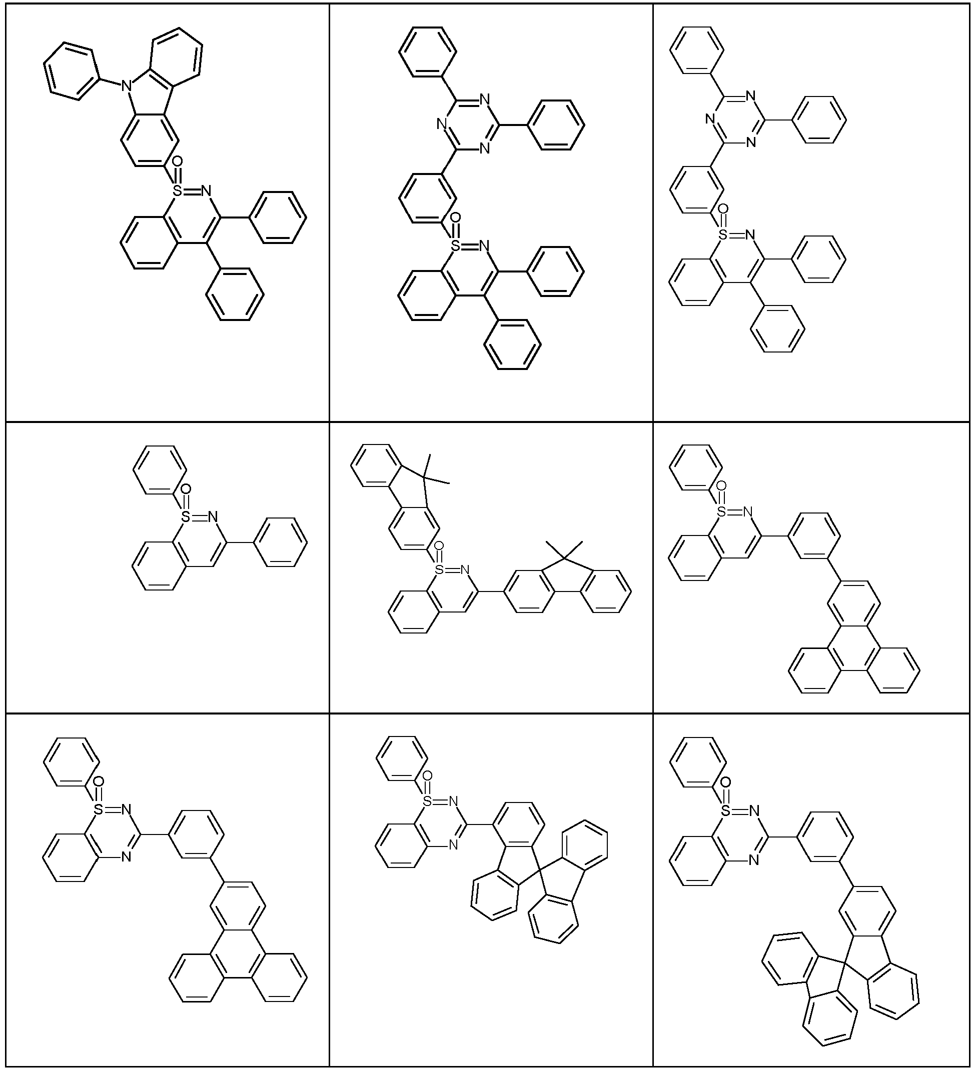

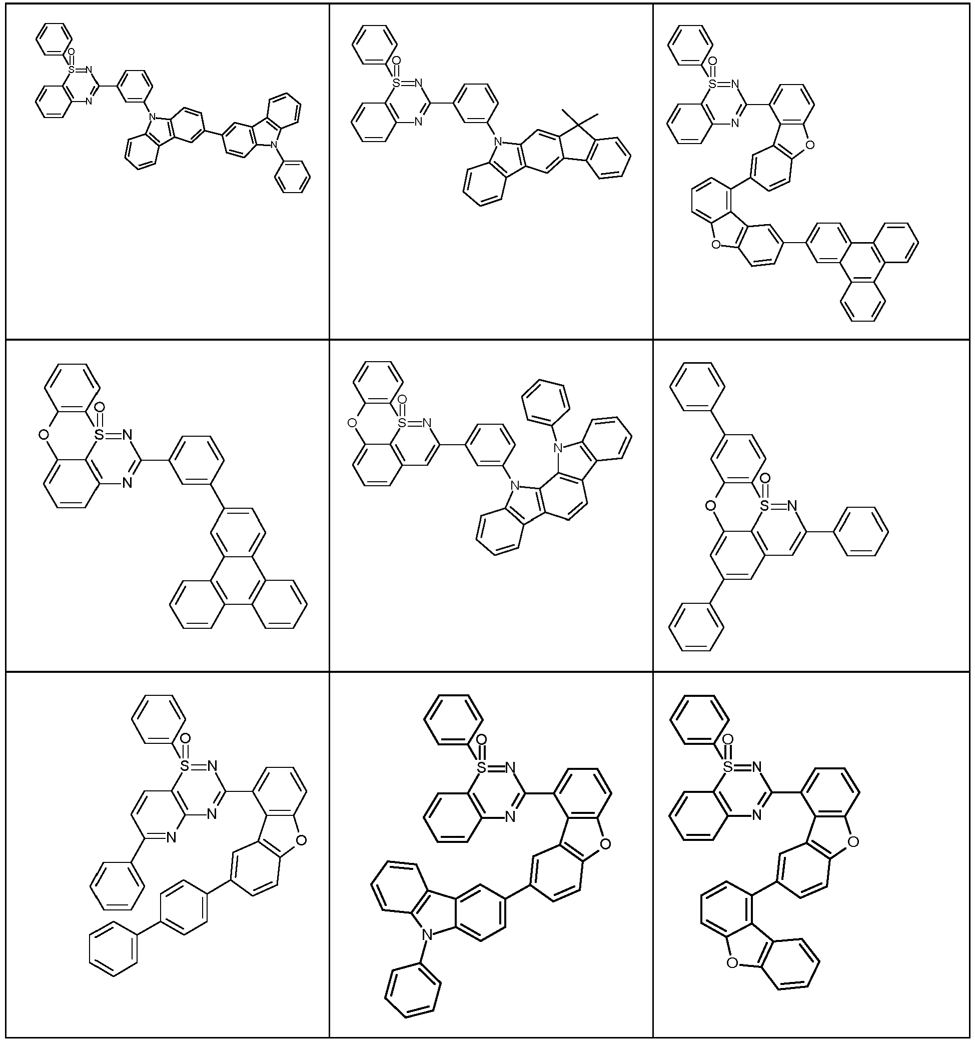

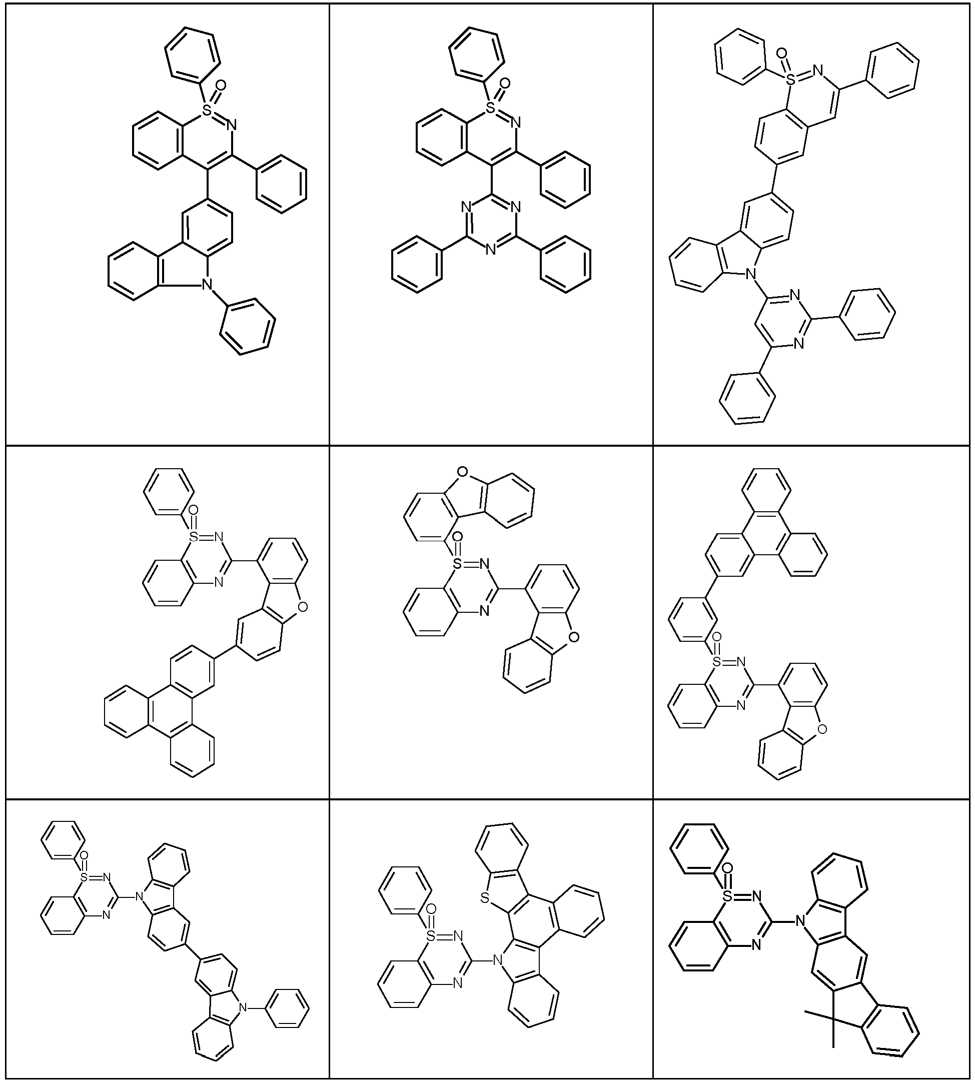

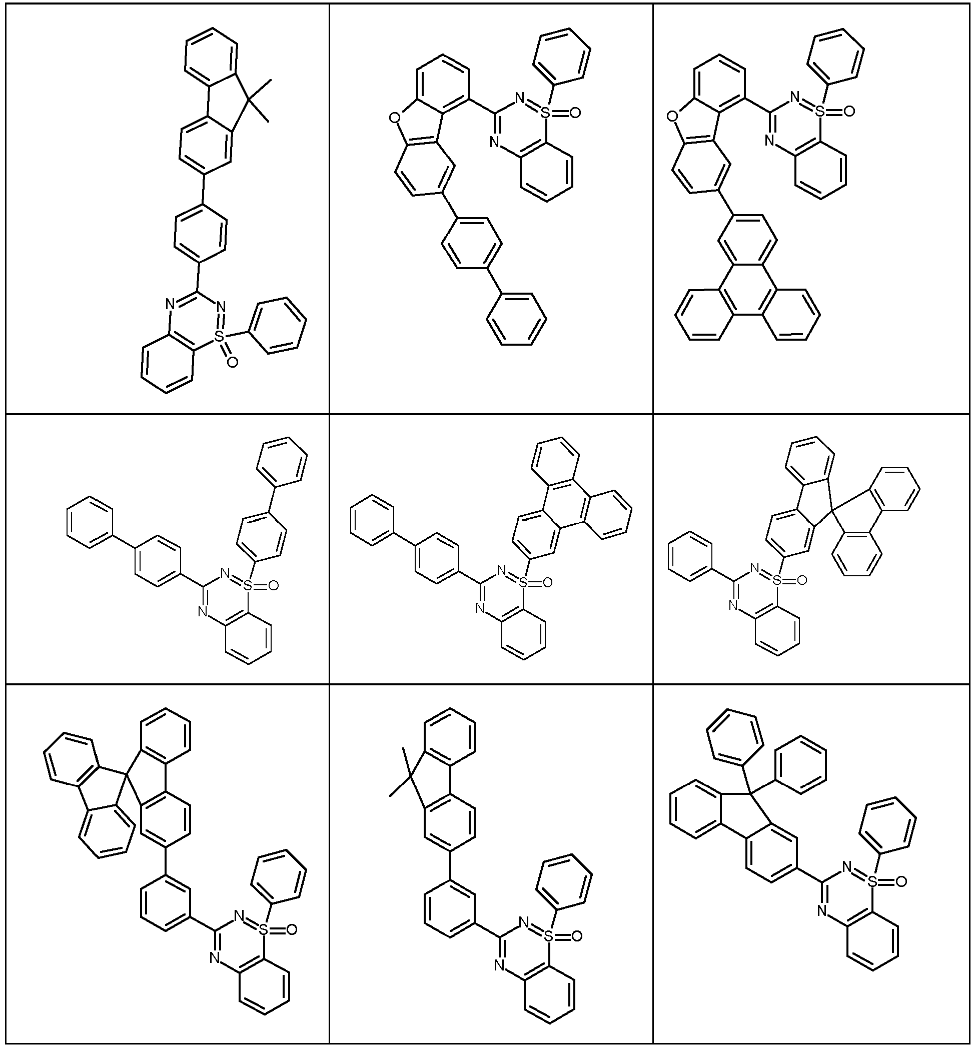

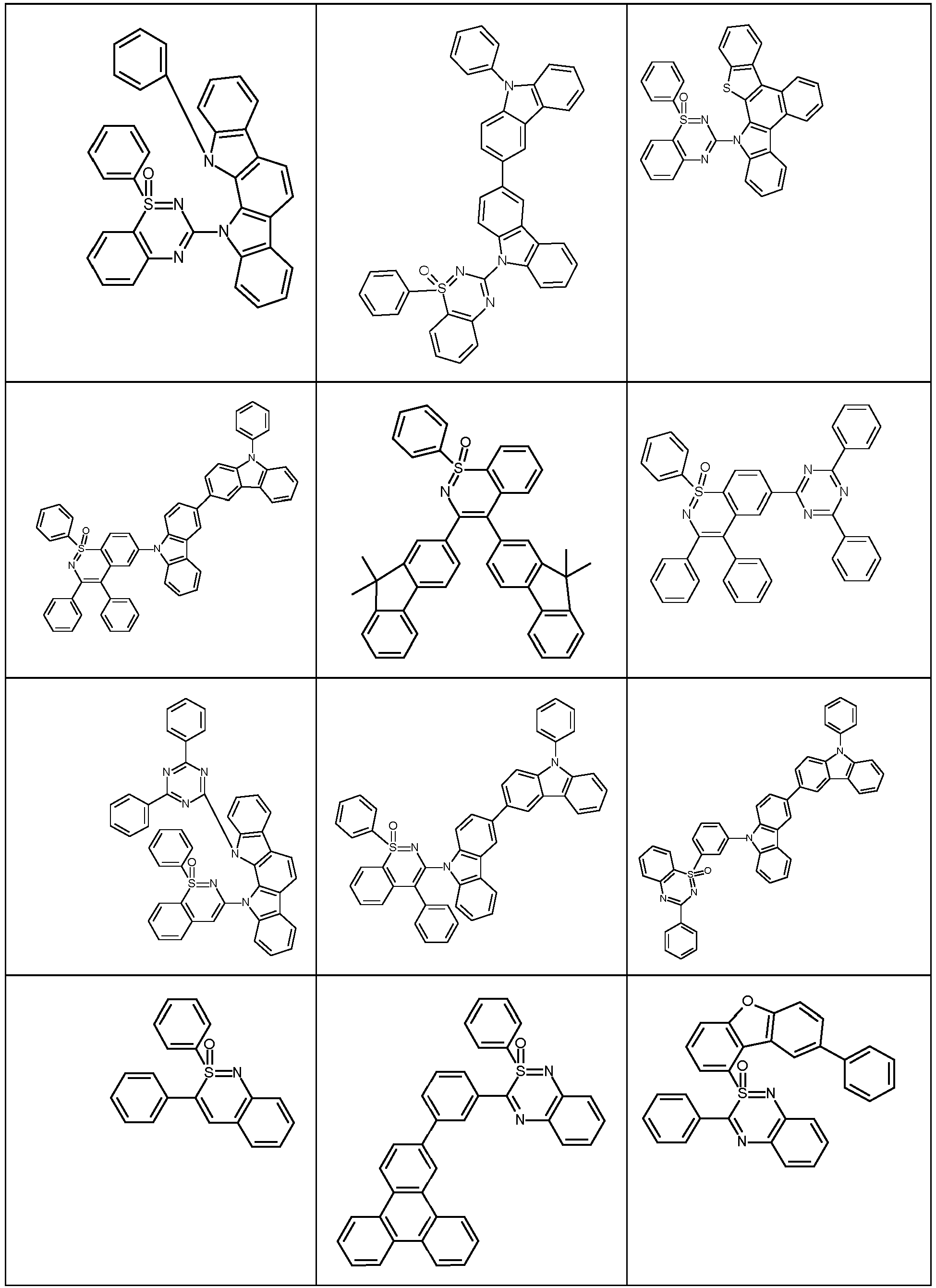

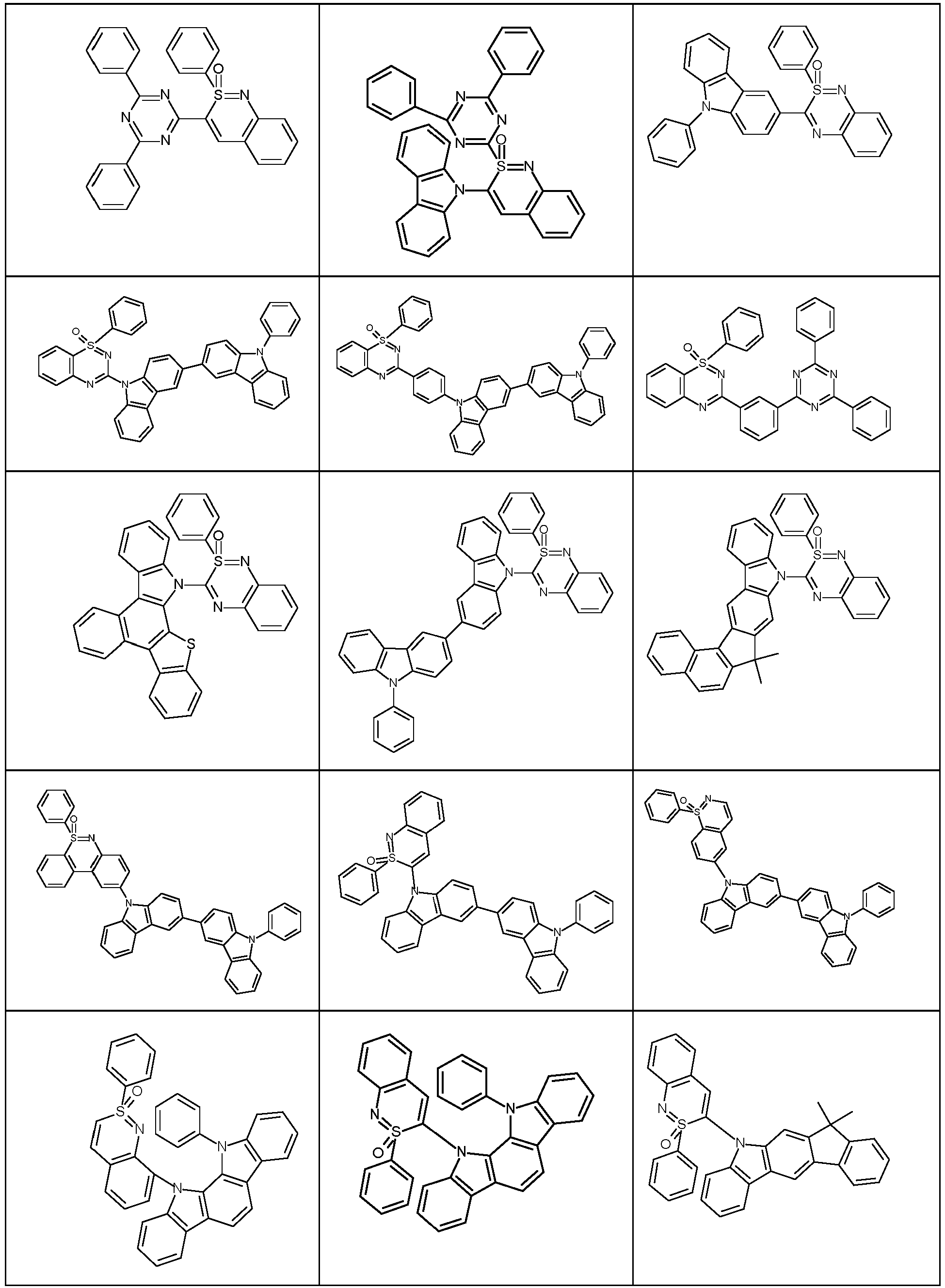

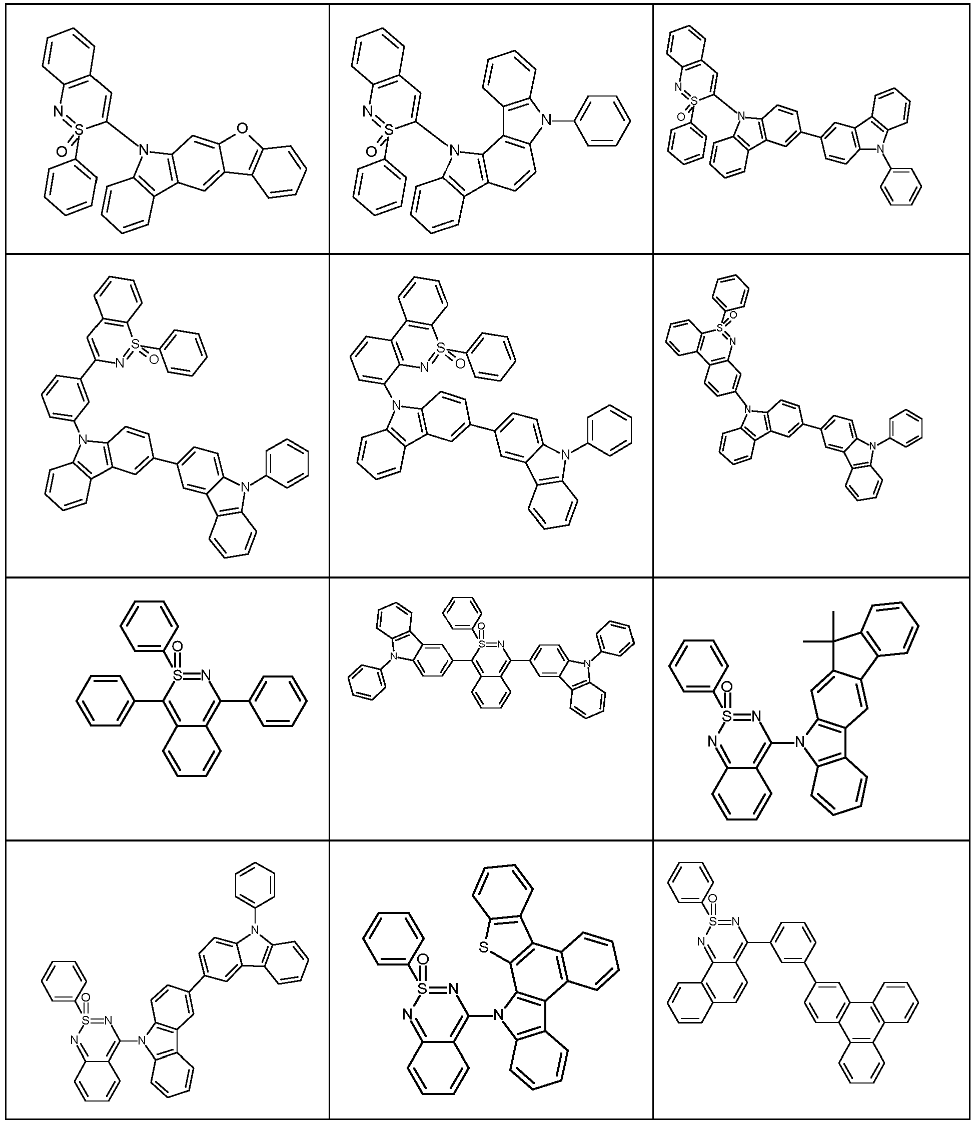

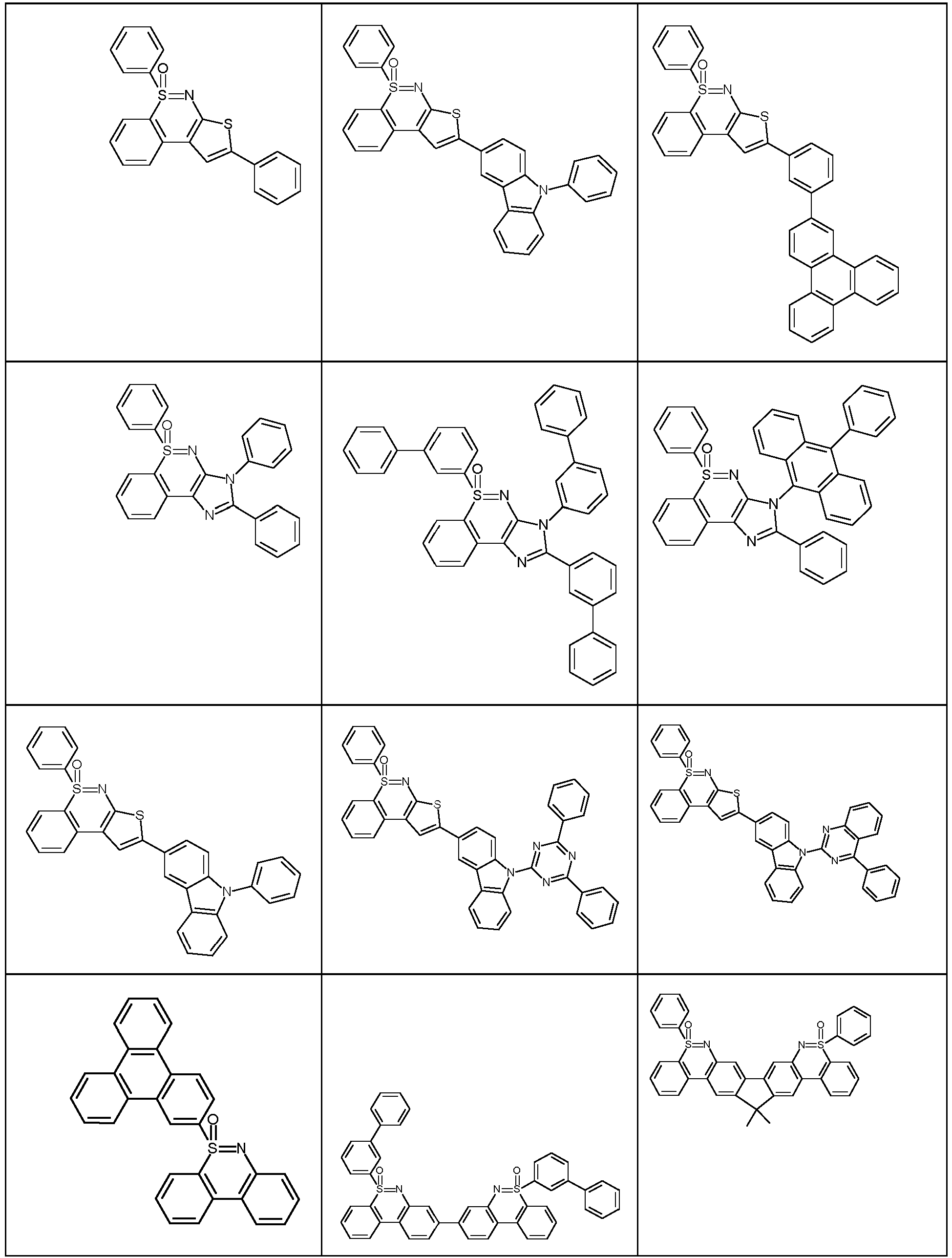

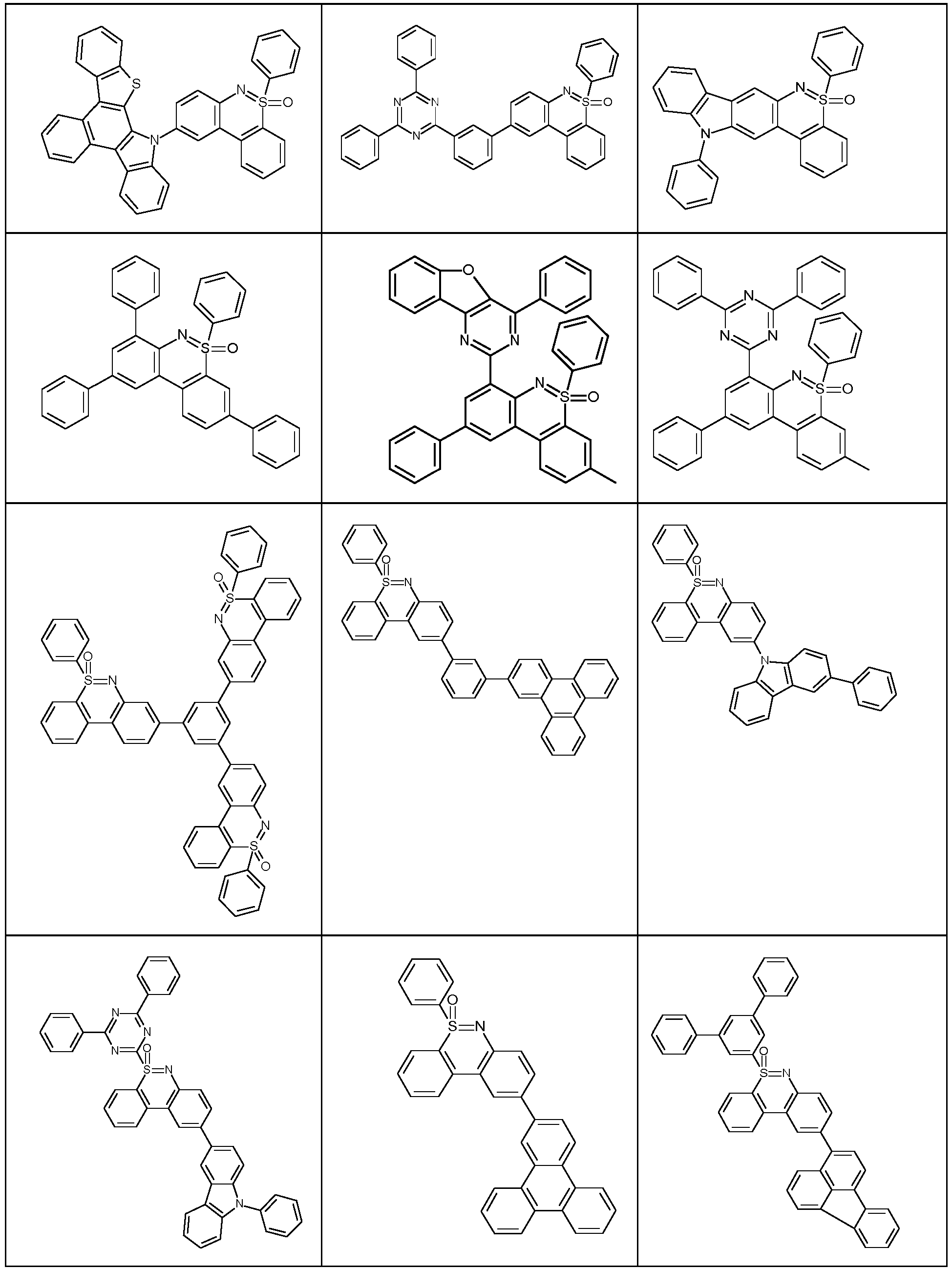

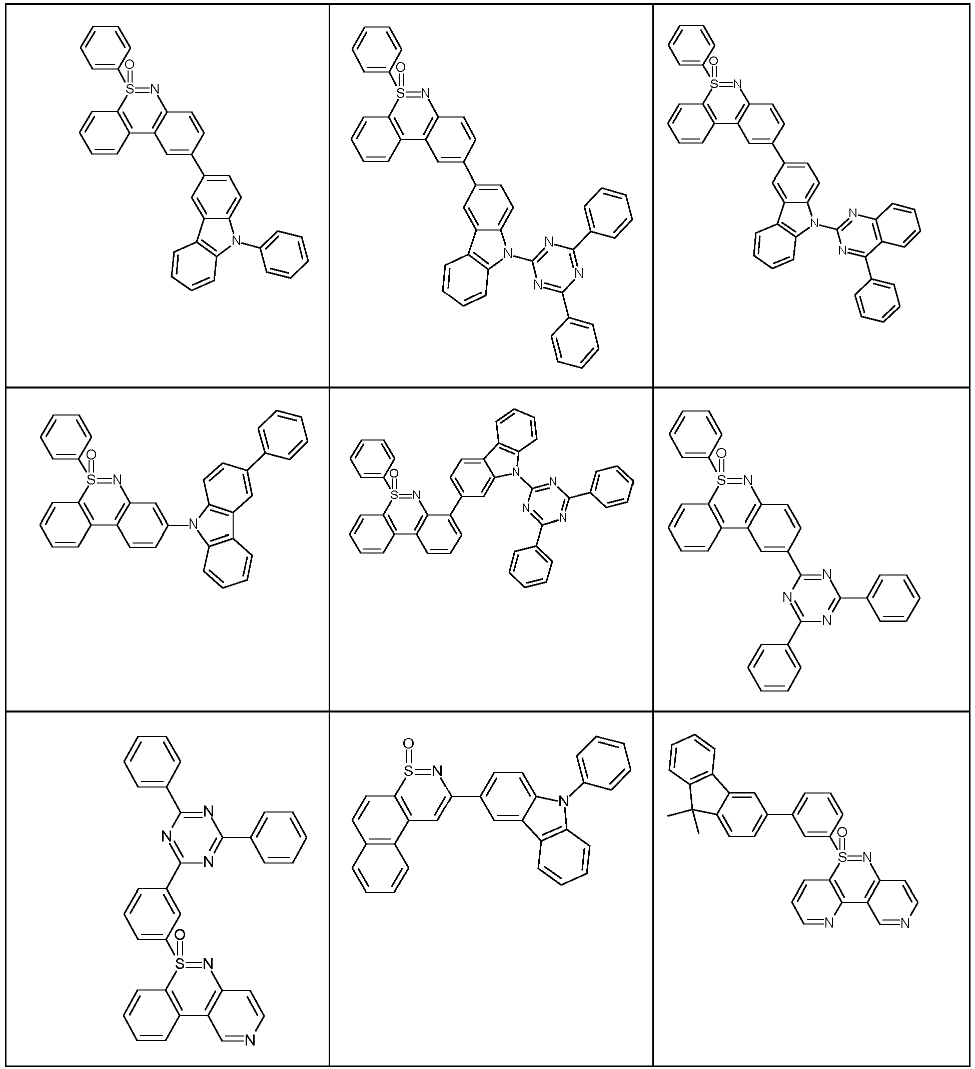

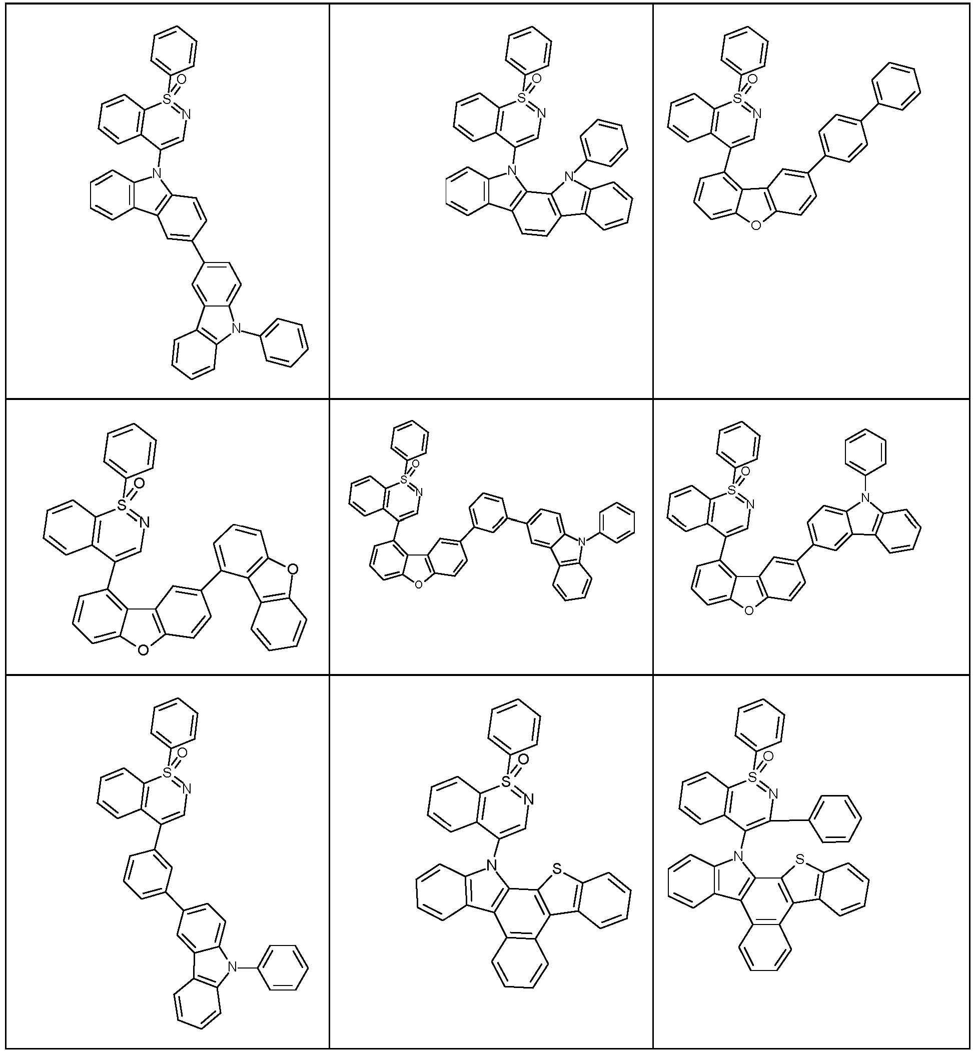

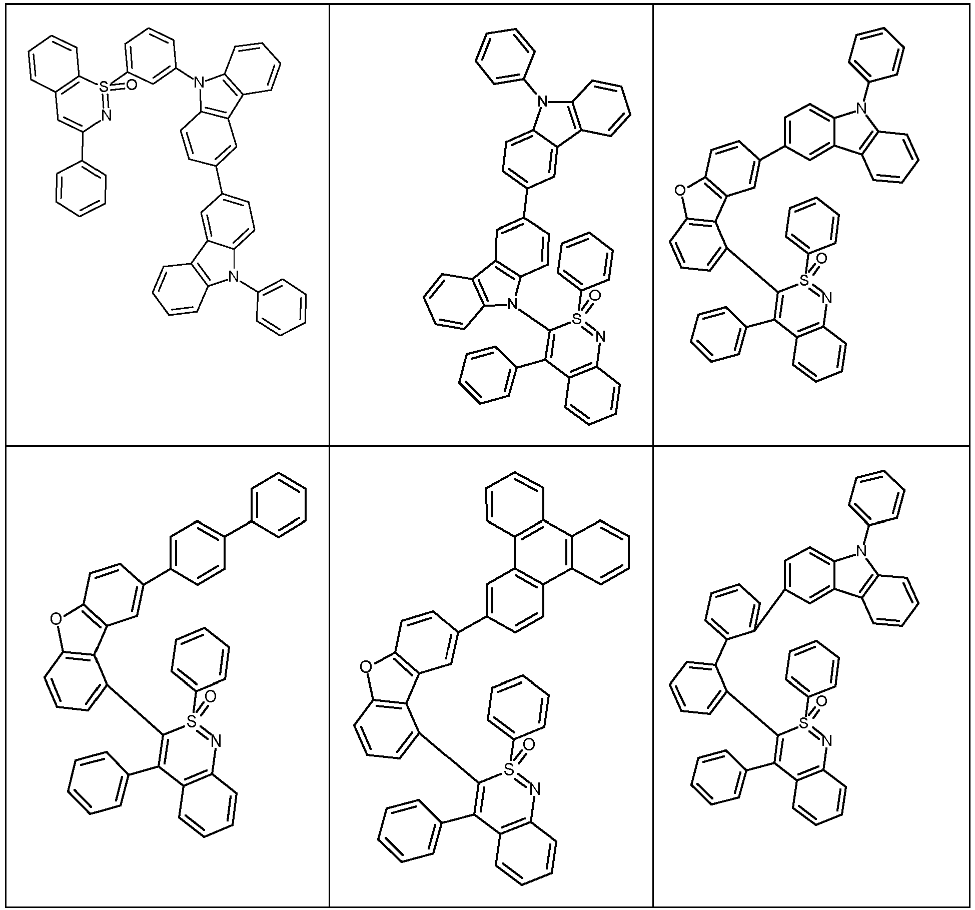

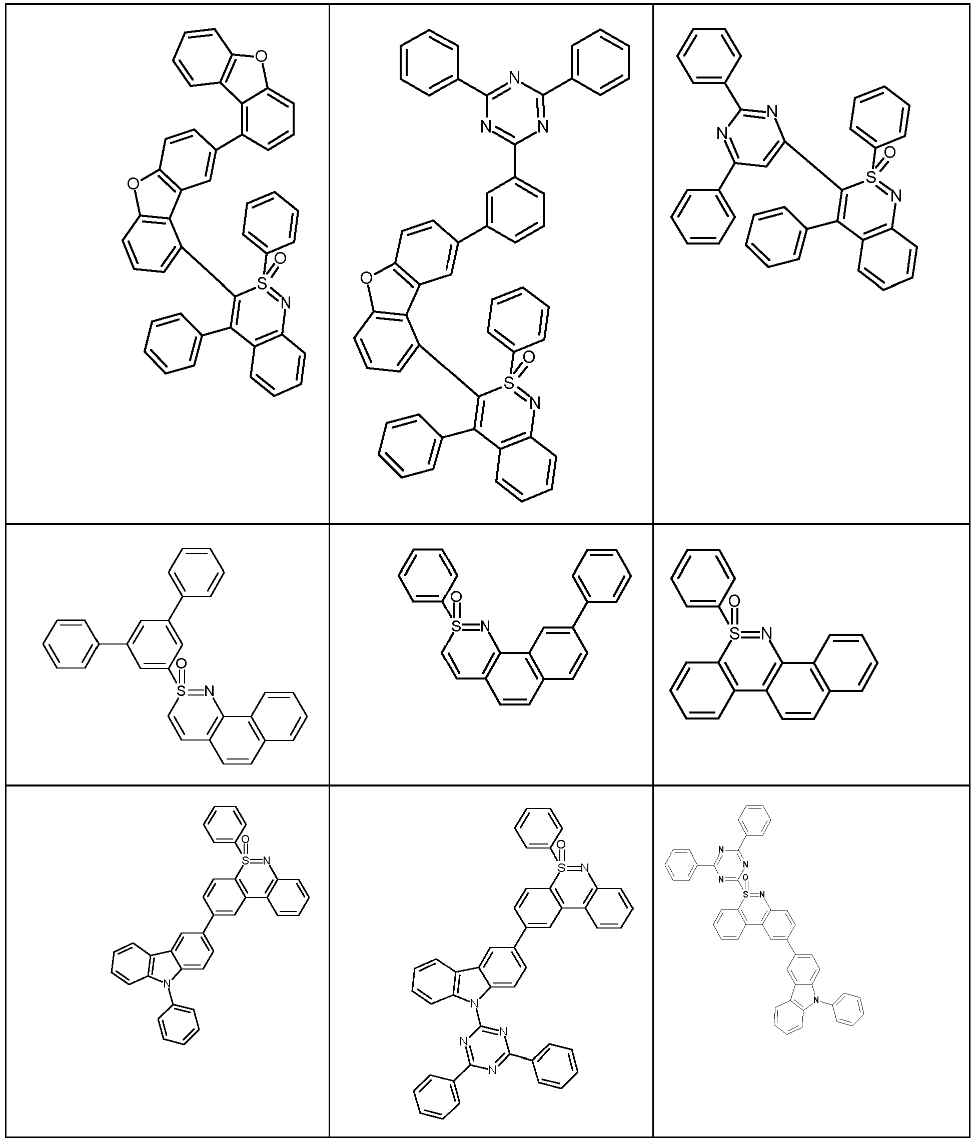

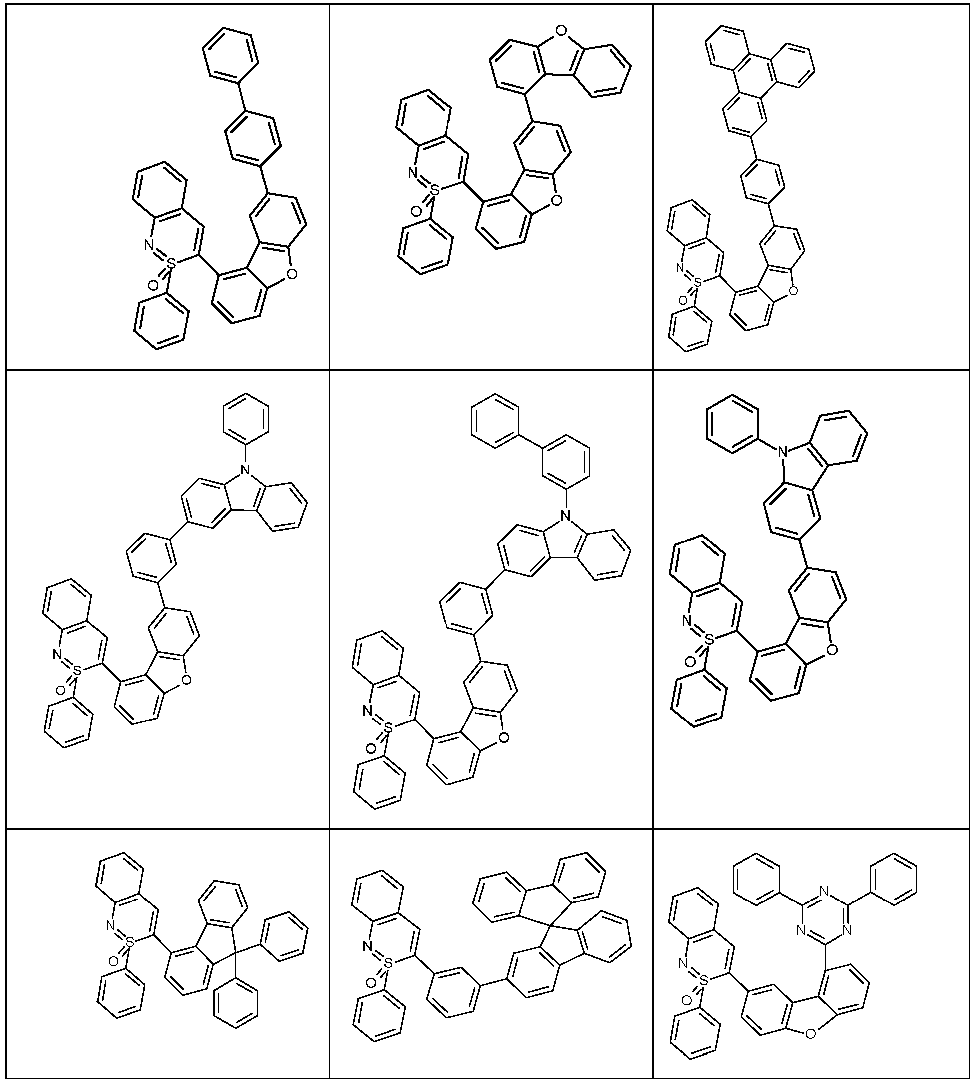

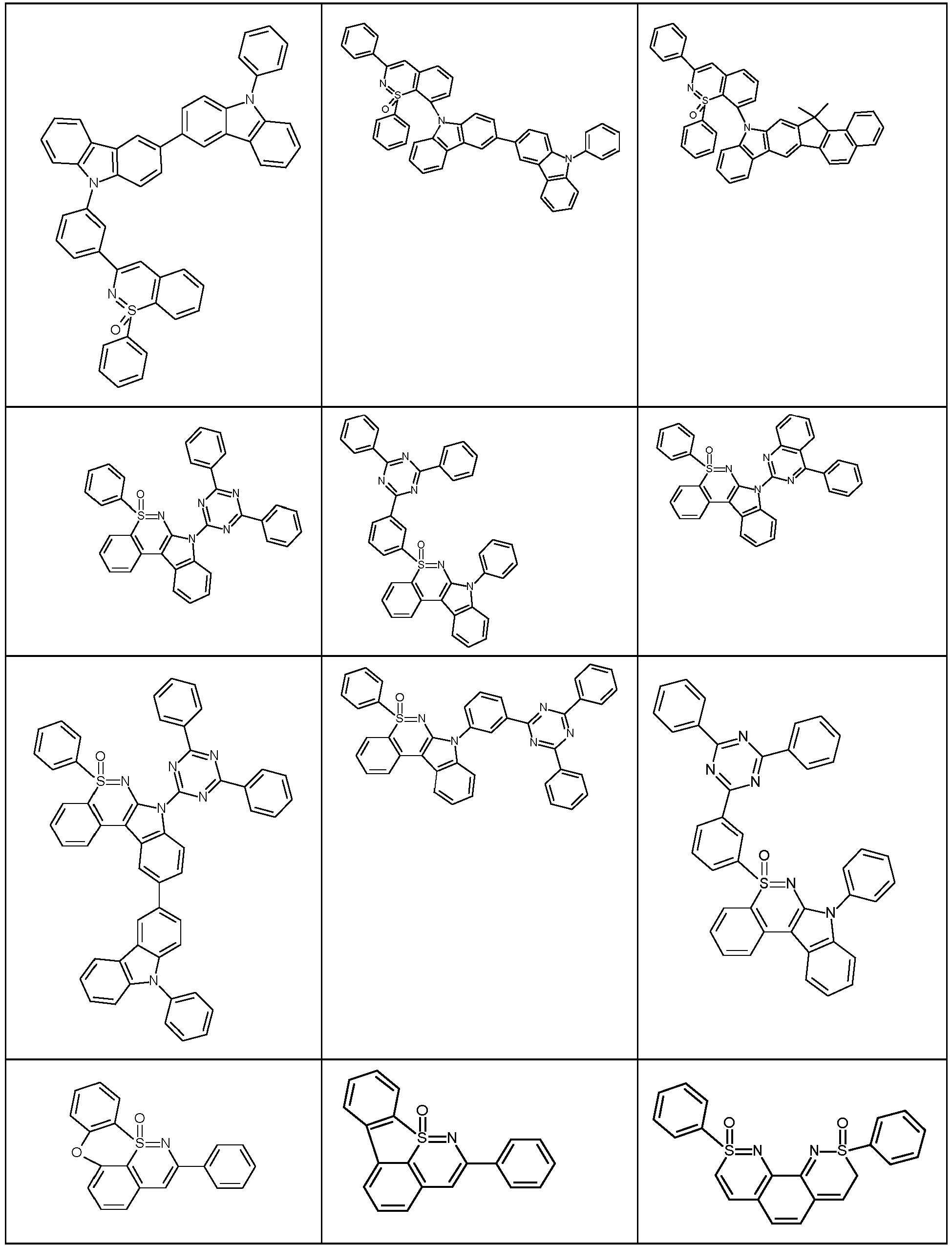

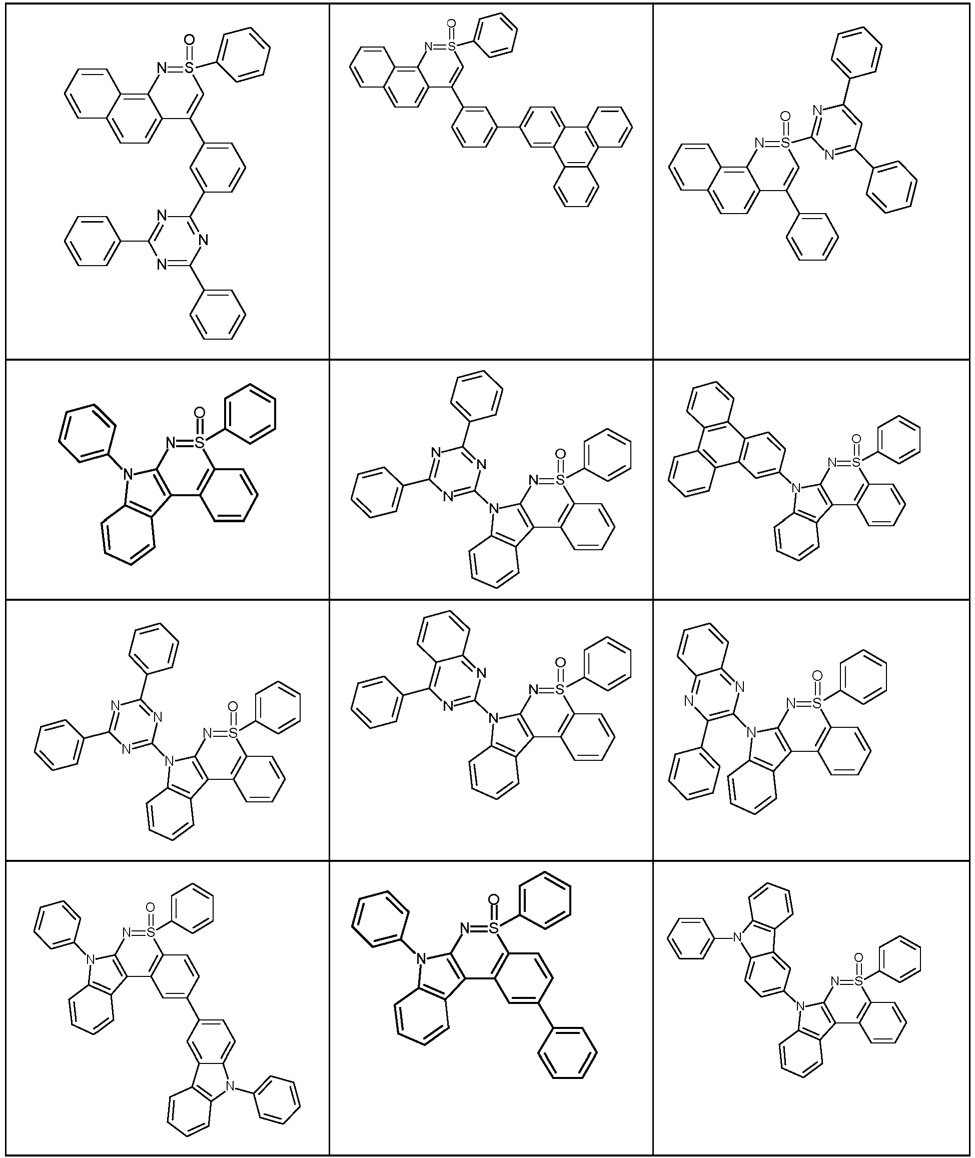

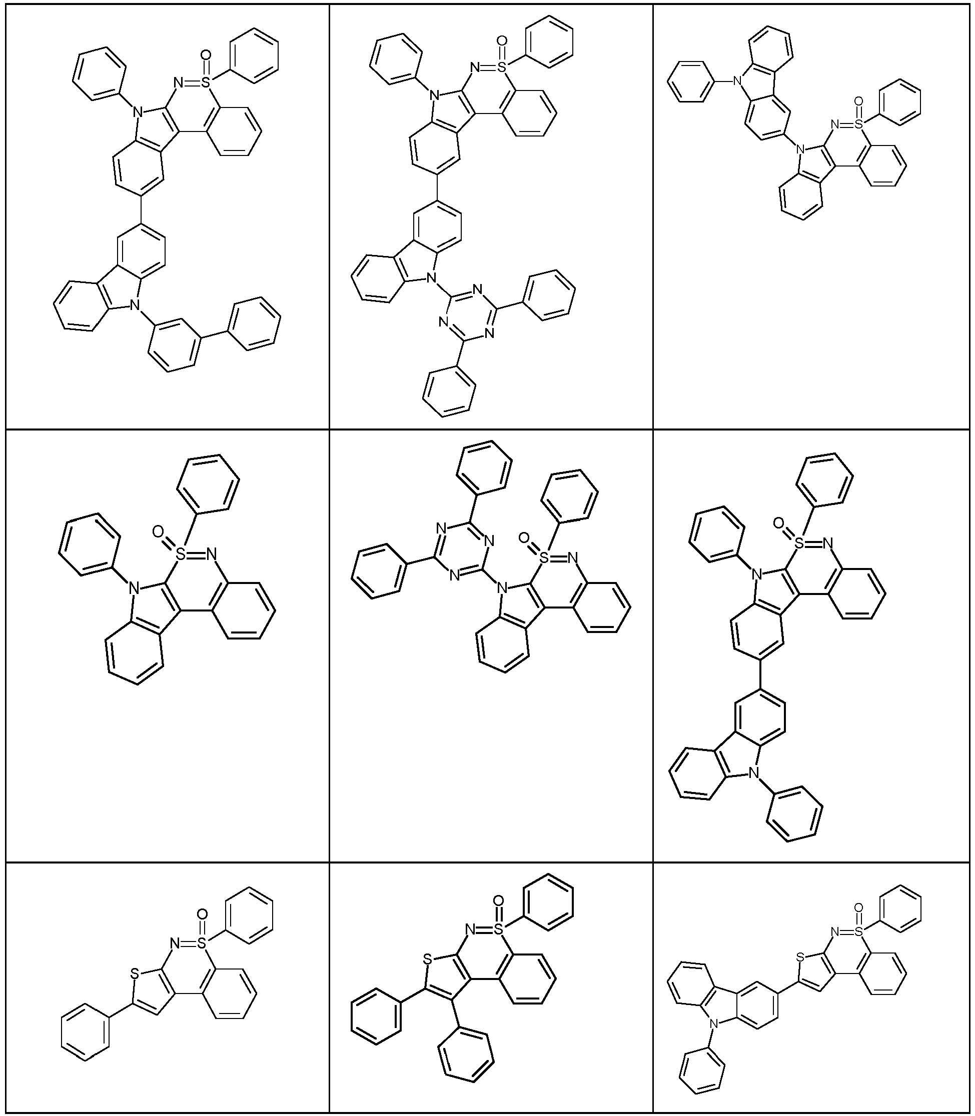

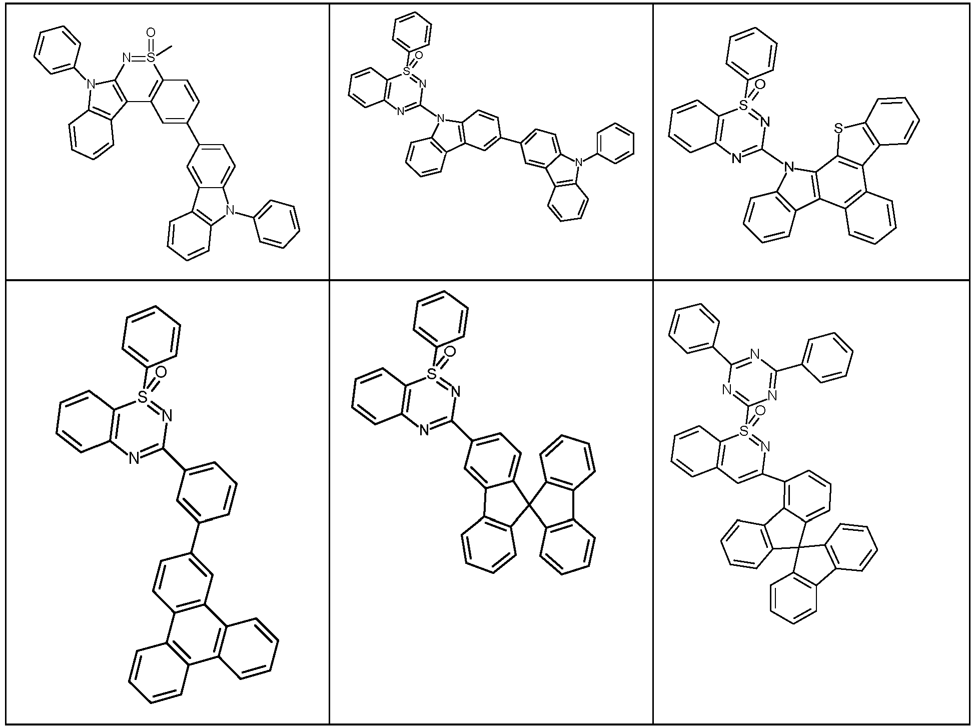

The organic electroluminescent device comprises at least one emitting layer. It may also comprise further layers, for example in each case one or more hole-injection layers, hole-transport layers, hole-blocking layers, electron-transport layers, electron-injection layers, exciton-blocking layers, electron-blocking layers and/or charge-generation layers. It is likewise possible for interlayers, which have, for example, an exciton-blocking function, to be introduced between two emitting layers. However, it should be pointed out that each of these layers does not necessarily have to be present. The organic electroluminescent device here may comprise one emitting layer or a plurality of emitting layers. If a plurality of emission layers are present, these preferably have in total a plurality of emission maxima between 380 nm and 750 nm, resulting overall in white emission, i.e. various emitting compounds which are able to fluoresce or phosphoresce are used in the emitting layers. Particular preference is given to systems having three emitting layers, where the three layers exhibit blue, green and orange or red emission (for the basic structure see, for example, WO 2005/011013). These can be fluorescent or phosphorescent emission layers or hybrid systems, in which fluorescent and phosphorescent emission layers are combined with one another.

The compound according to the invention in accordance with the above-mentioned embodiments can be employed in various layers, depending on the precise structure. Preference is given to an organic electroluminescent device comprising a compound of the formula (1) or the preferred embodiments indicated above as matrix material for fluorescent or phosphorescent emitters, in particular for phosphorescent emitters, and/or in a hole-blocking layer and/or in an electron-transport layer and/or in an electron-blocking or exciton-blocking layer and/or in a hole-transport layer, depending on the precise substitution.

In a preferred embodiment of the invention, the compound of the formula (1) or the preferred embodiments indicated above is employed as matrix material for a fluorescent or phosphorescent compound, in particular for a phosphorescent compound, in an emitting layer. The organic electroluminescent device here may comprise one emitting layer or a plurality of emitting layers, where at least one emitting layer comprises at least one compound according to the invention as matrix material.

If the compound of the formula (1) or the preferred embodiments indicated above is employed as matrix material for an emitting compound in an emitting layer, it is preferably employed in combination with one or more phosphorescent materials (triplet emitters). Phosphorescence in the sense of this invention is taken to mean the luminescence from an excited state of relatively high spin multiplicity, i.e. a spin state > 1, in particular from an excited triplet state. For the purposes of this application, all luminescent complexes with transition metals or lanthanides, in particular all iridium, platinum and copper complexes, are to be regarded as phosphorescent compounds.

The mixture of the compound of the formula (1) or the preferred embodiments indicated above and the emitting compound comprises between 99 and 1% by vol., preferably between 98 and 10% by vol., particularly preferably between 97 and 60% by vol., in particular between 95 and 80% by vol., of the compound of the formula (1) or the preferred embodiments indicated above, based on the entire mixture comprising emitter and matrix material. Correspondingly, the mixture comprises between 1 and 99% by vol., preferably between 2 and 90% by vol., particularly preferably between 3 and 40% by vol., in particular between 5 and 20% by vol., of the emitter, based on the entire mixture comprising emitter and matrix material.

A further preferred embodiment of the present invention is the use of the compound of the formula (1) or the preferred embodiments indicated above as matrix material for a phosphorescent emitter in combination with a further matrix material. Particularly suitable matrix materials which can be employed in combination with the compounds of the formula (1) or in accordance with the preferred embodiments are aromatic ketones, aromatic phosphine oxides or aromatic sulfoxides or sulfones, for example in accordance with WO 2004/013080, WO 2004/093207, WO 2006/005627 or WO 2010/006680, triarylamines, carbazole derivatives, for example CBP (N,N-bis-carbazolylbiphenyl) or the carbazole derivatives disclosed in WO 2005/039246, US 2005/0069729, JP 2004/288381, EP 1205527, WO 2008/086851 or WO 2013/041176, indolocarbazole derivatives, for example in accordance with WO 2007/063754 or WO 2008/056746, indenocarbazole derivatives, for example in accordance with WO 2010/136109, WO 2011/000455, WO 2013/041176 or WO 2013/056776, azacarbazole derivatives, for example in accordance with EP 1617710, EP 1617711, EP 1731584, JP 2005/347160, bipolar matrix materials, for example in accordance with WO 2007/137725, silanes, for example in accordance with WO 005/111172, azaboroles or boronic esters, for example in accordance with WO 2006/117052, triazine derivatives, for example in accordance with WO 2007/063754, WO 2008/056746, WO 2010/015306, WO 2011/057706, WO 2011/060859 or WO 2011/060877, zinc complexes, for example in accordance with EP 652273 or WO 2009/062578, diazasilole or tetraazasilole derivatives, for example in accordance with WO 2010/054729, diazaphosphole derivatives, for example in accordance with WO 2010/054730, bridged carbazole derivatives, for example in accordance with US 2009/0136779, WO 2010/050778, WO 2011/042107, WO 2011/060867, WO 2011/088877 und WO 2012/143080, triphenylene derivatives, for example in accordance with WO 2012/048781, or debinzofuran derivatives, for example in accordance with WO 2015/169412, WO 2016/015810, WO 2016/023608, WO 2017/148564 orr WO 2017/148565, or lactams, for example in accordance with WO 2011/116865 or WO 2011/137951. A further phosphorescent emitter which emits at shorter wavelength than the actual emitter may likewise be present in the mixture as co-host like, for example in WO 2010/108579.

If the compound of the formula (1) or in accordance with the preferred embodiments is employed as matrix material for an emitting compound in an emitting layer, it is preferably employed in combination with one or more phosphorescent materials (triplet emitters). Phosphorescence in the sense of this invention is taken to mean the luminescence from an excited state having spin multiplicity > 1, in particular from an excited triplet state. For the purposes of this application, all luminescent transition-metal complexes and luminescent lanthanide complexes, in particular all iridium, platinum and copper complexes, are to be regarded as phosphorescent compounds.

Preferably, when the compounds of the formula (1) or in accordance with the preferred embodiments are employed as matrix materials for an emitting compound in an emitting layer, they are preferably employed in combination with one or more phosphorescent material (triplet emitters).

The mixture comprising the compound of the formula (1) or in accordance with the preferred embodiments and the emitting compound comprises between 99 and 1% by vol., preferably between 98 and 10% by vol., particularly preferably between 97 and 60% by vol., in particular between 95 and 80% by vol., of the compound of the formula (1) or in accordance with the preferred embodiments, based on the entire mixture comprising emitter and matrix material. Correspondingly, the mixture comprises between 1 and 99% by vol., preferably between 2 and 90% by vol., particularly preferably between 3 and 40% by vol., in particular between 5 and 20% by vol., of the emitter, based on the entire mixture comprising emitter and matrix material.

Suitable phosphorescent compounds (= triplet emitters) are, in particular, compounds which emit light, preferably in the visible region, on suitable excitation and in addition contain at least one atom having an atomic number greater than 20, preferably greater than 38 and less than 84, particularly preferably greater than 56 and less than 80, in particular a metal having this atomic number. The phosphorescent emitters used are preferably compounds which contain copper, molybdenum, tungsten, rhenium, ruthenium, osmium, rhodium, iridium, palladium, platinum, silver, gold or europium, in particular compounds which contain iridium or platinum. For the purposes of the present invention, all luminescent compounds which contain the above-mentioned metals are regarded as phosphorescent compounds.

Examples of the emitters described above are revealed by the applications WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094962, WO 2014/094961, WO 2014/094960, WO 2016/124304, WO 2016/125715, WO 2017/032439, WO 2018/011186, WO 2018/041769, WO 2019/020538, WO 2018/178001, WO 2019/115423 and WO2019/158453. In general, all phosphorescent complexes as used in accordance with the prior art for phosphorescent OLEDs and as are known to the person skilled in the art in the area of organic electroluminescence are suitable, and the person skilled in the art will be able to use further phosphorescent complexes without inventive step.

Examples of suitable phosphorescent emitters are the phosphorescent emitters listed in the table below:

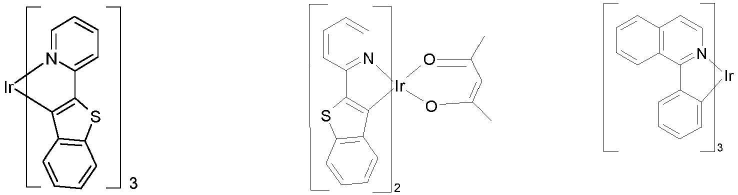

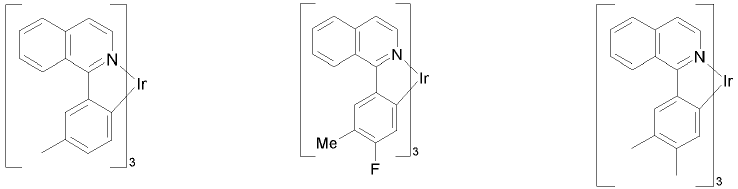

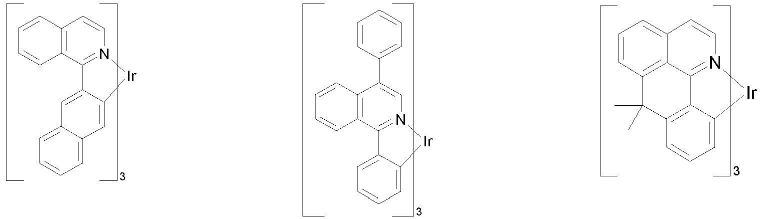

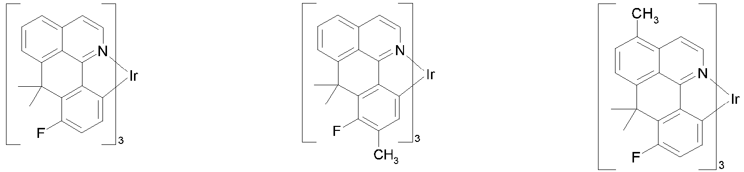

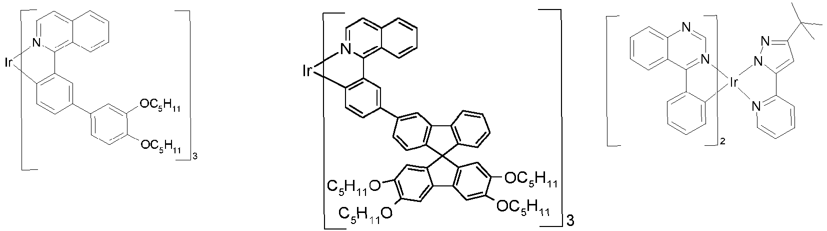

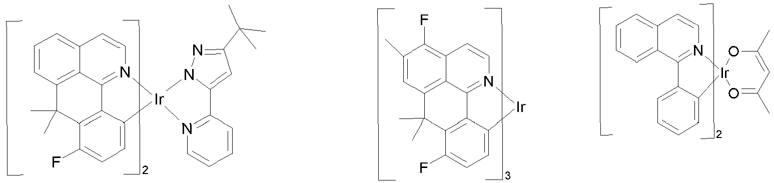

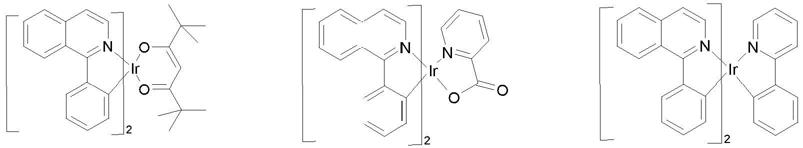

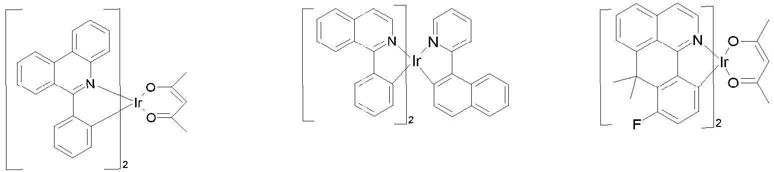

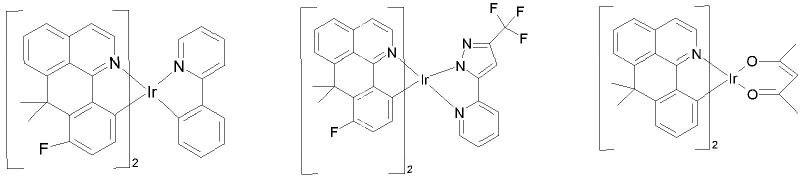

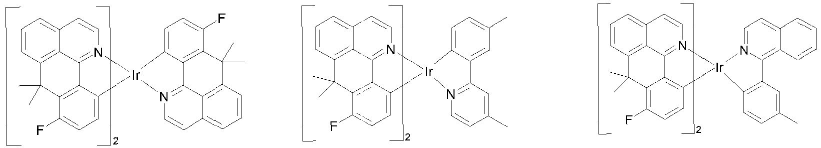

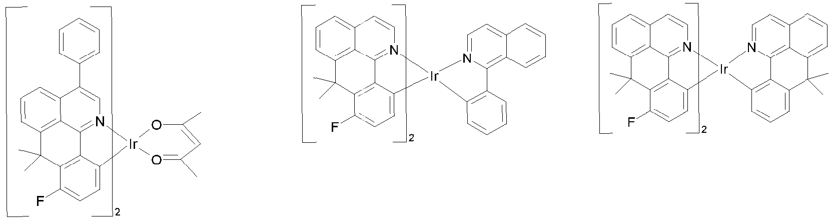

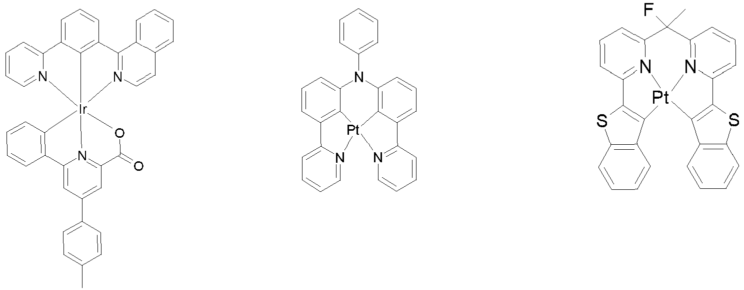

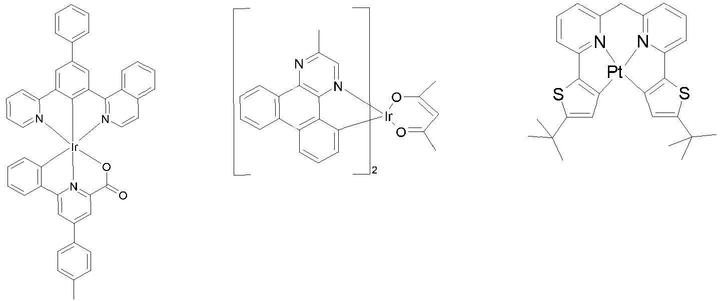

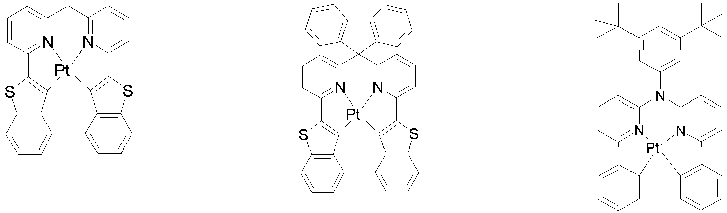

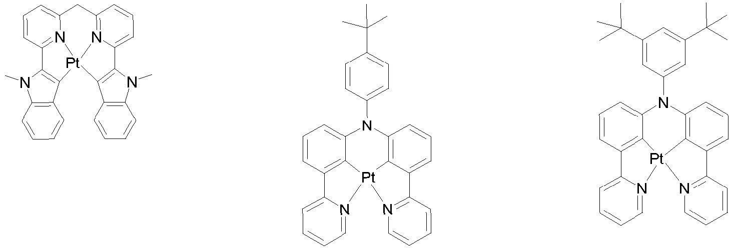

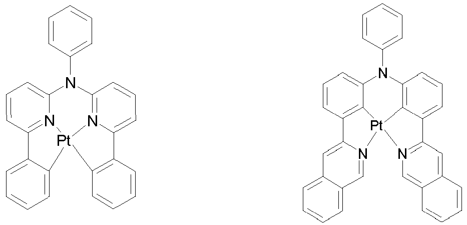

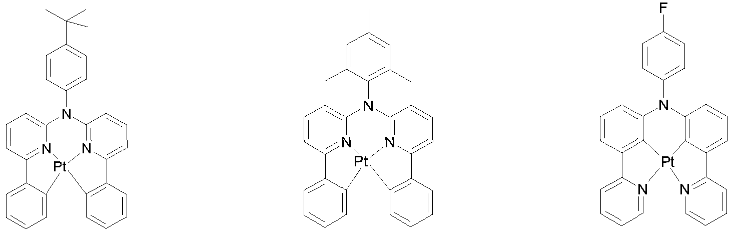

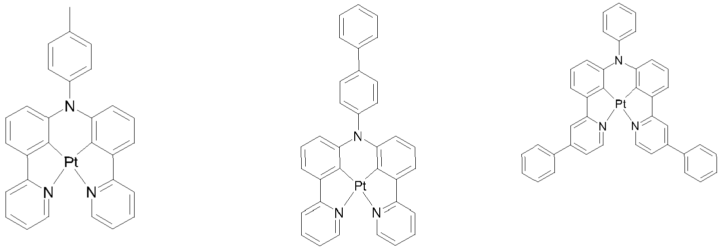

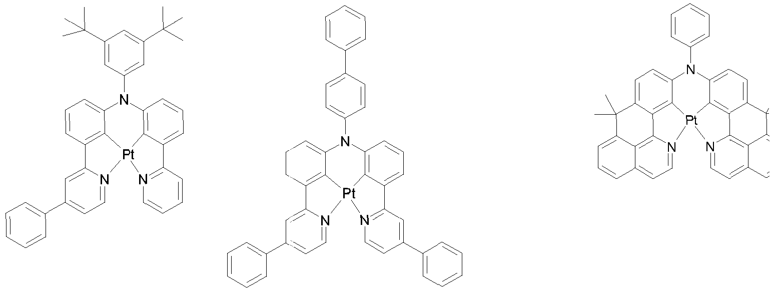

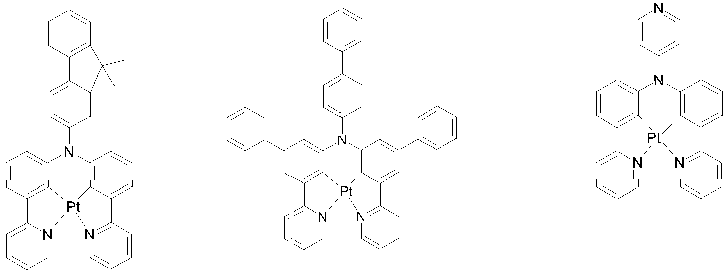

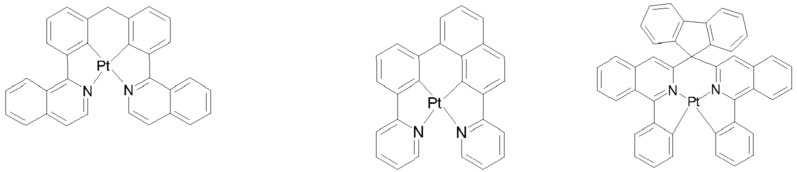

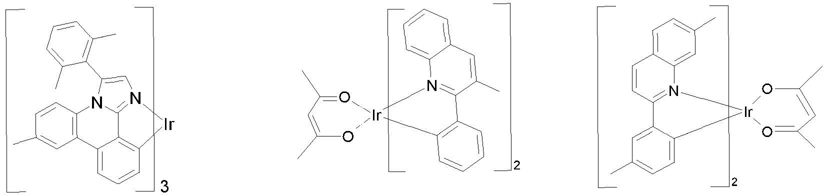

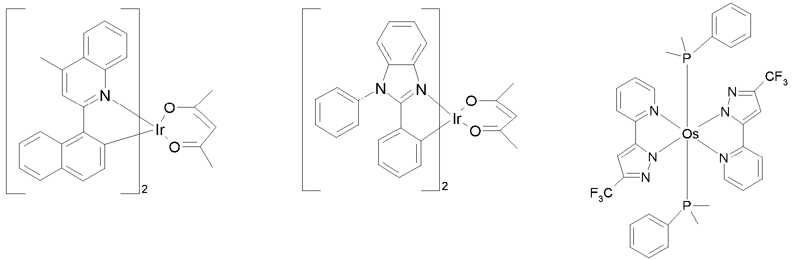

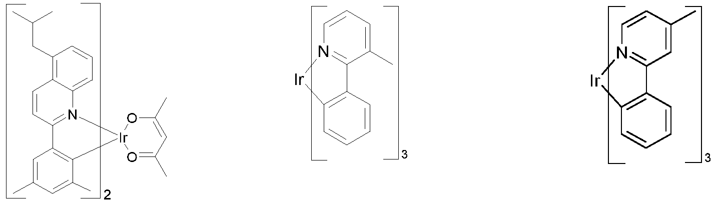

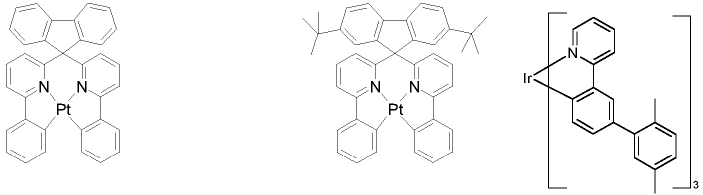

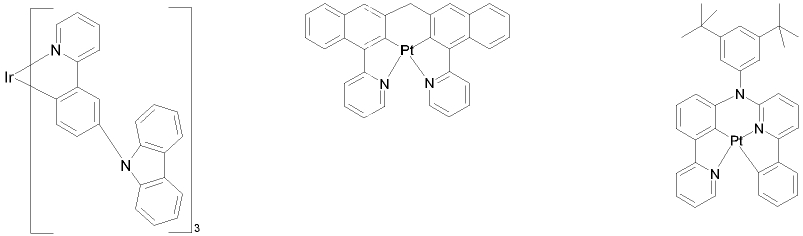

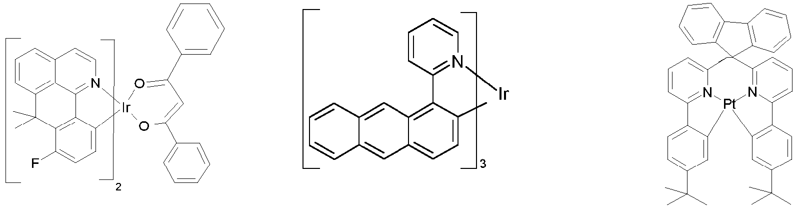

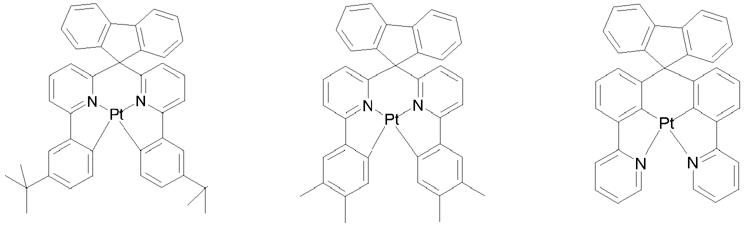

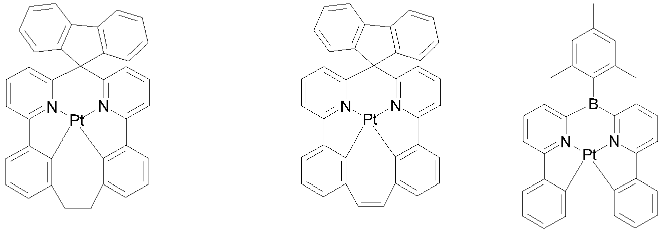

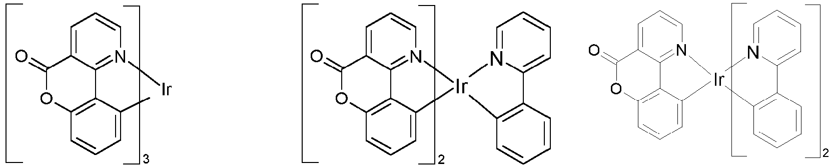

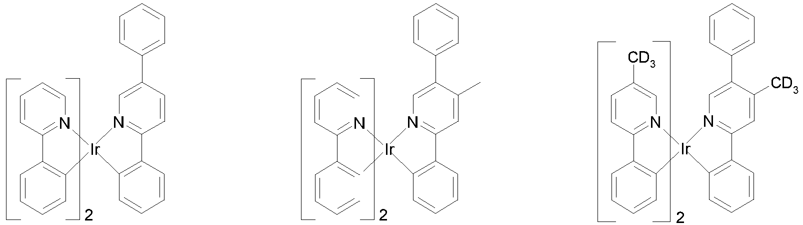

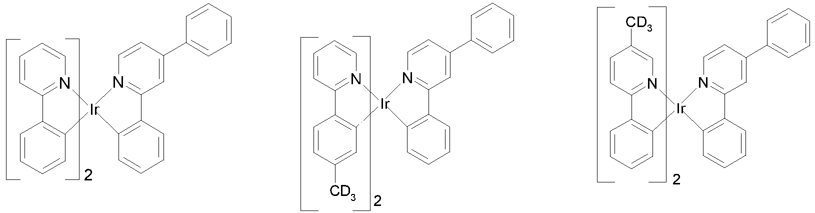

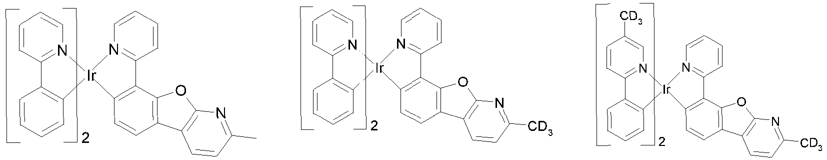

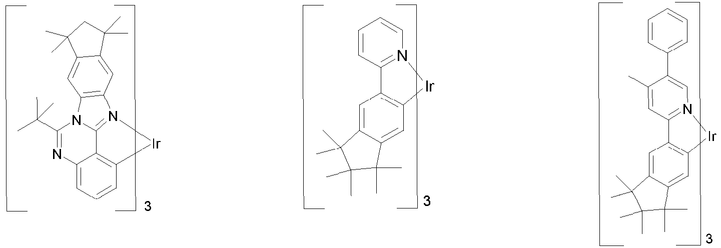

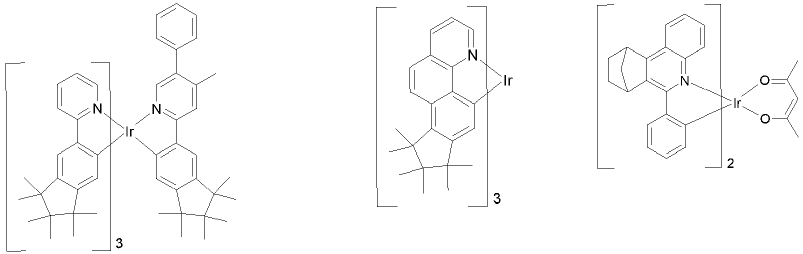

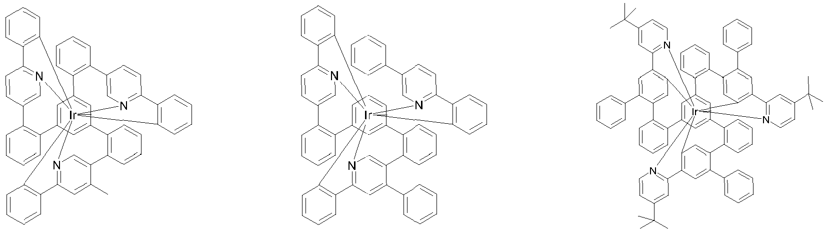

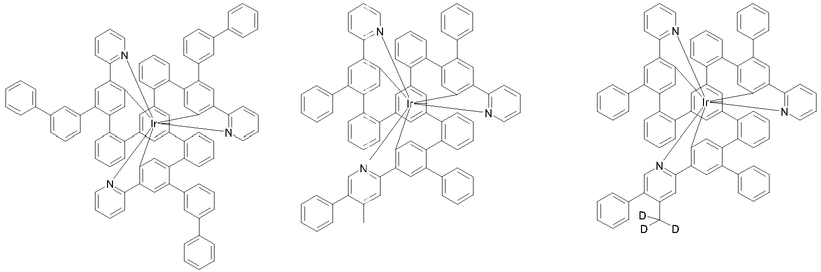

Suitable phosphorescent materials (= triplet emitters) that can be advantageously combined with the compounds of formula (1) are, as mentioned above, compounds which emit a red light on suitable excitation, which means phosphorescent materials having an excited triplet state level (T1) comprised between 550 and 680 nm.

In a further embodiment of the invention, the organic electroluminescent device according to the invention does not comprise a separate hole-injection layer and/or hole-transport layer and/or hole-blocking layer and/or electron-transport layer, i.e. the emitting layer is directly adjacent to the hole-injection layer or the anode, and/or the emitting layer is directly adjacent to the electron-transport layer or the electron-injection layer or the cathode, as described, for example, in WO 2005/053051. It is furthermore possible to use a metal complex which is identical or similar to the metal complex in the emitting layer as hole-transport or hole-injection material directly adjacent to the emitting layer, as described, for example, in WO 2009/030981.

It is furthermore possible to employ the compounds according to the invention in a hole-blocking or electron-transport layer. This applies, in particular, to compounds according to the invention which do not have a carbazole structure. These may preferably also be substituted by one or more further electron-transporting groups, for example benzimidazole groups.

In the further layers of the organic electroluminescent device according to the invention, it is possible to use all materials as usually employed in accordance with the prior art. The person skilled in the art will therefore be able, without inventive step, to employ all materials known for organic electroluminescent devices in combination with the compounds of the formula (1) or in accordance with the preferred embodiments.

For example, the compound according to the invention can also be used as a matrix for semiconducting light-emitting nanoparticles. In the context of the present invention, the term "nano" denotes a size in the range from 0.1 to 999 nm, preferably from 1 to 150 nm. In a preferred embodiment, the semiconducting light-emitting nano-particle is a quantum material ("Quantum sized material"). The term "quantum material" in the sense of the present invention refers to the size of the semiconductor material itself without further connections or a further surface modification, which shows the so-called quantum confinement effect, as for example in ISBN: 978-3-662-44822-9. In one embodiment of the invention, the total size of the quantum material is in the range from 1 to 100 nm, more preferably from 1 to 30 nm and particularly preferably from 5 to 15 nm. In this case, the core of the semiconducting light-emitting nano-particle can vary. Suitable examples are CdS, CdSe, CdTe, ZnS, ZnSe, ZnSeS, ZnTe, ZnO, GaAs, GaP, GaSb, HgS, HgSe, HgSe, HgTe, InAs, InP, InPS, InPZnS, InPZn, InPGa, InSb, AlAs , AlP, AlSb, Cu₂S, Cu₂Se, CuInS₂, CuInSe₂, Cu₂(ZnSn)S₄, Cu₂(InGa) S₄, TiO₂, or a combination of said materials. In a preferred embodiment, the core of the semiconductive light-emitting particle contains one or more elements of group 13 and one or more elements of group 15 of the periodic system of the elements, for example GaAs, GaP, GaSb, InAs, InP, InPS, InPZnS, InPZn, InPGa, InSb, AlAs, AlP, AlSb, CuInS₂, CuInSe₂, Cu₂(InGa)S₄ or a combination of the mentioned materials. Particularly preferably, the core contains In- and P-atoms, z. InP, InPS, InPZnS, InPZn or InPGa. In a further embodiment of the invention, the nanoparticle contains one or more shell layers, which comprise a first element from the group 12, 13 or 14 of the periodic table and a second element from the group 15 or 16 of the periodic table. Preferably, all shell layers contain a first element from the group 12, 13 or 14 of the periodic system and a second element from the group 15 or 16 of the periodic system. In a preferred embodiment of the invention, at least one of the shell layers contains a first element from the group 12 and a second element from the group 16 of the periodic table, for example CdS, CdZnS, ZnS, ZnSe, ZnSSe, ZnSSeTe, CdS/ZnS, ZnSe/ZnS or ZnS/ZnSe. Particularly preferably, all shell layers contain a first element from the group 12 and a second element from the group 16 of the periodic table.

Preference is furthermore given to an organic electroluminescent device, characterised in that one or more layers are applied by means of a sublimation process, in which the materials are vapour-deposited in vacuum sublimation units at an initial pressure of less than 10⁻⁵ mbar, preferably less than 10⁻⁶ mbar. However, it is also possible for the initial pressure to be even lower or higher, for example less than 10⁻⁷ mbar.

Preference is furthermore given to an organic electroluminescent device, characterised in that one or more layers are applied by means of a sublimation process, in which the materials are vapour-deposited in vacuum sublimation units at an initial pressure of less than 10⁻⁵ mbar, preferably less than 10⁻⁶ mbar. However, it is also possible for the initial pressure to be even lower or higher, for example less than 10⁻⁷ mbar.

Preference is likewise given to an organic electroluminescent device, characterised in that one or more layers are applied by means of the OVPD (organic vapour phase deposition) process or with the aid of carrier-gas sublimation, in which the materials are applied at a pressure between 10⁻⁵ mbar and 1 bar. A special case of this process is the OVJP (organic vapour jet printing) process, in which the materials are applied directly through a nozzle and thus structured (for example M. S. Arnold et al., Appl. Phys. Lett. 2008, 92, 053301).

Preference is furthermore given to an organic electroluminescent device, characterised in that one or more layers are produced from solution, such as, for example, by spin coating, or by means of any desired printing process, such as, for example, ink-jet printing, LITI (light induced thermal imaging, thermal transfer printing), screen printing, flexographic printing, offset printing or nozzle printing. Soluble compounds, which are obtained, for example, by suitable substitution, are necessary for this purpose.

Also possible are hybrid processes, in which, for example, one or more layers are applied from solution and one or more further layers are applied by vapour deposition. Thus, it is possible, for example, to apply the emitting layer from solution and to apply the electron-transport layer by vapour deposition.

These processes are generally known to the person skilled in the art and can be applied by him without inventive step to organic electroluminescent devices comprising the compounds according to the invention.

The compounds according to the invention generally have very good properties on use in organic electroluminescent devices. In particular, the lifetime on use of the compounds according to the invention in organic electroluminescent devices is significantly better compared with similar compounds in accordance with the prior art. The other properties of the organic electroluminescent device, in particular the efficiency and the voltage, are likewise better or at least comparable. Furthermore, the compounds have a high glass transition temperature and high thermal stability.

The present invention furthermore relates to the compounds of the following formulae (1'), 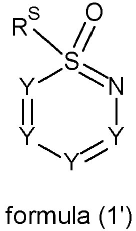 where the following applies to the symbols and indices used:
Y stands for CR^{Y} or N; with the proviso that at least two adjacent groups Y stand for a condensed aromatic or heteroaromatic ring system with the benzothiazine ring of formula (1');
R^{S}, R^{Y} stand on each occurrence, identically or differently, for H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R, where in each case one or more non-adjacent CH₂ groups may be replaced by RC=CR, C≡C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 60 aromatic ring atoms, which may be substituted by one or more radicals R;
   - where the radical R^{S} may form an aliphatic, aromatic or heteroaromatic ring system ring with a radical R^{Y} or with the adjacent ring formed by two groups Y, which may be substituted by one or more radicals R; and
   - where two adjacent radicals R^{Y} may form an aliphatic, aromatic or heteroaromatic ring system together, which may be substituted by one or more radicals R;
R stands on each occurrence, identically or differently, for H, D, F, Cl, Br, I, CHO, CN, C(=O)Ar, P(=O)(Ar)₂, S(=O)Ar, S(=O)₂Ar, N(R')₂, N(Ar)₂, NO₂, Si(R')₃, B(OR')₂, OSO₂R', a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R', where in each case one or more non-adjacent CH₂ groups may be replaced by R'C=CR', C≡C, Si(R')₂, Ge(R')₂, Sn(R')₂, C=O, C=S, C=Se, P(=O)(R'), SO, SO₂, O, S or CONR' and where one or more H atoms may be replaced by D, F, Cl, Br, I, CN or NO₂, an aromatic or heteroaromatic ring systems having 5 to 60 aromatic ring atoms, which may in each case be substituted by one or more radicals R', or an aryloxy group having 5 to 60 aromatic ring atoms, which may be substituted by one or more radicals R'; where two adjacent substituents R may form an aliphatic or aromatic ring system together, which may be substituted by one or more radicals R';
Ar is, on each occurrence, identically or differently, an aromatic or heteroaromatic ring system having 5 to 60 aromatic ring atoms, which may in each case also be substituted by one or more radicals R'; and
R' stands on each occurrence, identically or differently, for H, D, F, Cl, Br, I, CN, a straight-chain alkyl, alkoxy or thioalkyl group having 1 to 20 C atoms or abranched or cyclic alkyl, alkoxy or thioalkyl group having 3 to 20 C atoms, where in each case one or more non-adjacent CH₂ groups may be replaced by SO, SO₂, O, S and where one or more H atoms may be replaced by D, F, Cl, Br or I, or an aromatic or heteroaromatic ring system having 5 to 24 aromatic ring atoms;
characterized in that it the compounds of formulae (1') comprise at least one radical, which stands for an aromatic or heteroaromatic ring system selected from the groups of formula (R-2), 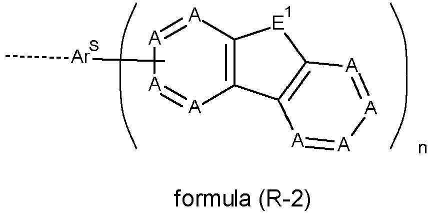 where the dashed bond indicates the bond to the structure, and where:
   Ar^{S} stands for a single bond or for an aromatic or heteroaromatic ring system having 5 to 30 aromatic ring atoms, which may be substituted by one or more radicals R;
   A stands, identically or differently on each occurrence, for CR^{A} or N; or two adjacent groups A stand for a group of the formula (Ar-5) or (Ar-6), 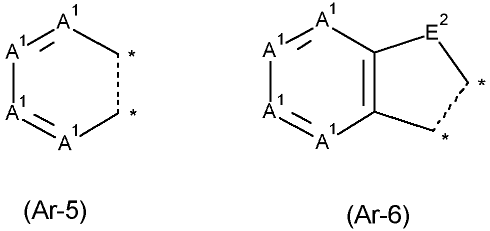
   where the dashed bonds between the signs "*" correspond to the common bond of the group of formula (Ar-5) or (Ar-6) with the group of formula (R-2), and
   where:
      E¹, E² stands for C(R⁰)₂, C=O, NR^{N}, O or S;
      A¹ stands, identically or differently on each occurrence, for CR^{A1} or N;
      R⁰, R^{N} are on each occurrence, identically or differently, for H, D, F, Cl, Br, I, CN, Si(R)₃, a straight-chain alkyl, alkoxy or thioalkyl group having 1 to 40, preferably 1 to 20, more preferably 1 to 10 C atoms or a branched or a cyclic alkyl, alkoxy or thioalkyl group having 3 to 40, preferably 3 to 20, more preferably 3 to 10 C atoms, each of which may be substituted by one or more radicals R, where in each case one or more non-adjacent CH₂ groups may be replaced by RC=CR, C≡C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60, preferably 5 to 40, more preferably 5 to 30, particularly preferably 5 to 24, very particularly preferably 5 to 18 aromatic ring atoms, which may in each case be substituted by one or more radicals R, or an aryloxy group having 5 to 60, preferably 5 to 40, more preferably 5 to 30, particularly preferably 5 to 24, very particularly preferably 5 to 18 aromatic ring atoms, which may be substituted by one or more radicals R, where two adjacent substituents R⁰ may form an aliphatic, aromatic or heteroaromatic ring system together, which may be substituted by one or more radicals R;
      R^{A}, R^{A1} are on each occurrence, identically or differently, for H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R, where in each case one or more non-adjacent CH₂ groups may be replaced by RC=CR, C≡C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 60 aromatic ring atoms, which may be substituted by one or more radicals R; where two adjacent substituents R^{A} may form an aliphatic, aromatic or heteroaromatic ring system together, which may be substituted by one or more radicals R; and where two adjacent substituents R^{A1} may form an aliphatic, aromatic or heteroaromatic ring system together, which may be substituted by one or more radicals R; and
      n is an integer equal to 1, 2, 3 or 4.

Preferably, the compounds of formula (1') are selected from the compound of formulae (2-1') to (4-5'): 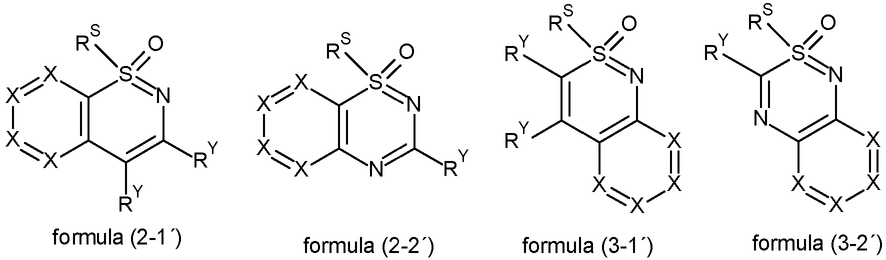 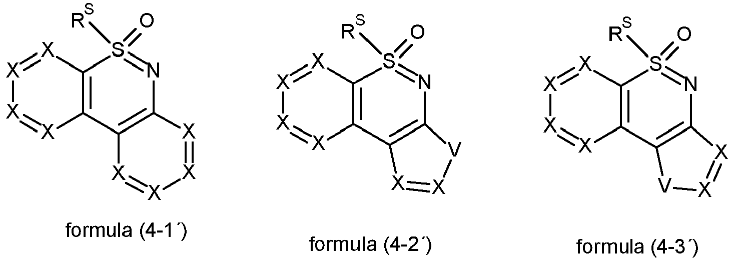 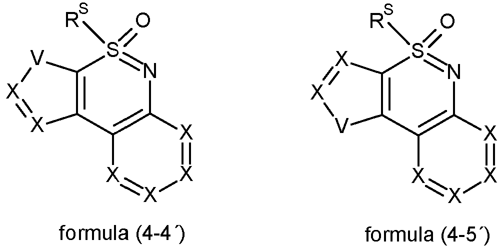 where the symbol R^{S}, R^{Y} have the same meaning as above and where:
V stands for C(R⁰)₂, NR^{N}, O or S; preferably for NR^{N}, O or S, more preferably for NR^{N};
X stands, identically or differently on each occurrence, for CR^{X} or N; or two adjacent groups X stand for a group of the formula (Ar-3) or (Ar-4), 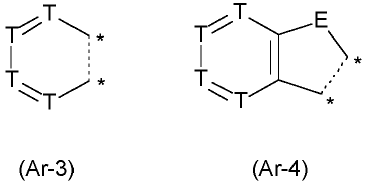 where the dashed bonds between the signs "*" correspond to the common bond of the group of formula (Ar-3) or (Ar-4) with the rest of the structure, and furthermore:
   - E stands for C(R⁰)₂, C=O, NR^{N}, O or S; preferably for NR^{N}, O or S, more preferably for NR^{N}; where R^{N} and R⁰ have the same meaning as above;
   - T stands, identically or differently on each occurrence, for CR^{T} or N;
R^{X}, R^{T} stand on each occurrence, identically or differently, for H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R, where in each case one or more non-adjacent CH₂ groups may be replaced by RC=CR, C≡C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 60 aromatic ring atoms, which may be substituted by one or more radicals R; where two adjacent substituents R^{T} may form an aliphatic, aromatic or heteroaromatic ring system together, which may be substituted by one or more radicals R; and where two adjacent substituents R^{X} may form an aliphatic, aromatic or heteroaromatic ring system together, which may be substituted by one or more radicals R;
characterized in that the compounds of formulae (2-1') to (4-5') comprise at least one radical R^{S}, R^{X}, R^{Y}, R^{T} or R, preferably R^{X}, R^{Y}, R^{T} or R, which stands for a group of formula (R-2) as defined above.

More preferably, the compounds of formulae (2-1') to (4-5') are selected from compounds of formulae (2-1-1') to (4-5-3') as depicted below: 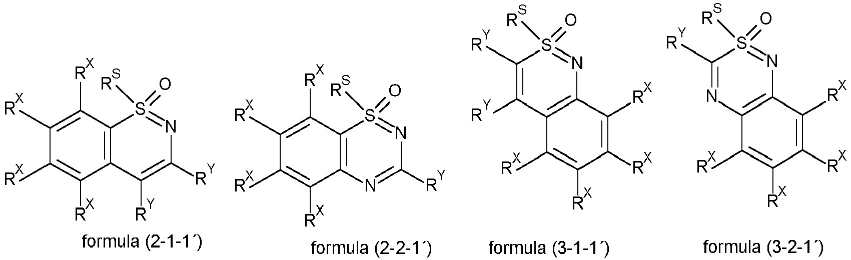 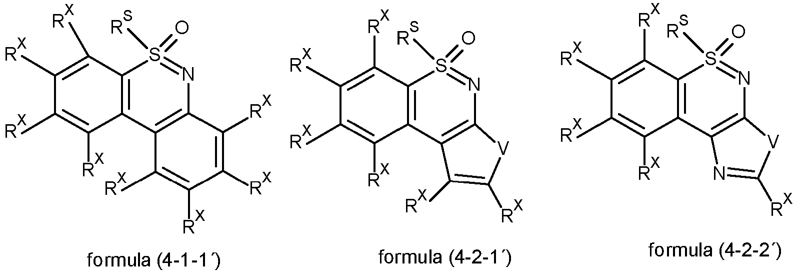 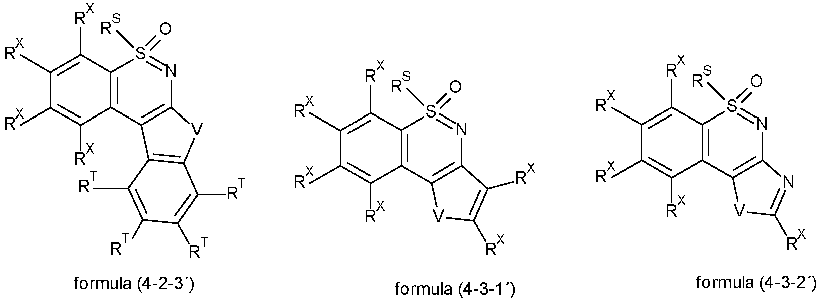 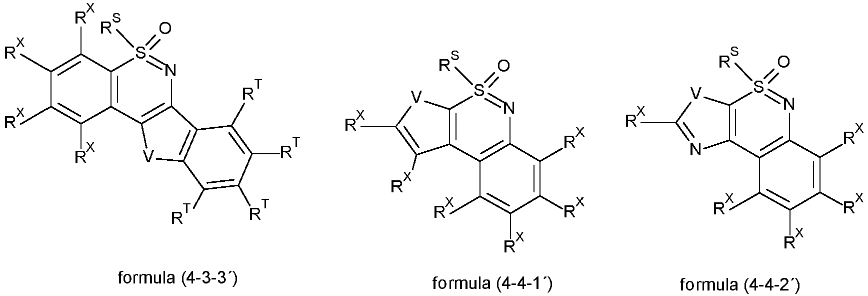 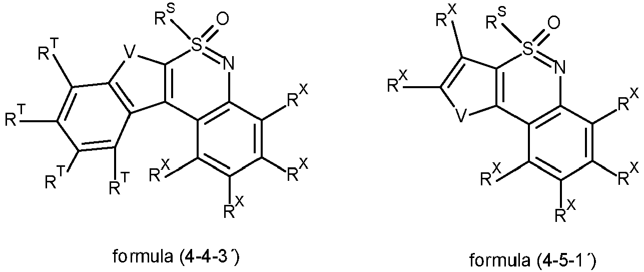 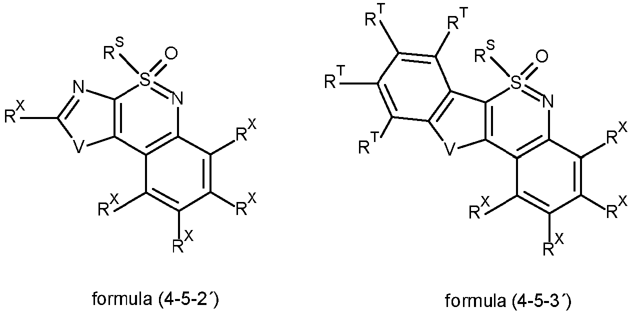 where the symbols have the same meaning as above and where the compounds of formulae (2-1-1') to (4-5-3') comprise at least one radical R^{S}, R^{X}, R^{Y}, R^{T} or R, preferably R^{X}, R^{Y}, R^{T} or R, more preferably R^{X}, R^{Y} or R^{T}, which stands for a group of formula (R-2).

More specifically, it is preferred that the compounds of formulae (2-1-1'), (2-2-1'), (3-1-1') and (3-2-1') comprise at least one radical R^{X}, R^{Y} or R, preferably one radical R^{X} or R^{Y}, which stands for a group of formula (R-2). It is preferred that the compounds of formulae (4-1-1'), (4-2-1'), (4-2-2'), (4-3-1'), (4-3-2'), (4-4-1'), (4-4-2'), (4-5-1') and (4-5-2') comprise at least one radical R^{X} or R, preferably R^{X}, which stands for a group of formula (R-2). And it is preferred that the compounds of formulae (4-2-3'), (4-3-3'), (4-4-3') and (4-5-3') comprise at least one radical R^{X}, R^{T} or R, preferably R^{X} or R^{T}, which stands for a group of formula (R-2) as defined above.

The same preferences as indicated above for the compounds of the formula (1), the symbols and indices apply to the compounds of the formula (1') according to the invention.

The compounds of the formula (1) or (1') or the preferred embodiments can be prepared by synthetic steps known to the person skilled in the art, as depicted schematically in Scheme 1 to 8.

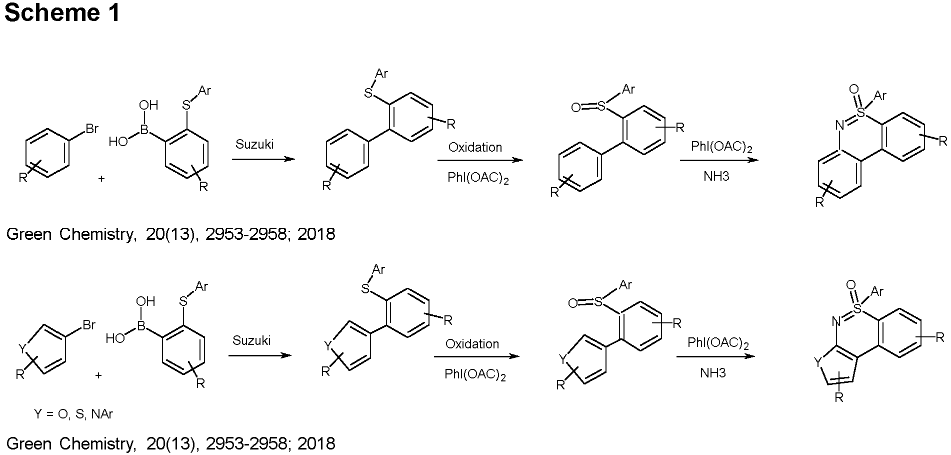

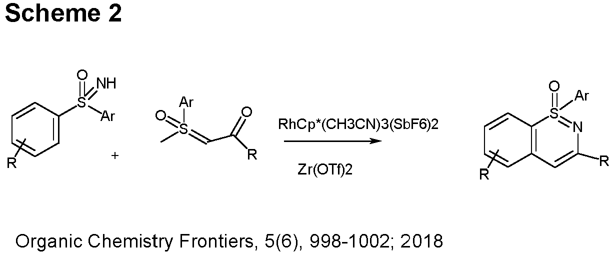

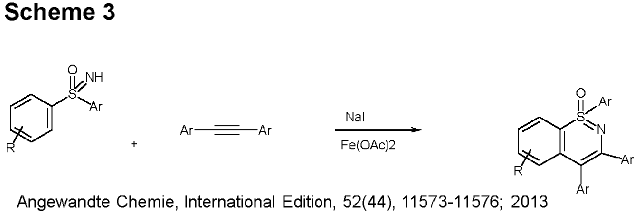

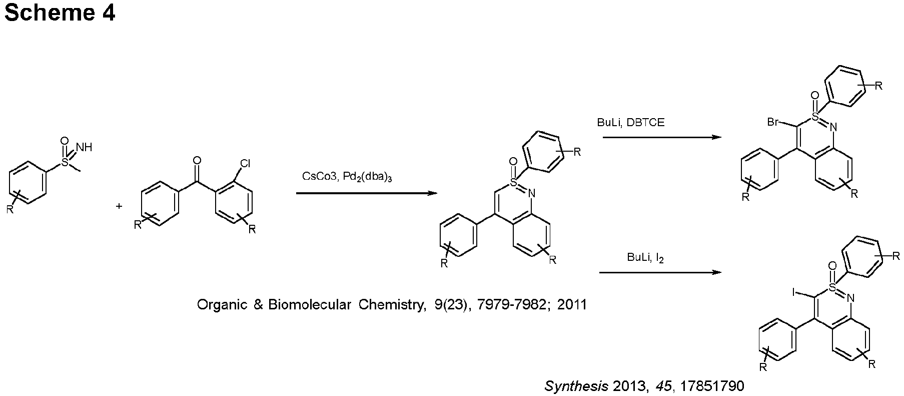

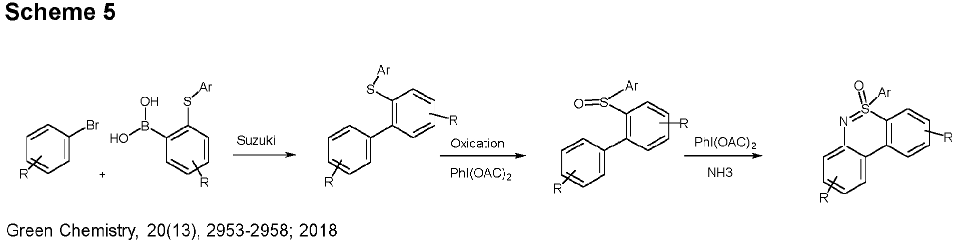

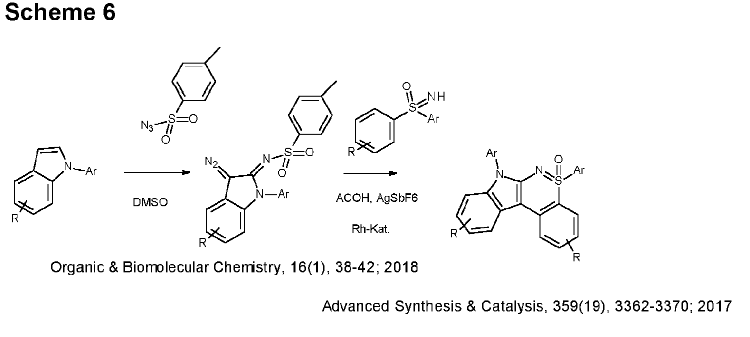

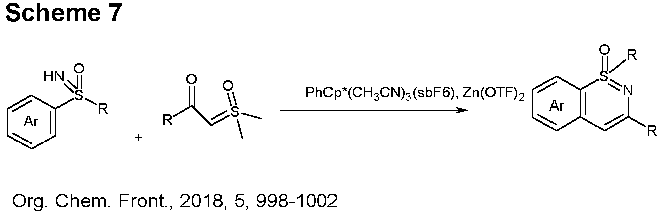

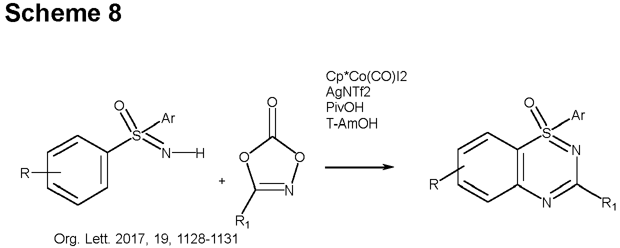

In schemes 1-8, the group Ar represents an aromatic or heteroaromatic ring system and the groups R and R¹ represent a radical.

R and R¹ may also correspond to a leaving group, preferably selected from halogens (like Cl, Br, I), boronic acid or boronic acid derivatives, triflate and tosylate. The functionalized compounds, namely the compounds substituted by a leaving group, in particular brominated compounds represent the central building block for further functionalisation. Thus, these functionalised compounds can easily be converted into compounds of the formula (1) or formula (1') by Suzuki coupling or coupling to diarylamines by the Hartwig-Buchwald method with carbazole derivatives or triarylamine derivatives.

The brominated compounds can furthermore be lithiated and converted into ketones by reaction with electrophiles, such as benzonitrile, and subsequent acidic hydrolysis or into phosphine oxides with chlorodiphenylphosphines and subsequent oxidation.

The compounds according to the invention described above, in particular compounds which are substituted by reactive leaving groups, such as bromine, iodine, chlorine, boronic acid or boronic acid ester, or by reactive, polymerisable groups, such as olefins or oxetanes, can be used as monomers for the generation of corresponding oligomers, dendrimers or polymers. The oligomerisation or polymerisation here preferably takes place via the halogen functionality or the boronic acid functionality or via the polymerisable group. It is furthermore possible to crosslink the polymers via groups of this type. The compounds and polymers according to the invention can be employed as crosslinked or uncrosslinked layer.

Oligomers, polymers or dendrimers comprising one or more of the compounds according to the invention mentioned above, are such that one or more bonds are present from the compound according to the invention to the polymer, oligomer or dendrimer. Depending on the linking of the compound according to the invention, this therefore forms a side chain of the oligomer or polymer or is linked in the main chain. The polymers, oligomers or dendrimers may be conjugated, partially conjugated or non-conjugated. The oligomers or polymers may be linear, branched or dendritic. The same preferences as described above apply to the recurring units of the compounds according to the invention in oligomers, dendrimers and polymers.

For the preparation of the oligomers or polymers, the monomers according to the invention are homopolymerised or copolymerised with further monomers. Preference is given to homopolymers or copolymers in which the units of the formula (1), (1') or the above-mentioned preferred embodiments are present to the extent of 0.01 to 99.9 mol%, preferably 5 to 90 mol%, particularly preferably 20 to 80 mol%. Suitable and preferred comonomers which form the polymer backbone are selected from fluorenes (for example in accordance with EP 842208 or WO 2000/22026), spirobifluorenes (for example in accordance with EP 707020, EP 894107 or WO 2006/061181), para-phenylenes (for example in accordance with WO 92/18552), carbazoles (for example in accordance with WO 2004/070772 or WO 2004/113468), thiophenes (for example in accordance with EP 1028136), dihydrophenanthrenes (for example in accordance with WO 2005/014689), cis- and trans-indenofluorenes (for example in accordance with WO 2004/041901 or WO 2004/113412), ketones (for example in accordance with WO 2005/040302), phenanthrenes (for example in accordance with WO 2005/104264 or WO 2007/017066) or also a plurality of these units. The polymers, oligomers and dendrimers may also comprise further units, for example hole-transport units, in particular those based on triarylamines, and/or electron-transport units. In addition, the polymers may comprise triplet emitters, either copolymerised or mixed in as a blend. In particular, the combination of units of the formula (1), (1') or the above-mentioned preferred embodiments with triplet emitters results in particularly good results.

Furthermore, the compounds of the formulae (1), (1') or the above-mentioned preferred embodiments may also be functionalised further and thus converted into extended structures. The reaction with arylboronic acids by the Suzuki method or with primary or secondary amines by the Hartwig-Buchwald method may be mentioned here as an example. Thus, the compounds of the formulae (1), (1') or the above-mentioned preferred embodiments may also be bonded directly to phosphorescent metal complexes or also to other metal complexes.

For the processing of the compounds according to the invention from the liquid phase, for example by spin coating or by printing processes, formulations of the compounds according to the invention are necessary. These formulations can be, for example, solutions, dispersions or emulsions. It may be preferred to use mixtures of two or more solvents for this purpose. The solvents are preferably selected from organic and inorganic solvents, more preferably organic solvents. The solvents are very preferably selected from hydrocarbons, alcohols, esters, ethers, ketones and amines. Suitable and preferred solvents are, for example, toluene, anisole, o-, m- or p-xylene, methyl benzoate, mesitylene, tetralin, veratrole, THF, methyl-THF, THP, chlorobenzene, dioxane, phenoxytoluene, in particular 3-phenoxytoluene, (-)-fenchone, 1,2,3,5-tetramethylbenzene, 1,2,4,5-tetramethylbenzene, 1-methylnaphthalene, 1-ethylnaphthalene, decylbenzene, phenyl naphthalene, menthyl isovalerate, para tolyl isobutyrate, cyclohexal hexanoate, ethyl para toluate, ethyl ortho toluate, ethyl meta toluate, decahydronaphthalene, ethyl 2-methoxybenzoate, dibutylaniline, dicyclohexylketone, isosorbide dimethyl ether, decahydronaphthalene, 2-methylbiphenyl, ethyl octanoate, octyl octanoate, diethyl sebacate, 3,3-dimethylbiphenyl, 1,4-dimethylnaphthalene, 2,2'-dimethylbiphenyl, 2-methylbenzothiazole, 2-phenoxyethanol, 2-pyrrolidinone, 3-methylanisole, 4-methylanisole, 3,4-dimethylanisole, 3,5-dimethylanisole, acetophenone, α-terpineol, benzothiazole, butyl benzoate, cumene, cyclohexanol, cyclohexanone, cyclohexylbenzene, decalin, dodecylbenzene, ethyl benzoate, indane, NMP, p-cymene, phenetole, 1,4-diisopropylbenzene, dibenzyl ether, diethylene glycol butyl methyl ether, triethylene glycol butyl methyl ether, diethylene glycol dibutyl ether, triethylene glycol dimethyl ether, diethylene glycol monobutyl ether, tripropylene glycol dimethyl ether, tetraethylene glycol dimethyl ether, 2-isopropylnaphthalene, pentylbenzene, hexylbenzene, heptylbenzene, octylbenzene, 1,1-bis(3,4-dimethylphenyl)ethane or mixtures of these solvents.

The present invention therefore furthermore relates to a formulation comprising a compound according to the invention and at least one further compound. The further compound may be, for example, a solvent, in particular one of the above-mentioned solvents or a mixture of these solvents. However, the further compound may also be at least one further organic or inorganic compound which is likewise employed in the electronic device, for example an emitting compound, in particular a phosphorescent dopant, and/or a further matrix material. Suitable emitting compounds and further matrix materials are indicated below in connection with the organic electroluminescent device. This further compound may also be polymeric.

The compounds (1') and mixtures according to the invention are suitable for use in an electronic device. An electronic device here is taken to mean a device which comprises at least one layer which comprises at least one organic compound. However, the component here may also comprise inorganic materials or also layers built up entirely from inorganic materials.

The present invention therefore furthermore relates to the use of the compounds (1') or mixtures according to the invention in an electronic device. Preferably, the electronic device comprising the compounds of formula (1') is selected from the group consisting of organic electroluminescent devices, organic integrated circuits, organic field-effect transistors, organic thin-film transistors, organic light-emitting transistors, organic solar cells, dye-sensitised organic solar cells, organic optical detectors, organic photoreceptors, organic field-quench devices, light-emitting electrochemical cells, organic laser diodes and organic plasmon emitting devices. More preferably, the electronic device comprising the compounds of formula (1') is an organic electroluminescent device. Particularly preferably, the compound of formula (1') is employed as a matrix material for emitters, a hole-transport-material or an electron-transport material in an organic electroluminescent device, more preferably as a matrix material for a phosphorescent emitter.

The same preferences as indicated above for an organic electroluminescent device comprising the compounds of the formula (1) apply to an organic electroluminescent device comprising the compounds of the formula (1').

The invention will now be explained in greater detail by the following examples, without wishing to restrict it thereby.

### A) Syntheses examples:

Unless otherwise stated, the following syntheses are carried out in a protective gas atmosphere in dried solvents. The solvents and reagents can be obtained from Sigma-ALDRICH or ABCR. The CAS numbers of the compounds known from the literature are also indicated below. The compounds according to the invention can be synthesised by means of synthesis methods known to the skilled person.

### a) Suzuki-Reaction

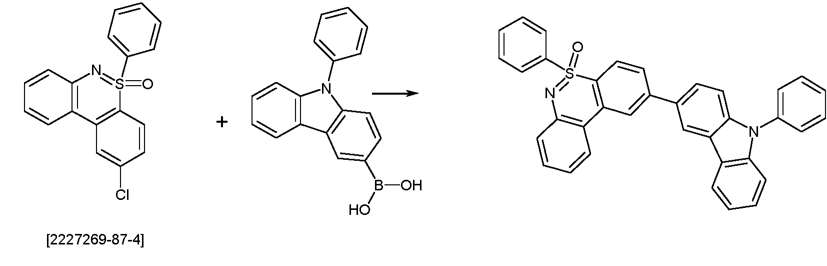

52.12 g (160 mmol) of the dibenzothiazine derivative, 50 g (172 mmol) of N-phenyl-carbazole-3-boronic acid and 36 g (340 mmol) of sodium carbonate are suspended in 1000 mL of ethylene glycol dimethyl ether and 280 mL of water. Afterwards, 1.8 g (1.5 mmol) of tetrakis(triphenylphosphine)-palladium(0) is added to the reaction mixture, which is then mixed for 16 h under reflux. After cooling, the organic phase is separated, filtered over silica gel, washed three times with 200 mL water and then concentrated to dryness. The yield is 51 g (96 mmol), corresponding to 60% of the theory.

The following compounds can be prepared analogously:

| | **Educt 1** | **Educt 2** | **Product** | **Yield** |
|---|---|---|---|---|
| **1a** | 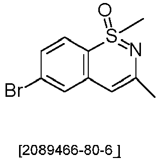 | 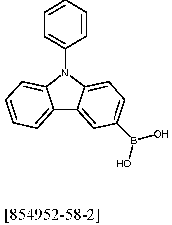 | 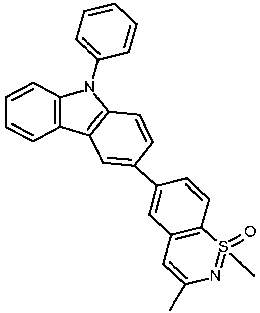 | 66% |
| **2a** | 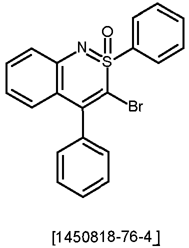 | 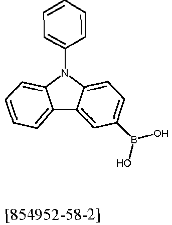 | 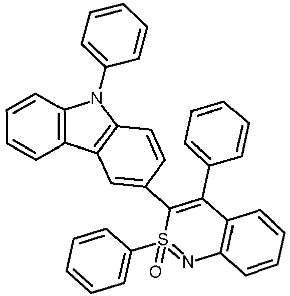 | **61%** |
| **3a** | 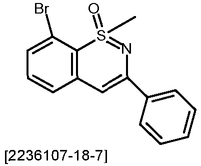 | 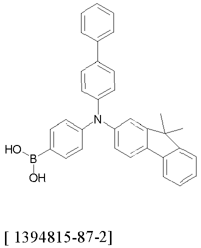 | 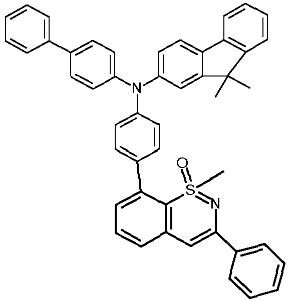 | **67%** |
| **4a** | 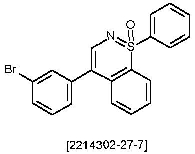 | 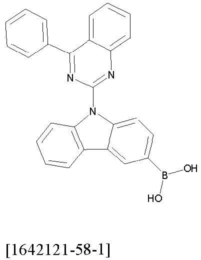 | 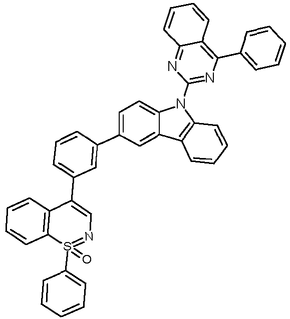 | **58%** |
| **5a** | 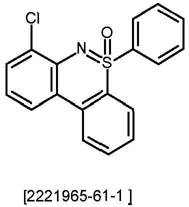 | 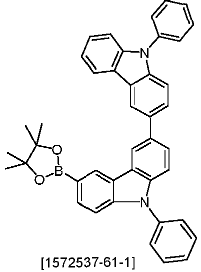 | 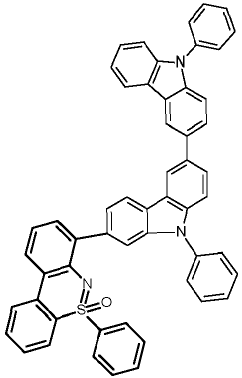 | **75%** |
| **6a** | 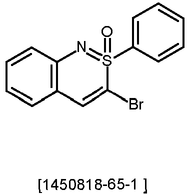 | 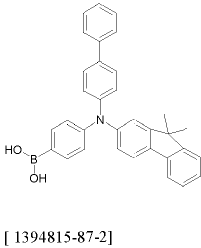 | 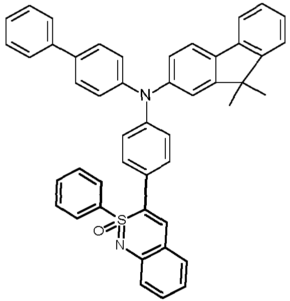 | **54%** |
| **7a** | 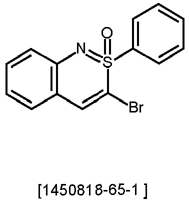 | 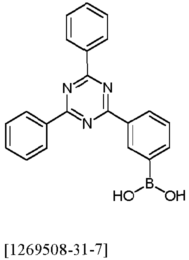 | 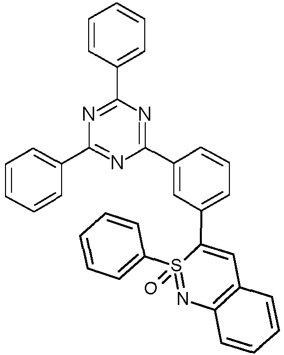 | **66%** |
| **8a** | 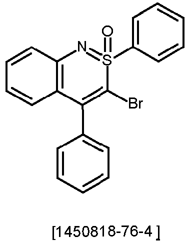 | 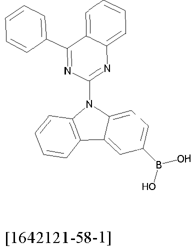 | 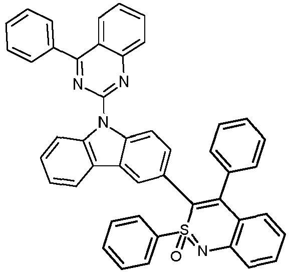 | **71%** |
| **9a** | 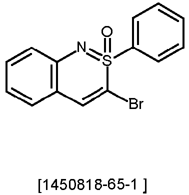 | 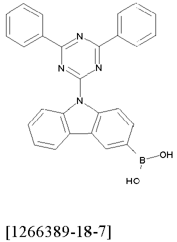 | 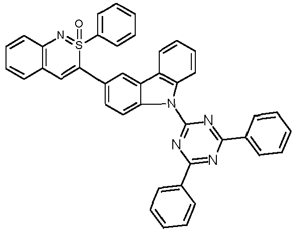 | **70%** |
| **10a** | 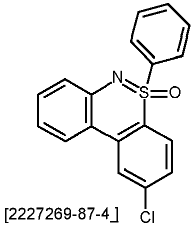 | 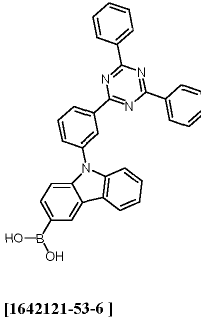 | 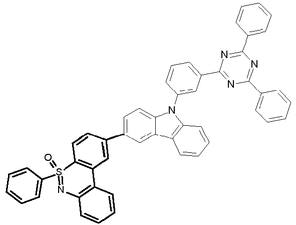 | **60%** |
| **11a** | 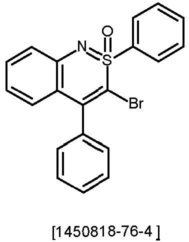 | 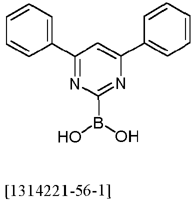 | 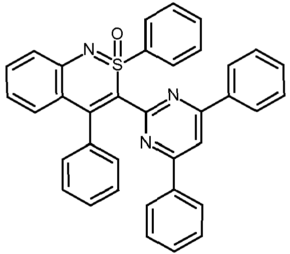 | **65%** |
| **12a** | 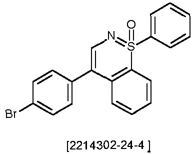 | 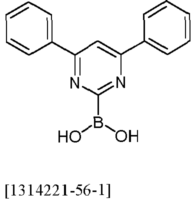 | 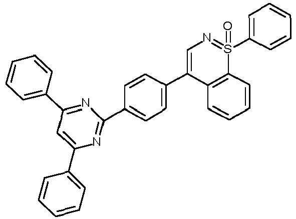 | **72%** |
| **13a** | 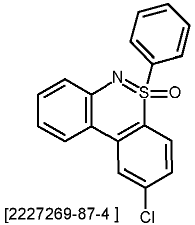 | 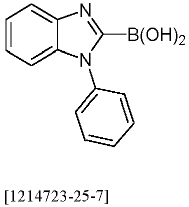 | 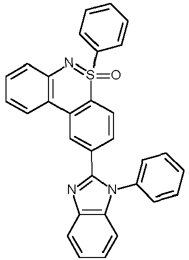 | **49%** |
| **14a** | 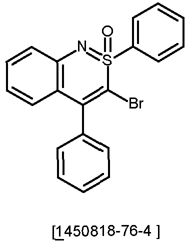 | 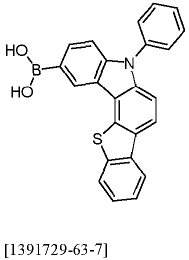 | 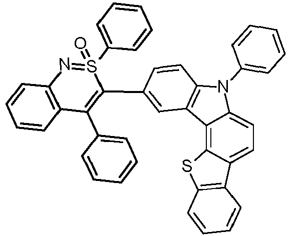 | **67%** |
| **15a** | 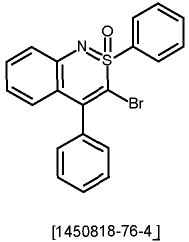 | 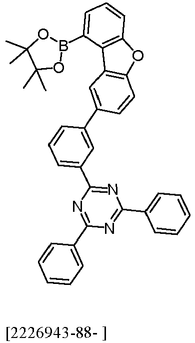 | 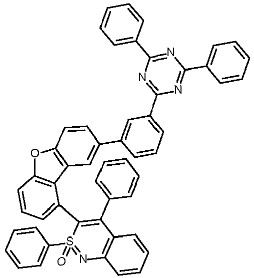 | **74%** |
| **16a** | 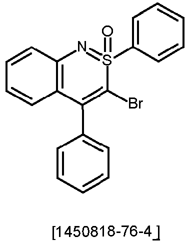 | 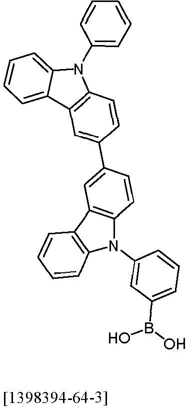 | 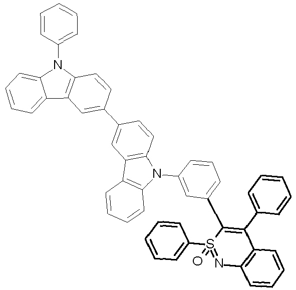 | **78%** |
| **17a** | 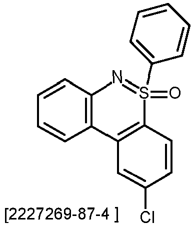 | 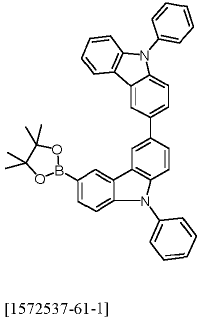 | 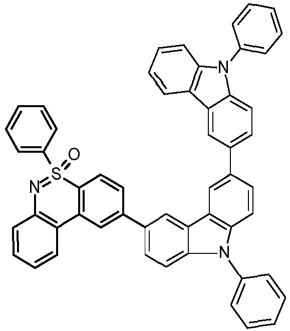 | **56%** |
| **18a** | 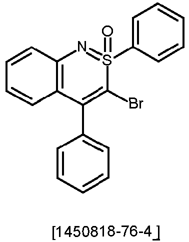 | 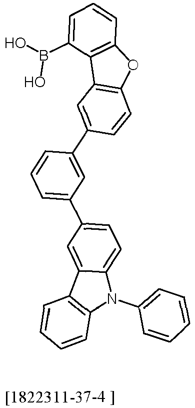 | 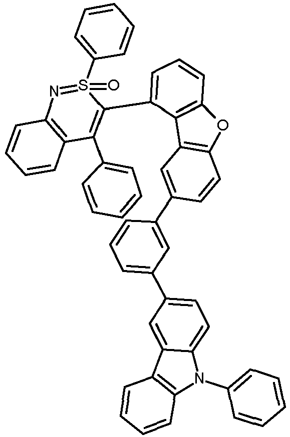 | **59%** |
| **19a** | 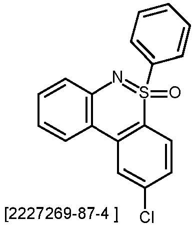 | 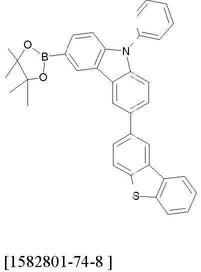 | 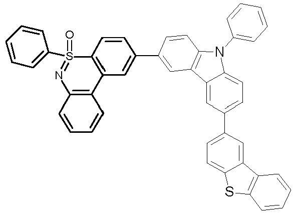 | **57%** |
| **20a** | 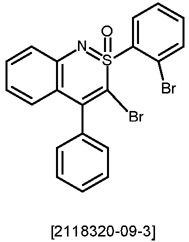 | 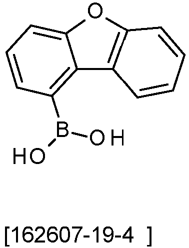 | 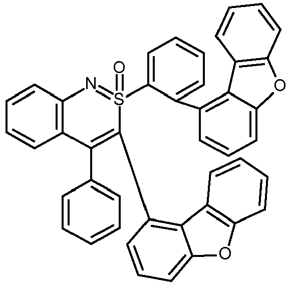 | **81%** |
| **21a** | 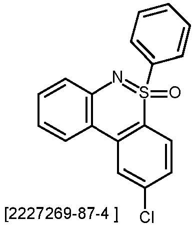 | 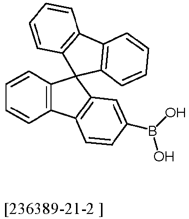 | 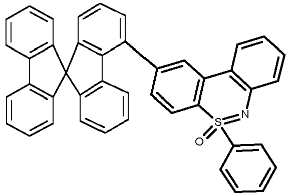 | **53%** |
| **22a** | 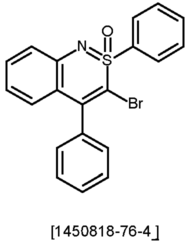 | 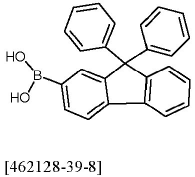 | 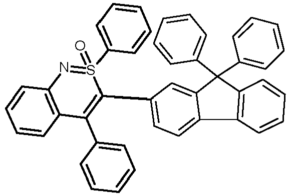 | **75%** |
| **23a** | 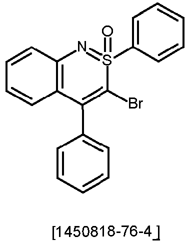 | 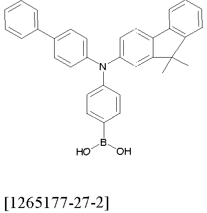 | 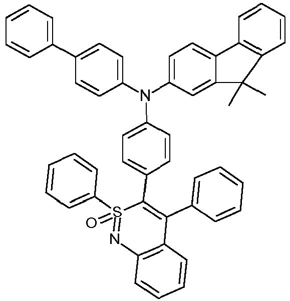 | **76%** |
| **24a** | 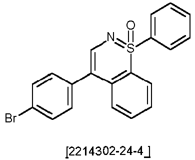 | 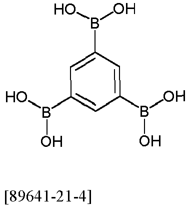 | 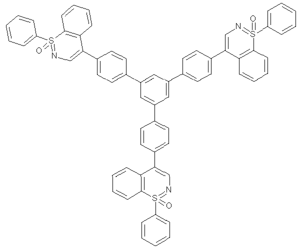 | **76%** |
| **25a** | 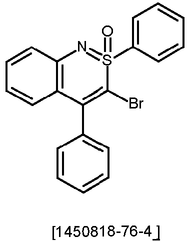 | 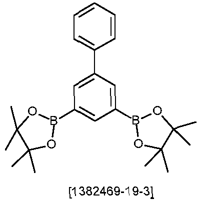 | 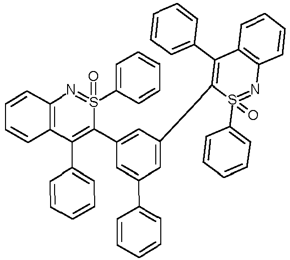 | **79%** |
| **26a** | 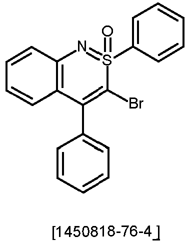 | 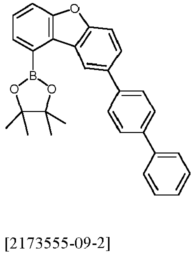 | 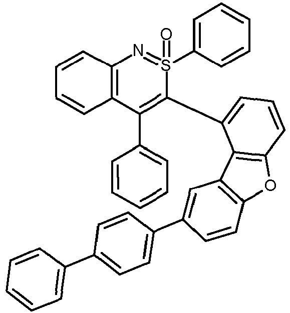 | **73%** |
| **27a** | 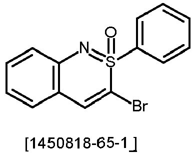 | 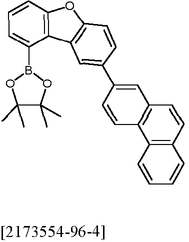 | 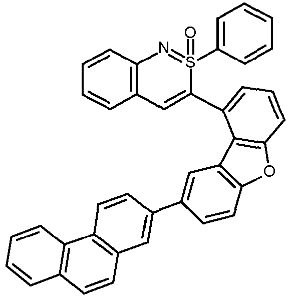 | **80%** |
| **28a** | 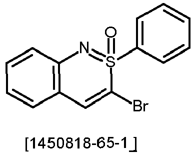 | 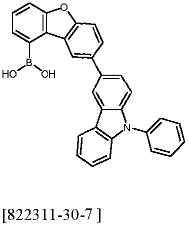 | 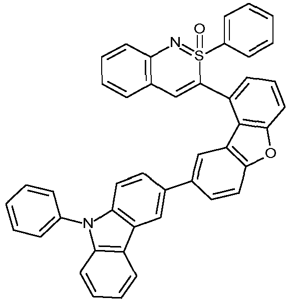 | **81%** |
| **29a** | 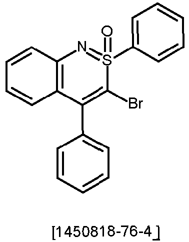 | 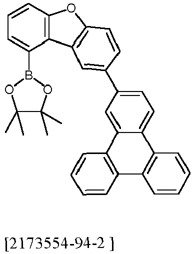 | 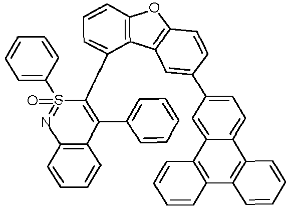 | **66%** |
| **30a** | 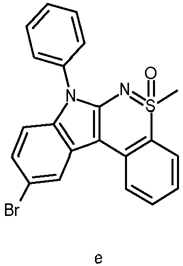 | 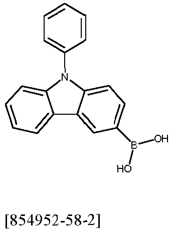 | 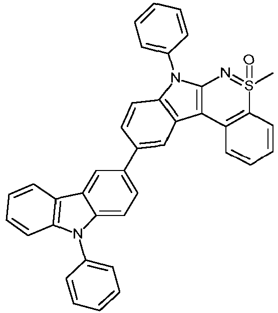 | **59%** |
| **31a** | 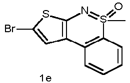 | 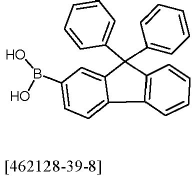 | 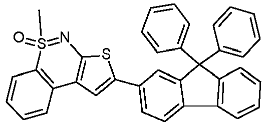 | **67%** |
| **32a** | 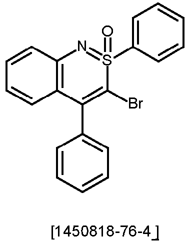 | 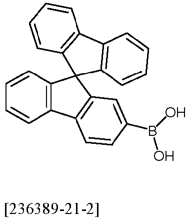 | 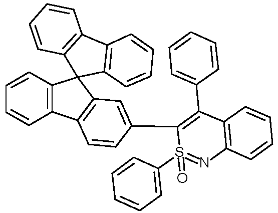 | **81%** |
| **33a** | 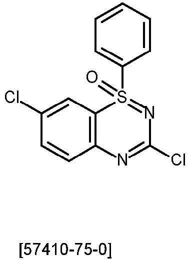 | 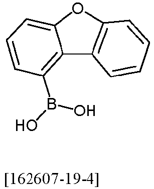 | 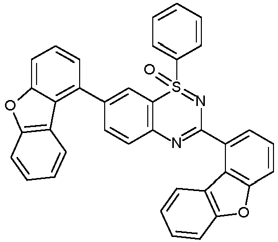 | **78%** |
| **34a** | 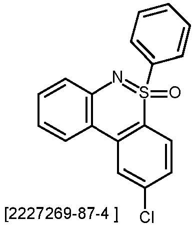 | 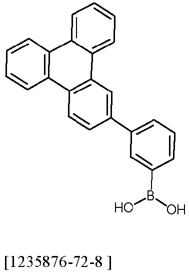 | 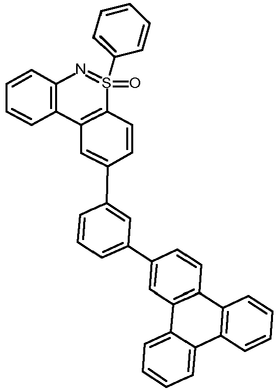 | **81%** |
| **35a** | 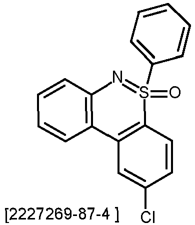 | 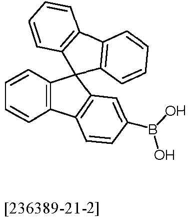 | 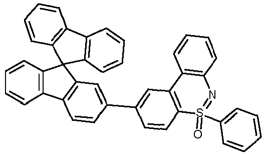 | **83%** |
| **36a** | 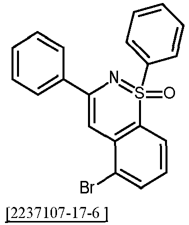 | 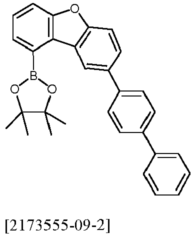 | 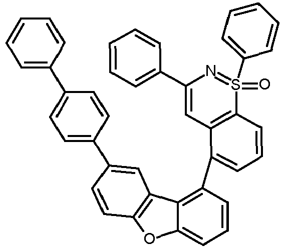 | **80%** |
| **37a** | 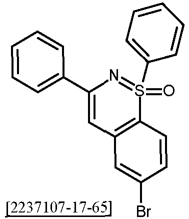 | 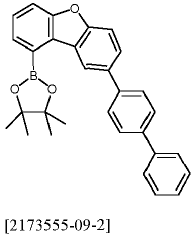 | 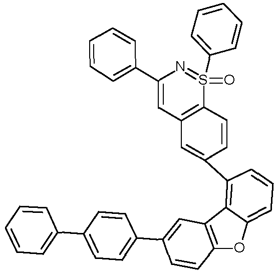 | **79%** |
| **38a** | 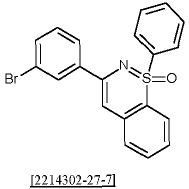 | 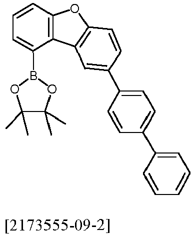 | 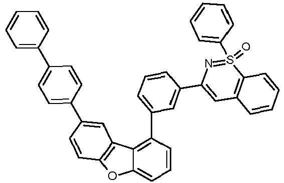 | **86%** |
| **39a** | 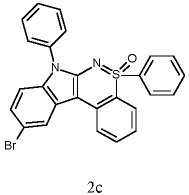 | 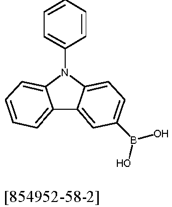 | 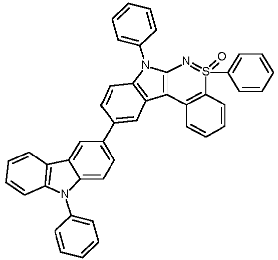 | **79%** |
| **40a** | 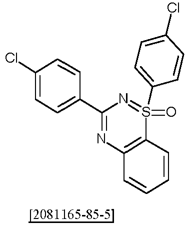 | 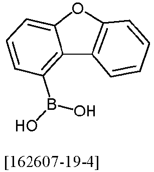 | 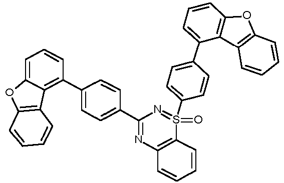 | **91%** |
| **41a** | 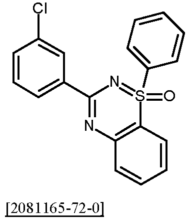 | 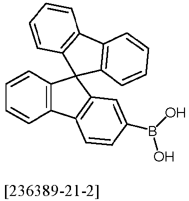 | 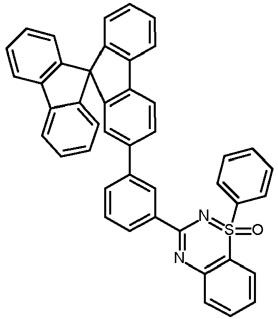 | **99%** |
| **42a** | 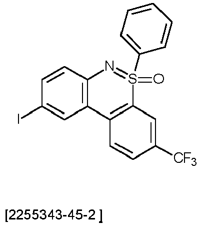 | 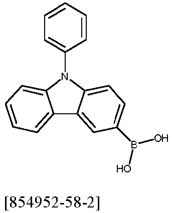 | 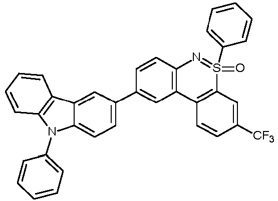 | **87%** |
| **43a** | 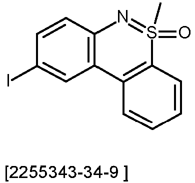 | 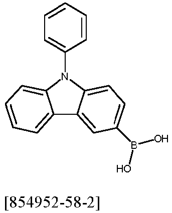 | 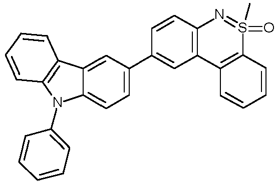 | **87%** |
| **44a** | 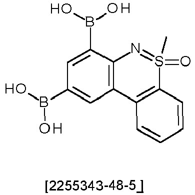 | 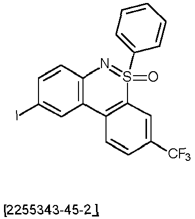 | 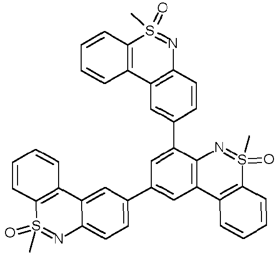 | **80%** |
| **45a** | 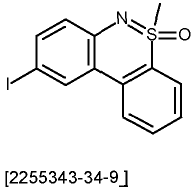 | 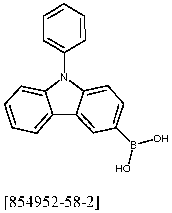 | 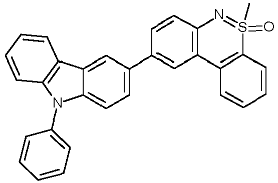 | **83%** |
| **46a** | 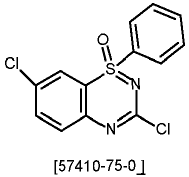 | 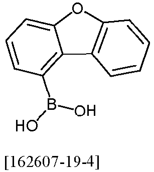 | 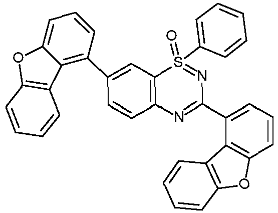 | **78%** |
| **47a** | 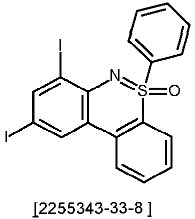 | 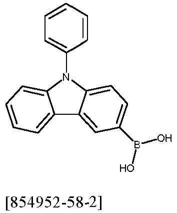 | 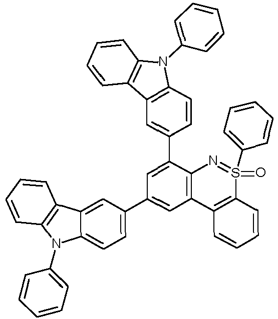 | **67%** |

### b) Buchwald

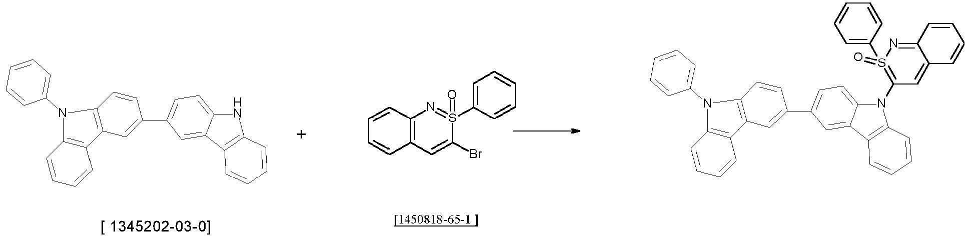

20.4 g (50 mmol) of 9-phenyl-3,3'-bis-9H-carbazole and 16 g (54 mmol) of 3-bromo-2-phenyl-2λ⁴-2,1-benzothiazine-2-oxide are dissolved in 400 ml of toluene in an argon atmosphere. Then, 1.0 g (5 mmol) of tri-tert-butyl-phosphine is added to the reaction mixture, which is stirred in an argon atmosphere. Then, 0.6 g (2 mmol) of Pd(OAc)2 is added to the reaction mixture, which is stirred in an argon atmosphere, after which 9,5 g (99 mmol) of sodium-tert-butanolate are added to the mixture. The reaction mixture is stirred under reflux for 24 hours. After cooling, the organic phase is separated, washed three times with 200 ml water, dried over MgSO4, filtered and finally, the solvent is removed under vacuum. The residue is purified by column chromatography over silica gel (eluent: DCM/Heptane (1:3)). The residue is hot extracted with toluene and recrystallized from toluene/n-heptane and finally sublimated in high vacuum. The yield is 29 g (45 mmol), corresponding to 90 % of the theory.

The following compounds can be produced analogously:

| | **Educt 1** | **Educt 2** | **Product** | **Yield** |
|---|---|---|---|---|
| 1b | 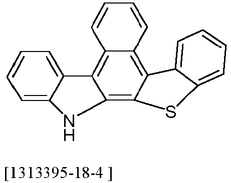 | 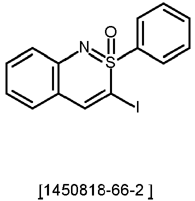 | 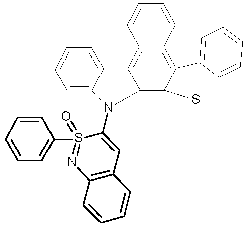 | 71% |

| | | | | |
|---|---|---|---|---|
| 2b | 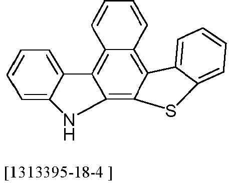 | 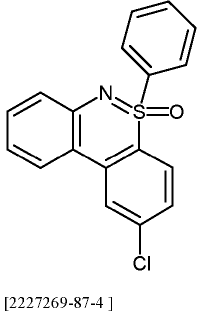 | 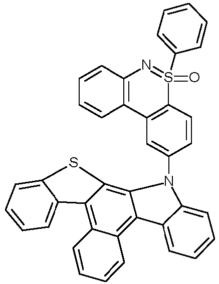 | 82% |
| 3b | 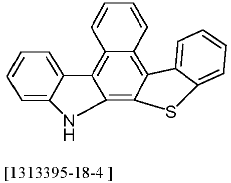 | 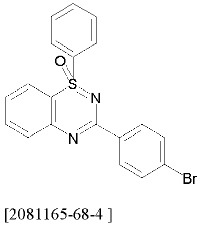 | 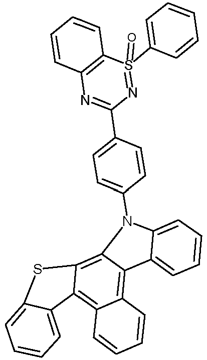 | 67% |
| 4b | 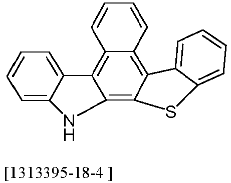 | 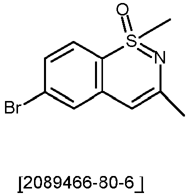 | 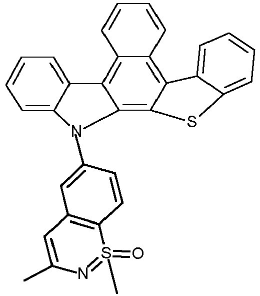 | 73% |

| | | | | |
|---|---|---|---|---|
| 5b | 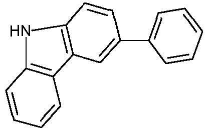 | 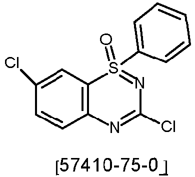 | 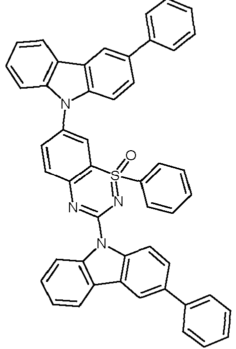 | 65% |
| 6b | 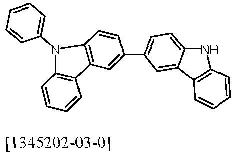 | 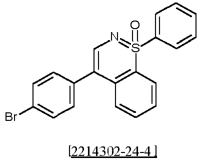 | 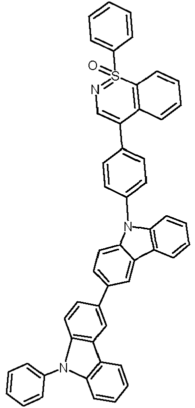 | 85% |
| 7b | 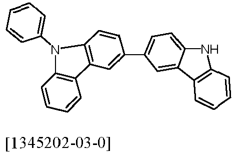 | 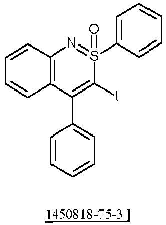 | 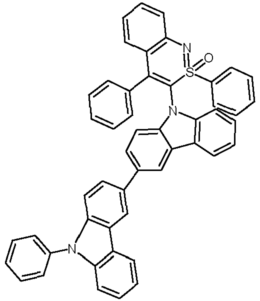 | 81% |

| | | | | |
|---|---|---|---|---|
| 8b | 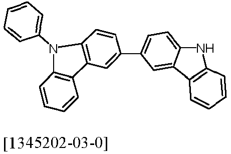 | 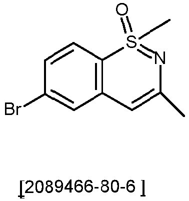 | 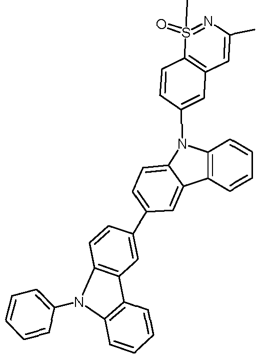 | 77% |
| 9b | 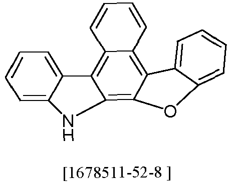 | 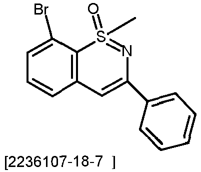 | 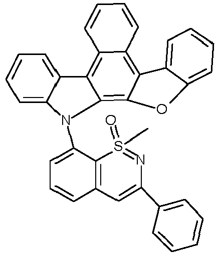 | 69% |
| 10b | 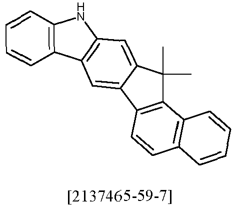 | 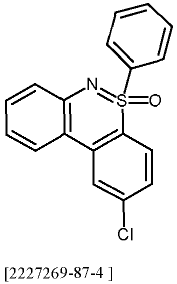 | 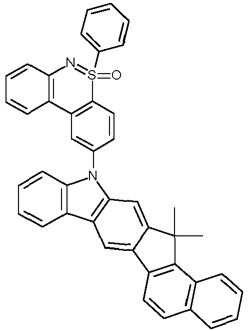 | 84% |
| 11b | 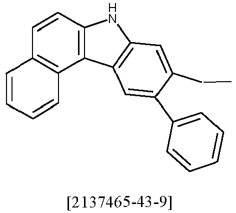 | 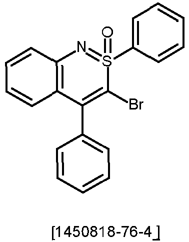 | 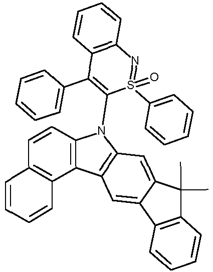 | 83% |

| | | | | |
|---|---|---|---|---|
| 12b | 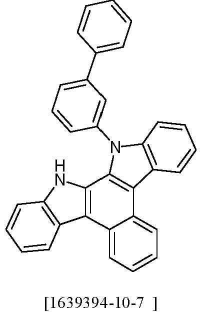 | 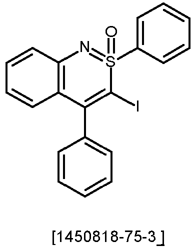 | 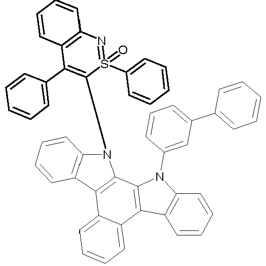 | 83% |
| 13b | 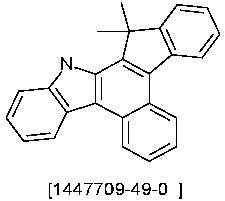 | 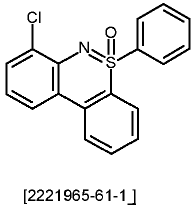 | 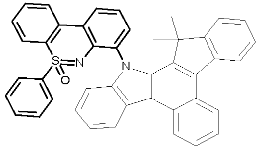 | 85% |
| 14b | 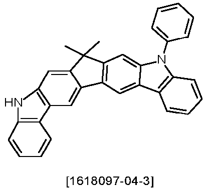 | 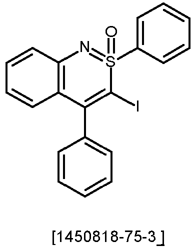 | 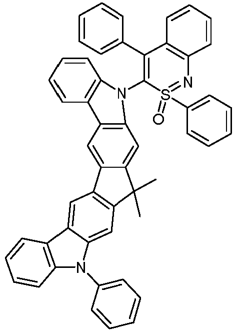 | 76% |
| 15b | 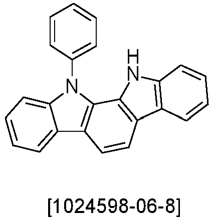 | 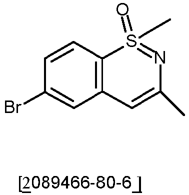 | 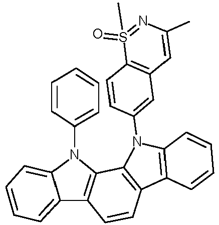 | 84% |

| | | | | |
|---|---|---|---|---|
| 16b | 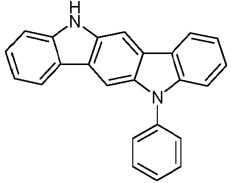 | 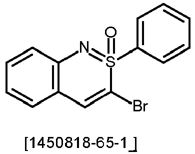 | 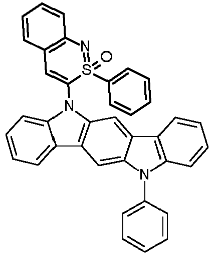 | 73% |
| 17b | 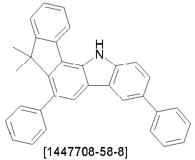 | 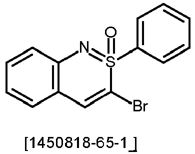 | 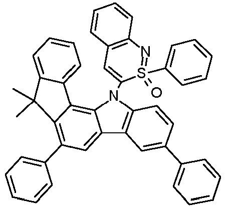 | 72% |
| 18b | 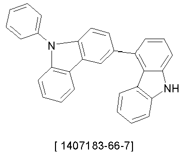 | 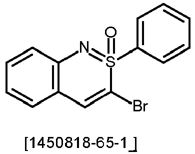 | 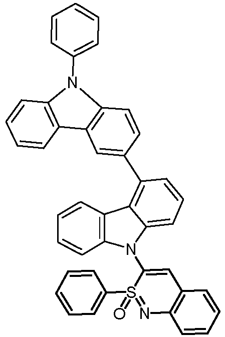 | 70% |
| 19b | 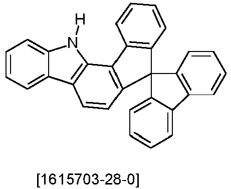 | 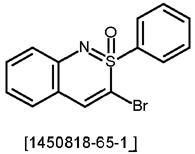 | 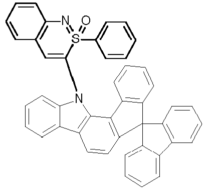 | 73% |

| | | | | |
|---|---|---|---|---|
| 20b | 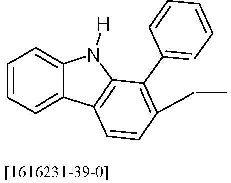 | 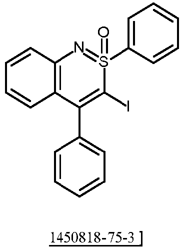 | 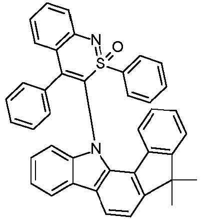 | 66% |
| 21b | 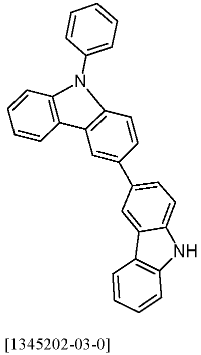 | 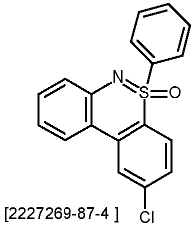 | 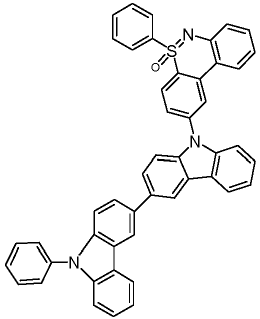 | 69% |
| 22b | 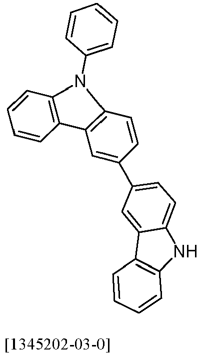 | 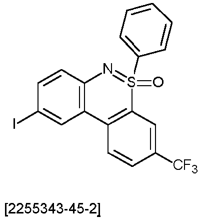 | 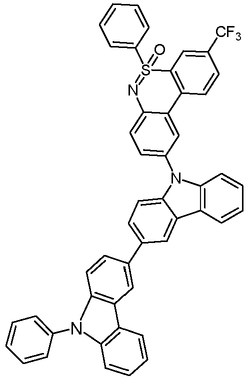 | 72% |

| | | | | |
|---|---|---|---|---|
| 23b | 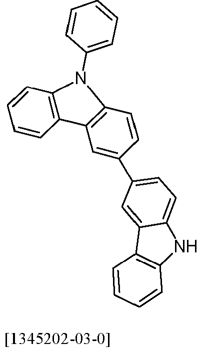 | 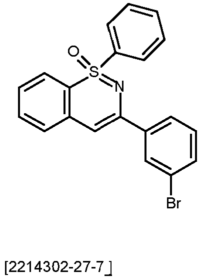 | 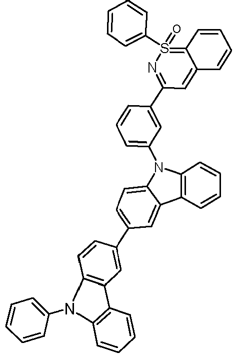 | 79% |
| 24b | 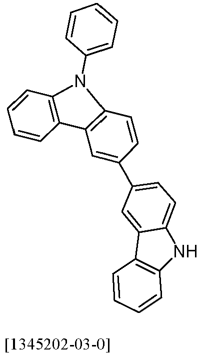 | 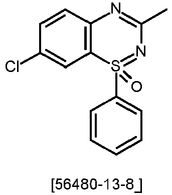 | 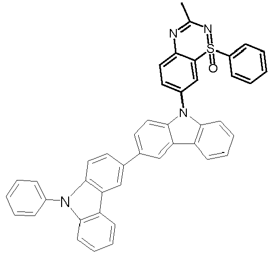 | 77% |
| 25b | 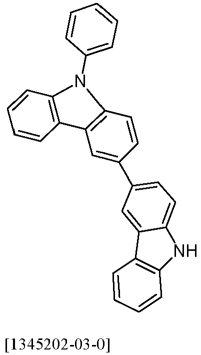 | 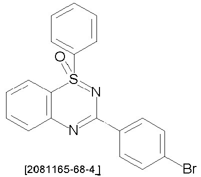 | 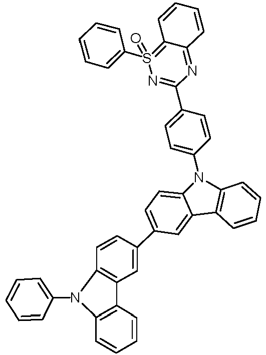 | 70% |

### c) Bromination

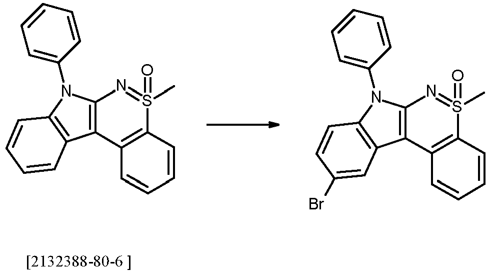

65 g (190 mmol) of the compound with the CAS number [2132388-80-6] are suspended in 1800 mL of DMF. Then, 34 g (190 mmol) of NBS are added portion wise to this suspension, which is stirred for 2 hours in the dark. Water/ice is then added, the solid is separated and washed with ethanol. The isomers are separated by recrystallization. The yield is 57 g (134 mmol), corresponding to 72 % of the theory.

The following compounds can be produced analogously:

| | **Educt 1** | **Product** | **Yield** |
|---|---|---|---|
| 1c | 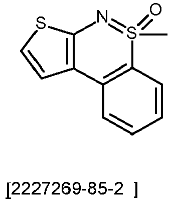 | 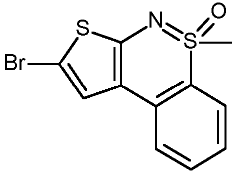 | 76% |
| 2c | 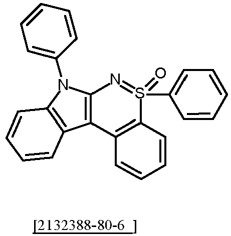 | 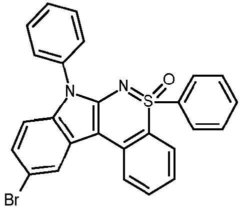 | 81% |

### B) Device examples

The use of the inventive materials in OLEDs is presented in the following examples.

Glass plates coated with structured ITO (indium tin oxide, 50 nm) are treated with an oxygen plasma followed by an argon plasma before coating. These plasma-treated glass plates form the substrates on which the OLEDs are applied.

In principle, the OLEDs have the following layer structure: substrate / optional interlayer (IL) / hole injection layer (HIL) / hole transport layer (HTL) / electron blocking layer (EBL) / emission layer (EML) / optional hole blocking layer (HBL) / electron transport layer (ETL) / optional electron injection layer (EIL) and finally a cathode. The cathode is formed by a 100 nm thick aluminium layer. The exact structure of the OLEDs is shown in Tables 1a to 1c. The materials used for the OLED fabrication are listed in Table 2. The data of the OLEDs are listed in Tables 2a to 2c. The materials used for the fabrication of the OLEDs are listed in Table 3.

All materials are applied by thermal vapour deposition in a vacuum chamber. The emission layer here always consists of at least one matrix material (host material) and an emitting dopant (emitter), which is admixed with the matrix material or matrix materials in a certain proportion by volume by co-evaporation. An expression such as IC1:EG1:TER1 (45%:45%:10%) here means that the material IC1 is present in the layer in a proportion by volume of 45%, EG1 is present in the layer in a proportion by volume of 45% and TER1 is present in the ayer in a proportion by volume of 10%. Analogously, the electron-transport layer may also consist of a mixture of two materials.

The OLEDs are characterized by standard methods. For this purpose, the electroluminescence spectra and the external quantum efficiency (EQE, measured in %) are determined as a function of luminance, calculated from current-voltage-luminance characteristics assuming a Lambertian radiation characteristic. The electroluminescence spectra are determined at a brightness of 1000 cd/m² and the CIE 1931 x and y colour coordinates are determined. The value EQE 1000 corresponds to the external quantum efficiency at 1000 cd/m². In the examples E1 to E6, the inventive materials are used as matrix materials in the emission layer of green phosphorescent OLEDs.

**Table 1a: Structure of the OLEDs**

| **Ex.** | **HIL** | **HTL** | **EBL** | **EML** | **HBL** | **ETL** | **EIL** |
|---|---|---|---|---|---|---|---|
| E1 | HATCN | SpMA1 | SpMA2 | IC1:21b:TEG1 | ST2 | ST2:LiQ | LiQ |
| | 5nm | 215nm | 20nm | (59%:29%:12%) 30nm | 10nm | (50%:50%) 30nm | 1nm |
| E2 | HATCN | SpMA1 | SpMA2 | IC1:30a:TEG1 | ST2 | ST2:LiQ | LiQ |
| | 5nm | 215nm | 20nm | (59%:29%:12%) 30nm | 10nm | (50%:50%) 30nm | 1nm |
| E3 | HATCN | SpMA1 | SpMA2 | IC1:47a:TEG1 | ST2 | ST2:LiQ | LiQ |
| | 5nm | 215nm | 20nm | (59%:29%:12%) 30nm | 10nm | (50%:50%) 30nm | 1nm |
| E4 | HATCN | SpMA1 | SpMA2 | IC1: a:TEG1 | ST2 | ST2:LiQ | LiQ |
| | 5nm | 215nm | 20nm | (59%:29%:12%) 30nm | 10nm | (50%:50%) 30nm | 1nm |
| E5 | HATCN | SpMA1 | SpMA2 | 25b:IC2:TEG1 | ST2 | ST2:LiQ | LiQ |
| | 5nm | 215nm | 20nm | (44%:44%:12%) 30nm | 10nm | (50%:50%) 30nm | 1nm |
| E6 | HATCN | SpMA1 | SpMA2 | 41a:IC2:TEG1 | ST2 | ST2:LiQ | LiQ |
| | 5nm | 215nm | 20nm | (29%:59%:12%) 30nm | 10nm | (50%:50%) 30nm | 1nm |

Very good results for the external quantum efficiency are obtained for all compounds according to the invention, at operating voltages U1000 in the range of 4V.

| **Table 2a: Data of the OLEDs** | | |
|---|---|---|
| **Ex.** | **EQE 1000 (%)** | **CIE x/y at 1000 cd/m²** |
| E1 | 22 | 0.36/0.61 |
| E2 | 21 | 0.35/0.61 |
| E3 | 22 | 0.35/0.61 |
| E4 | 20 | 0.35/0.61 |
| E5 | 23 | 0.35/0.61 |
| E6 | 20 | 0.36/0.61 |

Further inventive materials are used in the examples E7 and E9 as matrix material in the emission layer of red phosphorescent OLEDs.

**Table 1b: Structure of the OLEDs**

| **Ex.** | **HIL** | **HTL** | **EBL** | **EML** | **HBL** | **ETL** | **EIL** |
|---|---|---|---|---|---|---|---|
| E7 | HATCN | SpMA1 | SpMA2 | b:TER5 | ST2 | ST2:LiQ | LiQ 1nm |
| | 5nm | 125nm | 10nm | (97%:3%) 35nm | 10nm | (50%:50%) 30nm | |
| E8 | HATCN | SpMA1 | SpMA2 | 1b:TER5 | ST2 | ST2:LiQ | LiQ 1nm |
| | 5nm | 125nm | 10nm | (97%:3%) 35nm | 10nm | (50%:50%) 30nm | |
| E9 | HATCN | SpMA1 | SpMA2 | 28a:TER5 | ST2 | ST2:LiQ | LiQ 1nm |
| | 5nm | 125nm | 10nm | (97%:3%) 35nm | 10nm | (50%:50%) 30nm | |

Very good results for the external quantum efficiency are obtained for the three compounds in accordance with the invention, at operating voltages U1000 in the range of 4-5 V.

| **Table 2b: Data of the OLEDs** | | |
|---|---|---|
| **Ex.** | **EQE 1000 (%)** | **CIE x/y at 1000 cd/m²** |
| E7 | 22 | 0.67/0.33 |
| E8 | 22 | 0.67/0.33 |
| E9 | 19 | 0.67/0.33 |

Further inventive materials are used in the examples E10 and E11 as ETM (Electron -transport Material) in the ETL or as HBM (Hole-Blocking Material) in the HBL of blue fluorescent OLEDs. The inventive compounds can also be used as ETM and HBM in phosphorescent OLEDs.

**Table 1c: Structure of the OLEDs**

| **Bsp.** | **HIL** | **HTL** | **EBL** | **EML** | **HBL** | **ETL** | **EIL** |
|---|---|---|---|---|---|---|---|
| E10 | HATCN | SpMA1 | SpMA2 | M2:SEB | --- | 25b:LiQ | LiQ |
| | 5nm | 195nm | 10nm | (95%:5%) 20nm | | (50%:50%) 30nm | 1nm |
| E11 | HATCN | SpMA1 | SpMA2 | M2:SEB | a | ST2 | LiQ |
| | 5nm | 195nm | 10nm | (95%:5%) 20nm | 10nm | 20nm | 3nm |

Very good results for the external quantum efficiency are obtained for the two compounds in accordance with the invention, at operating voltages U1000 in the range of 4-5 V.

| **Table 2c: Data of the OLEDs** | | |
|---|---|---|
| **Ex.** | **EQE 1000 (%)** | **CIE x/y bei 1000 cd/m²** |
| E10 | 9 | 0.14/0.15 |
| E11 | 9 | 0.14/0.15 |

**Table 3: Structures of the materials for the fabrication of the OLEDs**

| | |
|---|---|
| 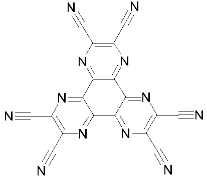 | 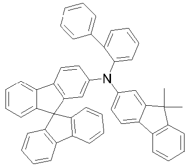 |
| HATCN | SpMA1 |
| 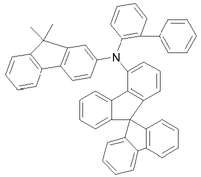 | 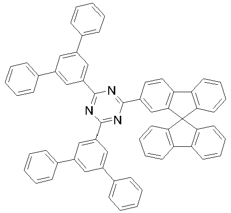 |
| SpMA2 | ST2 |
| 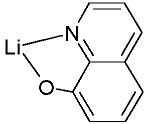 | 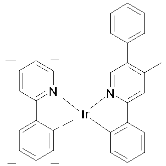 |
| LiQ | TEG1 |
| 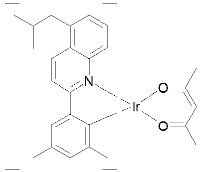 | 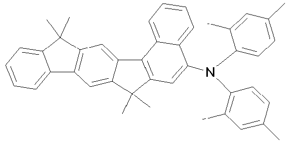 |
| TER5 | SEB |
| 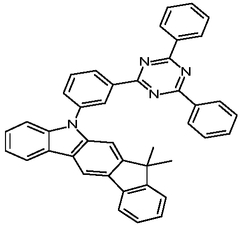 | 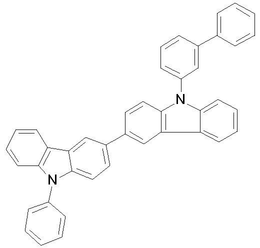 |
| IC1 | IC2 |
| 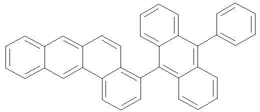 | 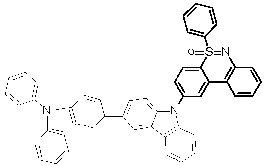 |
| M2 | 21b |
| 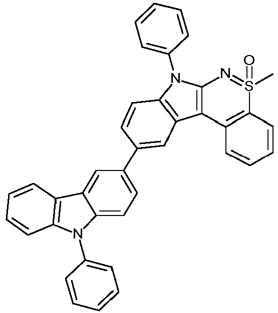 | 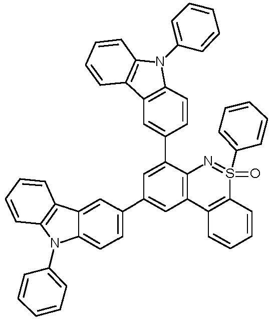 |
| 30a | 47a |
| 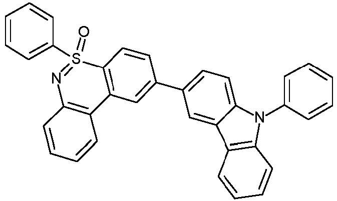 | 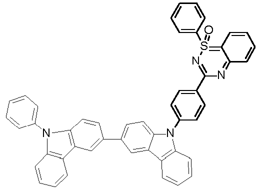 |
| a | 25b |
| 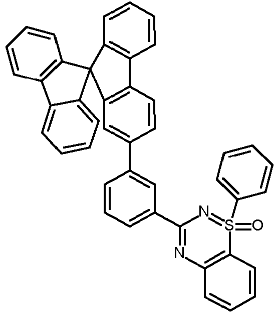 | 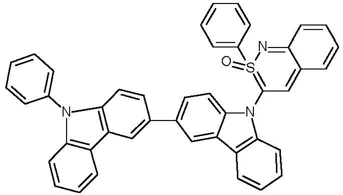 |
| 41a | b |
| 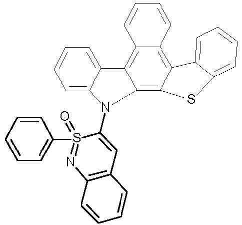 | 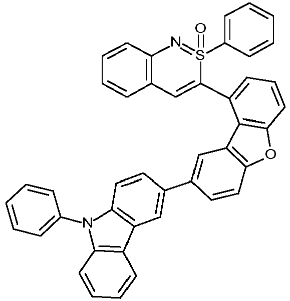 |
| 1b | 28a |

## Claims

1. An organic electroluminescent device comprising an anode, a cathode and at least one organic layer between the cathode and the anode, **characterized in that** the at least one organic layer comprises a compound of the formula (1), 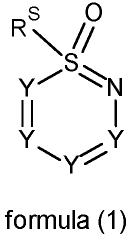 where the following applies to the symbols used:
Y stands for CR^{Y} or N; with the proviso that at least two adjacent groups Y form a condensed aromatic or heteroaromatic ring system with the benzothiazine ring of formula (1);
R^{S}, R^{Y} stand on each occurrence, identically or differently, for H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R, where in each case one or more non-adjacent CH₂ groups may be replaced by RC=CR, C≡C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 60 aromatic ring atoms, which may be substituted by one or more radicals R;
where the radical R^{S} may form an aliphatic, aromatic or heteroaromatic ring system ring with a radical R^{Y} or with the adjacent ring formed by two groups Y, which may be substituted by one or more radicals R; and where two adjacent radicals R^{Y} may form an aliphatic, aromatic or heteroaromatic ring system together, which may be substituted by one or more radicals R;
R stands on each occurrence, identically or differently, for H, D, F, Cl, Br, I, CHO, CN, C(=O)Ar, P(=O)(Ar)₂, S(=O)Ar, S(=O)₂Ar, N(R')₂, N(Ar)₂, NO₂, Si(R')₃, B(OR')₂, OSO₂R', a straight-chain alkyl, alkoxy or thioalkyl group having 1 to 40 C atoms or branched or a cyclic alkyl, alkoxy or thioalkyl group having 3 to 40 C atoms, each of which may be substituted by one or more radicals R', where in each case one or more non-adjacent CH₂ groups may be replaced by R'C=CR', C=C, Si(R')₂, Ge(R')₂, Sn(R')₂, C=O, C=S, C=Se, P(=O)(R'), SO, SO₂, O, S or CONR' and where one or more H atoms may be replaced by D, F, Cl, Br, I, CN or NO₂, an aromatic or heteroaromatic ring systems having 5 to 60 aromatic ring atoms, which may in each case be substituted by one or more radicals R', or an aryloxy group having 5 to 60 aromatic ring atoms, which may be substituted by one or more radicals R'; where two adjacent substituents R may form an aliphatic or aromatic ring system together, which may be substituted by one or more radicals R';
Ar is, on each occurrence, identically or differently, an aromatic or heteroaromatic ring system having 5 to 60 aromatic ring atoms, which may in each case also be substituted by one or more radicals R';
R' stands on each occurrence, identically or differently, for H, D, F, Cl, Br, I, CN, a straight-chain alkyl, alkoxy or thioalkyl group having 1 to 20 C atoms or a branched or cyclic alkyl, alkoxy or thioalkyl group having 3 to 20 C atoms, where in each case one or more non-adjacent CH₂ groups may be replaced by SO, SO₂, O, S and where one or more H atoms may be replaced by D, F, Cl, Br or I, or an aromatic or heteroaromatic ring system having 5 to 24 aromatic ring atoms.

2. An organic electroluminescent device according to claim 1, **characterized in that** the compound of formula (1) is selected from the compounds of formulae (2), (3) or (4); 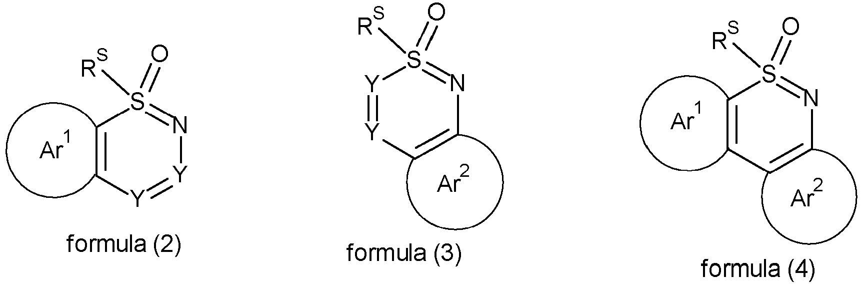 where the rings Ar¹ and Ar² are aromatic or heteroaromatic ring systems condensed with the benzothiazine ring, which have 5 to 30 aromatic ring atoms and which may be substituted by at least one radical R; and where the symbols Y and R^{S} have the same meaning as in claim1.

3. An organic electroluminescent device according to claim 2, **characterized in that** the rings Ar¹ and Ar² are selected from the rings of the formulae (Ar-1) and (Ar-2), 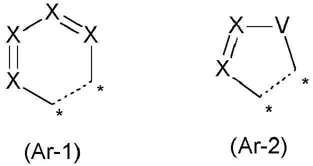 where the dashed bonds between the signs "*" correspond to the common bond of the group of formula (Ar-1) or (Ar-2) with the benzothiazine ring, and where:
V stands for C(R⁰)₂, NR^{N}, O or S;
X stands, identically or differently on each occurrence, for CR^{X} or N; or two adjacent groups X stand for a group of the formula (Ar-3) or (Ar-4), 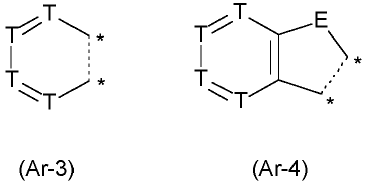
where the dashed bonds between the signs "*" correspond to the common bond of the group of formula (Ar-3) or (Ar-4) with a group of formula (Ar-1) or (Ar-2), and furthermore:
E stands for C(R⁰)₂, C=O, NR^{N}, O or S; and
T stands, identically or differently on each occurrence, for CR^{T} or N;
R^{T}, R^{X} stand on each occurrence, identically or differently, for H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R, where in each case one or more non-adjacent CH₂ groups may be replaced by RC=CR, C≡C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 60 aromatic ring atoms, which may be substituted by one or more radicals R; where two adjacent substituents R^{T} may form an aliphatic, aromatic or heteroaromatic ring system together, which may be substituted by one or more radicals R, and where two adjacent substituents R^{X} may form an aliphatic, aromatic or heteroaromatic ring system together, which may be substituted by one or more radicals R;
R⁰ stands on each occurrence, identically or differently, for H, D, F, Cl, Br, I, CN, a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R, where in each case one or more non-adjacent CH₂ groups may be replaced by RC=CR, C≡C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 60 aromatic ring atoms, which may be substituted by one or more radicals R, where two adjacent substituents R⁰ may form an aliphatic, aromatic or heteroaromatic ring system together, which may be substituted by one or more radicals R; and
R^{N} stands on each occurrence, identically or differently, for H, D, F, Cl, Br, I, CN, Si(R)₃, a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R, where in each case one or more non-adjacent CH₂ groups may be replaced by RC=CR, C≡C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 60 aromatic ring atoms, which may be substituted by one or more radicals R.

4. An organic electroluminescent device according to one or more of the preceding claims, **characterized in that** the compound of formula (1) is selected from the compounds of formulae (2-1) to (4-5) as depicted below: 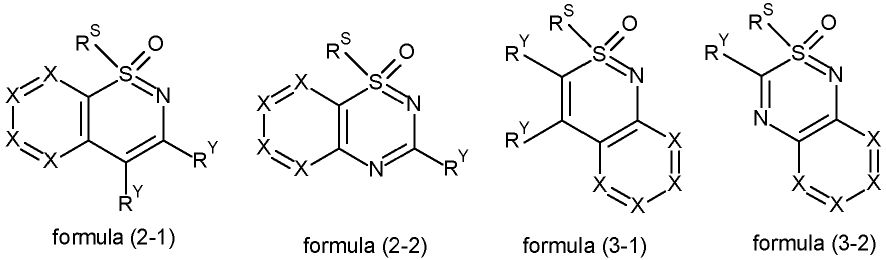 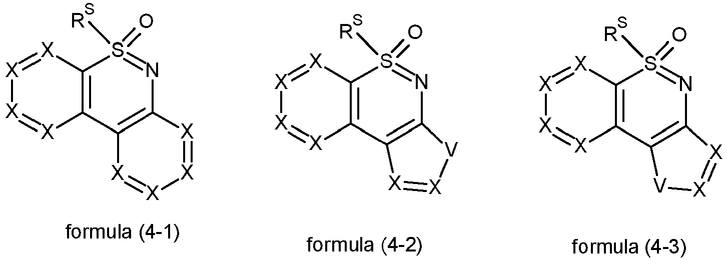 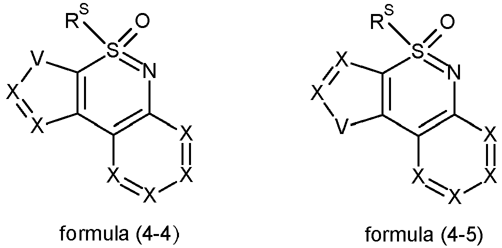 where the symbols X, R^{S}, R^{Y} have the same meaning as in claim 1 and the symbol V has the same meaning as in claim 3.

5. An organic electroluminescent device according to one or more of the preceding claims, **characterized in that** the compound of formula (1) is selected from the compounds of formulae (2-1-1) to (4-5-3) as depicted below: 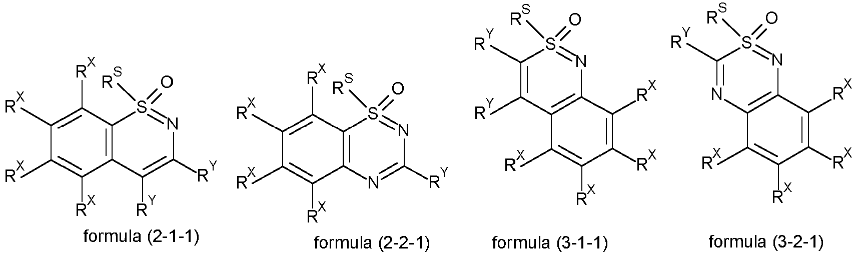 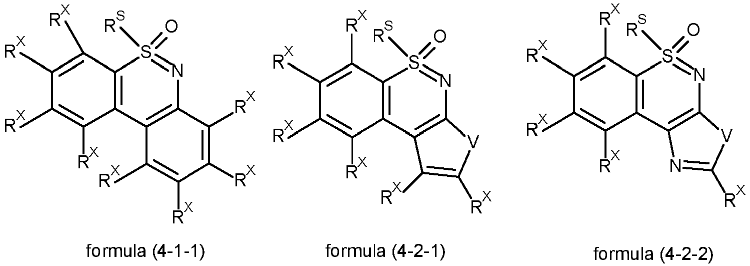 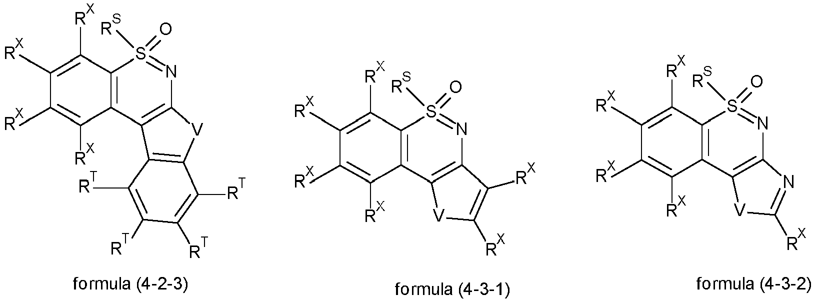 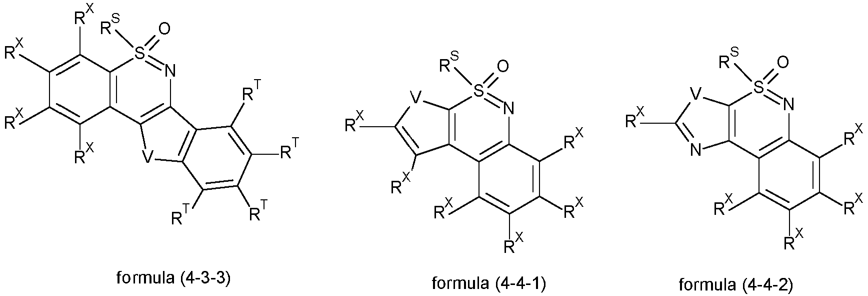 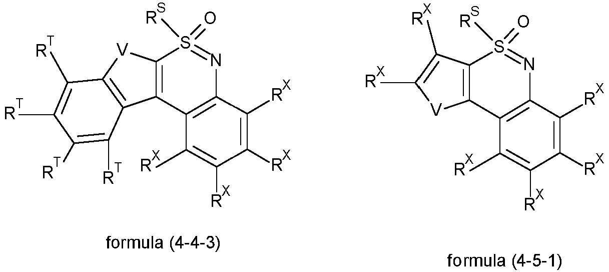 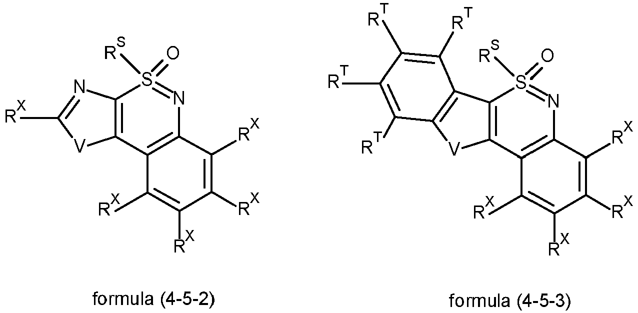 where the symbols R^{Y} and R^{S} have the same meaning as in claim 1 and the symbols R^{X}, R^{T} and V have the same meaning as in claim 3.

6. An organic electroluminescent device according to one or more of the preceding claims, **characterized in that** the compound of formula (1) comprises at least one radical selected from the groups of formula (R-1), 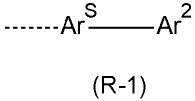 where
Ar^{S} stands for a single bond or for an aromatic or heteroaromatic ring system having 5 to 30 aromatic ring atoms, which may be substituted by one or more radicals R; and
Ar² stands for phenyl, biphenyl, terphenyl, quaterphenyl, fluorene, spirobifluorene, naphthalene, anthracene, phenanthrene, triphenylene, fluoranthene, indole, benzofuran, benzothiophene, dibenzofuran, dibenzothiophene, carbazole, indenocarbazole, indolocarbazole, phenanthroline, pyridine, pyrimidine, pyrazine, pyridazine, triazine, quinolone, benzopyridine, benzopyridazine, benzopyrimidine, quinazoline, benzimidazole, or a combination of two or three of these groups, each of which may be substituted by one or more radicals R.

7. An electroluminescent device according to one or more of the preceding claims, **characterized in that** the compound of formula (1) comprises at least one radical selected from the groups of formula (R-2), 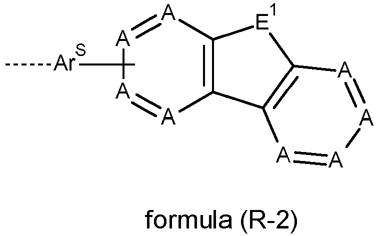 where the dashed bond indicates the bond to the structure of formula (1), Ar^{S} has the same definition as in claim 6 and where:
A stands, identically or differently on each occurrence, for CR^{A} or N; or two adjacent groups A stand for a group of the formula (Ar-5) or (Ar-6), 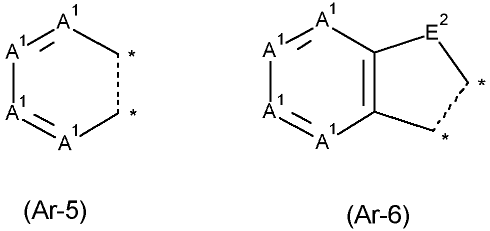
where the dashed bonds between the signs "*" correspond to the common bond of the group of formula (Ar-5) or (Ar-6) with the group of formula (R-2), and where:
E¹, E² stands for C(R⁰)₂, C=O, NR^{N}, O or S, where R⁰ and R^{N} have the same definition as in claim 3;
A¹ stands, identically or differently on each occurrence, for CR^{A1} or N;
R^{A}, R^{A1} are on each occurrence, identically or differently, for H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R, where in each case one or more non-adjacent CH₂ groups may be replaced by RC=CR, C≡C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 60 aromatic ring atoms, which may be substituted by one or more radicals R; where two adjacent substituents R^{A} may form an aliphatic, aromatic or heteroaromatic ring system together, which may be substituted by one or more radicals R, and where two adjacent substituents R^{A1} may form an aliphatic, aromatic or heteroaromatic ring system together, which may be substituted by one or more radicals R.

8. An organic electroluminescent device according to one or more of the preceding claims, **characterized in that** the compound of the formula (1) is employed as matrix material for fluorescent or phosphorescent emitters, and/or in a hole-blocking layer and/or in an electron-transport layer and/or in an electron-blocking or exciton-blocking layer and/or in a hole-transport layer.

9. An organic electroluminescent device according to one or more of the preceding claims, **characterized in that** the compound of the formula (1) is employed as matrix material for phosphorescent emitters.

10. Compound of formula (1'), 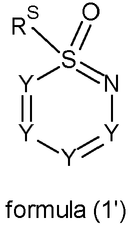 where the following applies to the symbols and indices used:
Y stands for CR^{Y} or N; with the proviso that at least two adjacent groups Y stand for a condensed aromatic or heteroaromatic ring system with the benzothiazine ring of formula (1');
R^{S}, R^{Y} stand on each occurrence, identically or differently, for H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R, where in each case one or more non-adjacent CH₂ groups may be replaced by RC=CR, C≡C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 60 aromatic ring atoms, which may be substituted by one or more radicals R;
- where the radical R^{S} may form an aliphatic, aromatic or heteroaromatic ring system ring with a radical R^{Y} or with the adjacent ring formed by two groups Y, which may be substituted by one or more radicals R; and
- where two adjacent radicals R^{Y} may form an aliphatic, aromatic or heteroaromatic ring system together, which may be substituted by one or more radicals R;
R stands on each occurrence, identically or differently, for H, D, F, Cl, Br, I, CHO, CN, C(=O)Ar, P(=O)(Ar)₂, S(=O)Ar, S(=O)₂Ar, N(R')₂, N(Ar)₂, NO₂, Si(R')₃, B(OR')₂, OSO₂R', a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R', where in each case one or more non-adjacent CH₂ groups may be replaced by R'C=CR', C=C, Si(R')₂, Ge(R')₂, Sn(R')₂, C=O, C=S, C=Se, P(=O)(R'), SO, SO₂, O, S or CONR' and where one or more H atoms may be replaced by D, F, Cl, Br, I, CN or NO₂, an aromatic or heteroaromatic ring systems having 5 to 60 aromatic ring atoms, which may in each case be substituted by one or more radicals R', or an aryloxy group having 5 to 60 aromatic ring atoms, which may be substituted by one or more radicals R'; where two adjacent substituents R may form an aliphatic or aromatic ring system together, which may be substituted by one or more radicals R';
Ar is, on each occurrence, identically or differently, an aromatic or heteroaromatic ring system having 5 to 60 aromatic ring atoms, which may in each case also be substituted by one or more radicals R'; and
R stands on each occurrence, identically or differently, for H, D, F, Cl, Br, I, CN, a straight-chain alkyl, alkoxy or thioalkyl group having 1 to 20 C atoms or abranched or cyclic alkyl, alkoxy or thioalkyl group having 3 to 20 C atoms, where in each case one or more non-adjacent CH₂ groups may be replaced by SO, SO₂, O, S and where one or more H atoms may be replaced by D, F, Cl, Br or I, or an aromatic or heteroaromatic ring system having 5 to 24 aromatic ring atoms;
**characterized in that** it the compounds of formula (1') comprises at least one radical, which stands for a group of formula (R-2), 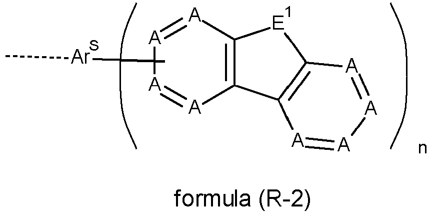
where the dashed bond indicates the bond to the structure, and where:
Ar^{S} stands for a single bond or for an aromatic or heteroaromatic ring system having 5 to 30 aromatic ring atoms, which may be substituted by one or more radicals R;
A stands, identically or differently on each occurrence, for CR^{A} or N; or two adjacent groups A stand for a group of the formula (Ar-5) or (Ar-6), 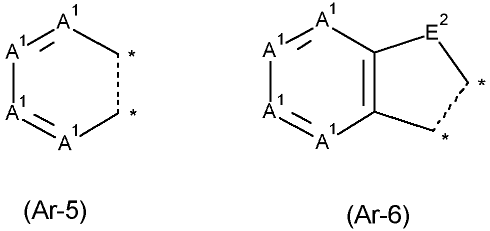
where the dashed bonds between the signs "*" correspond to the common bond of the group of formula (Ar-5) or (Ar-6) with the group of formula (R-2), and where:
E¹, E² stands for C(R⁰)₂, C=O, NR^{N}, O or S;
A¹ stands, identically or differently on each occurrence, for CR^{A1} or N;
R⁰, R^{N} are on each occurrence, identically or differently, for H, D, F, Cl, Br, I, CN, Si(R)₃, a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R, where in each case one or more non-adjacent CH₂ groups may be replaced by RC=CR, C=C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 60 aromatic ring atoms, which may be substituted by one or more radicals R, where two adjacent substituents R⁰ may form an aliphatic, aromatic or heteroaromatic ring system together, which may be substituted by one or more radicals R;
R^{A}, R^{A1} are on each occurrence, identically or differently, for H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R, where in each case one or more non-adjacent CH₂ groups may be replaced by RC=CR, C≡C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals **R,** or an aryloxy group having 5 to 60 aromatic ring atoms, which may be substituted by one or more radicals R; where two adjacent substituents R^{A} may form an aliphatic, aromatic or heteroaromatic ring system together, which may be substituted by one or more radicals R; and where two adjacent substituents R^{A1} may form an aliphatic, aromatic or heteroaromatic ring system together, which may be substituted by one or more radicals R; and
n is an integer equal to 1, 2, 3 or 4.

11. Compound according to claim 10, **characterized in that** it is selected from compounds of formulae (2-1') to (4-5') as depicted below: 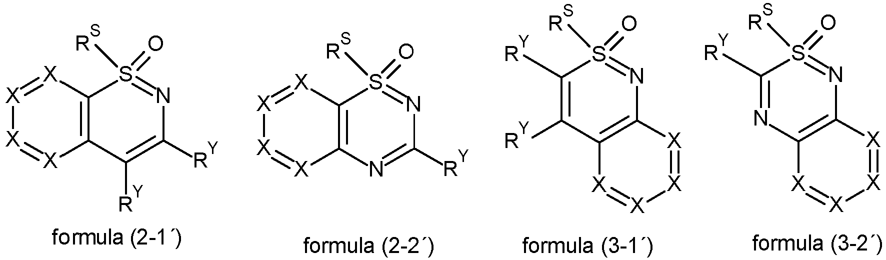 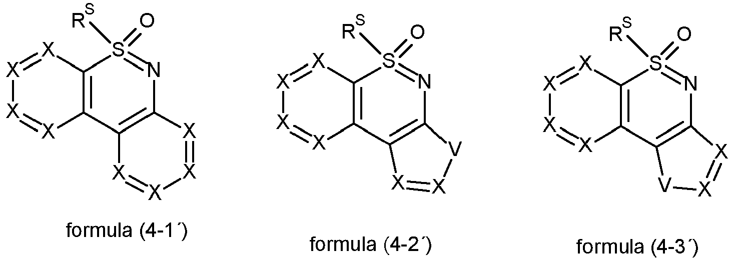 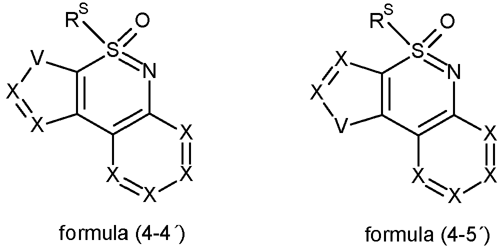 where the symbols R^{S}, R^{Y} have the same meaning as in claim 10, and where:
V stands for C(R⁰)₂, NR^{N}, O or S;
X stands, identically or differently on each occurrence, for CR^{X} or N; or two adjacent groups X stand for a group of the formula (Ar-3) or (Ar-4), 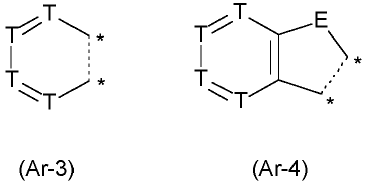
where the dashed bonds between the signs "*" correspond to the common bond of the group of formula (Ar-3) or (Ar-4) with the rest of the structure,
and furthermore:
- E stands for C(R⁰)₂, C=O, NR^{N}, O or S; where R^{N} and R⁰ have the same meaning as in claim 10;
- T stands, identically or differently on each occurrence, for CR^{T} or N;
- R^{T}, R^{X} stand on each occurrence, identically or differently, for H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, a straight-chain alkyl, alkoxy or thioalkyl group having 1 to 40 C atoms or a branched or a cyclic alkyl, alkoxy or thioalkyl group having 3 to 40 C atoms, each of which may be substituted by one or more radicals R, where in each case one or more non-adjacent CH₂ groups may be replaced by RC=CR, C≡C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 60 aromatic ring atoms, which may be substituted by one or more radicals R; where two adjacent substituents R^{T} may form an aliphatic, aromatic or heteroaromatic ring system together, which may be substituted by one or more radicals R, and where two adjacent substituents R^{X} may form an aliphatic, aromatic or heteroaromatic ring system together, which may be substituted by one or more radicals R;
**characterized in that** the compounds of formulae (2-1') to (4-5') comprise at least one radical R^{S}, R^{X}, R^{Y}, R^{T} or R, which stands for a group of formula (R-2) as defined in claim 10.

12. Formulation comprising at least one compound according to claim 10 or 11 and at least one solvent.

13. Electronic device comprising at least one compound according to claim 10 or 11.

14. Electronic device according to claim 13, **characterized in that** it is selected from the group consisting of organic electroluminescent devices, organic integrated circuits, organic field-effect transistors, organic thin-film transistors, organic light-emitting transistors, organic solar cells, dye-sensitised organic solar cells, organic optical detectors, organic photoreceptors, organic field-quench devices, light-emitting electrochemical cells, organic laser diodes and organic plasmon emitting devices.

15. Electronic device according to claim 13 or 14, which is an organic electroluminescent device, **characterised in that** the compound according to claim 10 or 11 is employed as a matrix material for emitters, a hole-transport-material or an electron-transport material.

## Patentansprüche

1. Organische Elektrolumineszenzvorrichtung, umfassend eine Anode, eine Kathode und mindestens eine organische Schicht zwischen der Kathode und der Anode, **dadurch gekennzeichnet, dass** die mindestens eine organische Schicht eine Verbindung der Formel (1) umfasst, 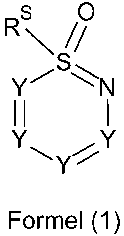 wobei für die verwendeten Symbole Folgendes gilt:
Y steht für CR^{Y} oder N; mit der Maßgabe, dass mindestens zwei benachbarte Gruppen Y mit dem Benzothiazinring der Formel (1) ein kondensiertes aromatisches oder heteroaromatisches Ringsystem bilden;
R^{S}, R^{Y} stehen bei jedem Vorkommen, gleich oder verschieden, für H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, eine geradkettige Alkyl-, Alkoxyoder Thioalkylgruppe mit 1 bis 40 C-Atomen oder eine verzweigte oder cyclische Alkyl-, Alkoxy- oder Thioalkylgruppe mit 3 bis 40 C-Atomen, von denen jede durch einen oder mehrere Reste R substituiert sein kann, wobei jeweils eine oder mehrere nicht benachbarte CH₂-Gruppen durch RC=CR, C≡C, Si(R)₂, Ge(R)₂ , Sn(R)₂ , C=O, C=S, C=Se, P(=O)(R), SO, SO₂, O, S oder CONR ersetzt sein können und wobei ein oder mehrere H-Atome durch D, F, Cl, Br, I, CN oder NO₂ ersetzt sein können; ein aromatisches oder heteroaromatisches Ringsystem mit 5 bis 60 aromatischen Ringatomen, das jeweils durch einen oder mehrere Reste R substituiert sein kann, oder eine Aryloxygruppe mit 5 bis 60 aromatischen Ringatomen, die durch einen oder mehrere Reste R substituiert sein kann;
wobei der Rest R^{S} mit einem Rest R^{Y} oder mit dem benachbarten, aus zwei Gruppen Y gebildeten Ring ein aliphatisches, aromatisches oder heteroaromatisches Ringsystem bilden kann, das durch einen oder mehrere Reste R substituiert sein kann;
und wobei zwei benachbarte Reste R^{Y} gemeinsam ein aliphatisches, aromatisches oder heteroaromatisches Ringsystem bilden können, das durch einen oder mehrere Reste R substituiert sein kann;
R steht bei jedem Vorkommen, gleich oder verschieden, für H, D, F, Cl, Br, I, CHO, CN, C(=O)Ar, P(=O)(Ar)₂, S(=O)Ar, S(=O)₂Ar, N(R')₂, N(Ar)₂, NO₂, Si(R')₃, B(OR')₂, OSO₂R', eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, von denen jede durch einen oder mehrere Reste R' substituiert sein kann, wobei jeweils eine oder mehrere nicht benachbarte CH₂-Gruppen durch R' C=CR', C=C, Si(R')₂, Ge(R')₂, Sn(R')₂, C=O, C=S, C=Se, P(=O)(R'), SO, SO₂, O, S oder CONR' ersetzt sein können und wobei ein oder mehrere H-Atome durch D, F, Cl, Br, I, CN oder NO₂ ersetzt sein können, ein aromatisches oder heteroaromatisches Ringsystem mit 5 bis 60 aromatischen Ringatomen, das jeweils durch einen oder mehrere Reste R' substituiert sein kann, oder eine Aryloxygruppe mit 5 bis 60 aromatischen Ringatomen, die durch einen oder mehrere Reste R' substituiert sein kann; wobei zwei benachbarte Substituenten R gemeinsam ein aliphatisches oder aromatisches Ringsystem bilden können, das durch einen oder mehrere Reste R' substituiert sein kann;
Ar steht bei jedem Vorkommen gleich oder verschieden für ein aromatisches oder heteroaromatisches Ringsystem mit 5 bis 60 aromatischen Ringatomen, das jeweils auch durch einen oder mehrere Reste R' substituiert sein kann;
R' steht bei jedem Vorkommen, gleich oder verschieden, für H, D, F, Cl, Br, I, CN, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen, wobei jeweils eine oder mehrere nicht benachbarte CH₂-Gruppen durch SO, SO₂, O, S ersetzt sein können und wobei ein oder mehrere H-Atome durch D, F, Cl, Br oder I ersetzt sein können, oder ein aromatisches oder heteroaromatisches Ringsystem mit 5 bis 24 aromatischen Ringatomen.

2. Organische Elektrolumineszenzvorrichtung nach Anspruch 1, **dadurch gekennzeichnet, dass** die Verbindung der Formel (1) aus den Verbindungen der Formeln (2), (3) oder (4) ausgewählt ist; 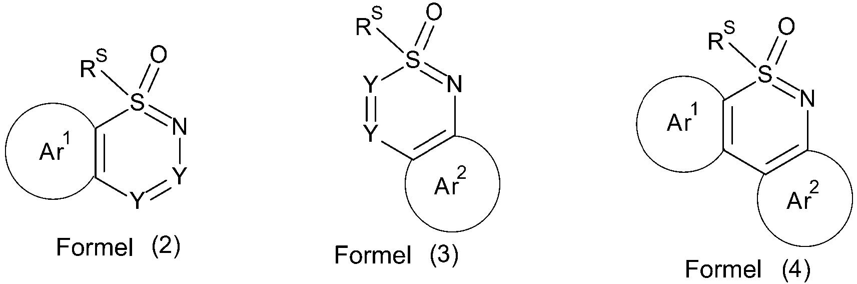 wobei die Ringe Ar¹ und Ar² mit dem Benzothiazinring kondensierte aromatische oder heteroaromatische Ringsysteme sind, die 5 bis 30 aromatische Ringatome aufweisen und durch mindestens einen Rest R substituiert sein können; und wobei die Symbole Y und R^{S} dieselbe Bedeutung wie in Anspruch 1 haben.

3. Organische Elektrolumineszenzvorrichtung nach Anspruch 2, **dadurch gekennzeichnet, dass** die Ringe Ar¹ und Ar² aus den Ringen der Formeln (Ar-1) und (Ar-2) ausgewählt sind, 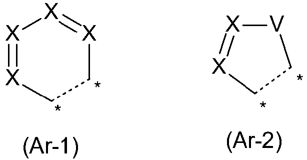 wobei die gestrichelten Bindungen zwischen den Zeichen "*" der gemeinsamen Bindung der Gruppe der Formel (Ar-1) oder (Ar-2) mit dem Benzothiazinring entsprechen und wobei:
V für C(R⁰)₂, NR^{N}, O oder S steht;
X steht, bei jedem Vorkommen gleich oder verschieden, für CR^{X} oder N; oder zwei benachbarte Gruppen X stehen für eine Gruppe der Formel (Ar-3) oder (Ar-4), 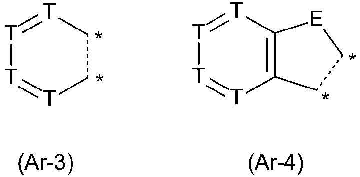
wobei die gestrichelten Bindungen zwischen den Zeichen "*" der gemeinsamen Bindung der Gruppe der Formel (Ar-3) oder (Ar-4) mit einer Gruppe der Formel (Ar-1) oder (Ar-2) entsprechen, und ferner:
E für C(R⁰)₂ , C=O, NR^{N}, O oder S steht; und
T steht, bei jedem Vorkommen gleich oder verschieden, für CR^{T} oder N;
R^{T}, R^{X} stehen bei jedem Vorkommen, gleich oder verschieden, für H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, von denen jede durch einen oder mehrere Reste R substituiert sein kann, wobei jeweils eine oder mehrere nicht benachbarte CH₂-Gruppen durch RC=CR, C=C, Si(R)₂, Ge(R)₂, Sn(R)₂, C=O, C=S, C=Se, P(=O)(R), SO, SO₂, O, S oder CONR ersetzt sein können und wobei ein oder mehrere H-Atome durch D, F, Cl, Br, I, CN oder NO₂ersetzt sein können; ein aromatisches oder heteroaromatisches Ringsystem mit 5 bis 60 aromatischen Ringatomen, das jeweils durch einen oder mehrere Reste R substituiert sein kann, oder eine Aryloxygruppe mit 5 bis 60 aromatischen Ringatomen, die durch einen oder mehrere Reste R substituiert sein kann; wobei zwei benachbarte Substituenten R^{T} gemeinsam ein aliphatisches, aromatisches oder heteroaromatisches Ringsystem bilden können, das durch einen oder mehrere Reste R substituiert sein kann, und wobei zwei benachbarte Substituenten R^{X} gemeinsam ein aliphatisches, aromatisches oder heteroaromatisches Ringsystem bilden können, das durch einen oder mehrere Reste R substituiert sein kann;
R⁰ steht bei jedem Vorkommen, gleich oder verschieden, für H, D, F, Cl, Br, I, CN, eine geradkettige Alkyl-, Alkoxy- oder Thioalkylgruppe mit 1 bis 40 C-Atomen oder eine verzweigte oder cyclische Alkyl-, Alkoxy-oder Thioalkylgruppe mit 3 bis 40 C-Atomen, von denen jede durch einen oder mehrere Reste R substituiert sein kann, wobei jeweils eine oder mehrere nicht benachbarte CH₂-Gruppen durch RC=CR, C=C, Si(R)₂, Ge(R)₂, Sn(R)₂, C=O, C=S, C=Se, P(=O)(R), SO, SO₂, O, S oder CONR ersetzt sein können und wobei ein oder mehrere H-Atome durch D, F, Cl, Br, I, CN oder NO₂ ersetzt sein können, ein aromatisches oder heteroaromatisches Ringsystem mit 5 bis 60 aromatischen Ringatomen, das jeweils durch einen oder mehrere Reste R substituiert sein kann, oder eine Aryloxygruppe mit 5 bis 60 aromatischen Ringatomen, die durch einen oder mehrere Reste R substituiert sein kann, wobei zwei benachbarte Substituenten R⁰ gemeinsam ein aliphatisches, aromatisches oder heteroaromatisches Ringsystem bilden können, das durch einen oder mehrere Reste R substituiert sein kann; und
R^{N} steht bei jedem Vorkommen, gleich oder verschieden, für H, D, F, Cl, Br, I, CN, Si(R)₃, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, von denen jede durch einen oder mehrere Reste R substituiert sein kann, wobei jeweils eine oder mehrere nicht benachbarte CH₂-Gruppen durch RC=CR, C=C, Si(R)₂, Ge(R)₂, Sn(R)₂, C=O, C=S, C=Se, P(=O)(R), SO, SO₂, O, S oder CONR ersetzt sein können und wobei ein oder mehrere H-Atome durch D, F, Cl, Br, I, CN oder NO₂ ersetzt sein können, ein aromatisches oder heteroaromatisches Ringsystem mit 5 bis 60 aromatischen Ringatomen, das jeweils durch einen oder mehrere Reste R substituiert sein kann, oder eine Aryloxygruppe mit 5 bis 60 aromatischen Ringatomen, die durch einen oder mehrere Reste R substituiert sein kann.

4. Organische Elektrolumineszenzvorrichtung gemäß einem oder mehreren der vorstehenden Ansprüche, **dadurch gekennzeichnet, dass** die Verbindung der Formel (1) aus den nachstehend dargestellten Verbindungen der Formeln (2-1) bis (4-5) ausgewählt ist: 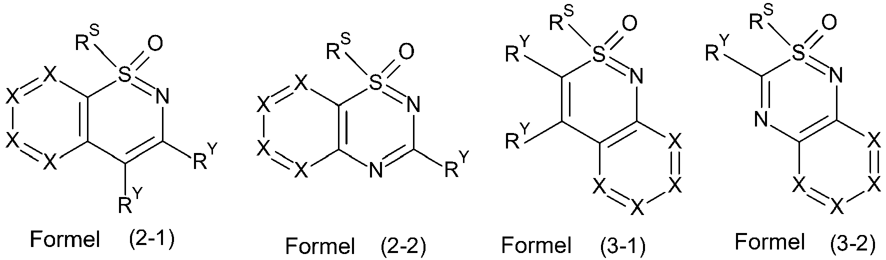 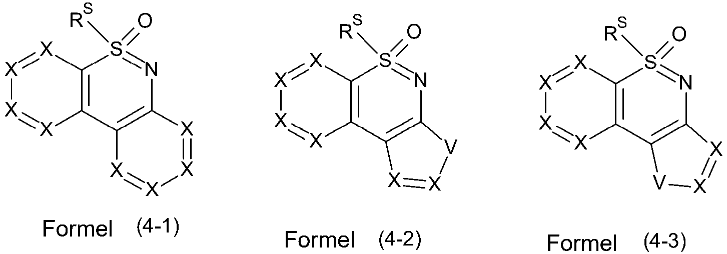 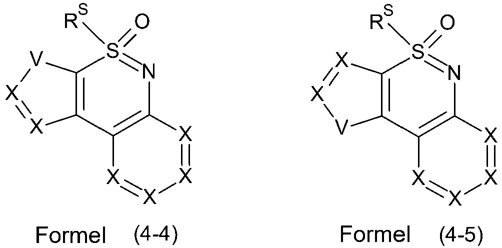 wobei die Symbole X, R^{S}, R^{Y} dieselbe Bedeutung wie in Anspruch 1 haben und das Symbol V dieselbe Bedeutung wie in Anspruch 3 hat.

5. Organische Elektrolumineszenzvorrichtung gemäß einem oder mehreren der vorstehenden Ansprüche, **dadurch gekennzeichnet, dass** die Verbindung der Formel (1) aus den Verbindungen der Formeln (2-1-1) bis (4-5-3) ausgewählt ist, wie nachstehend dargestellt: 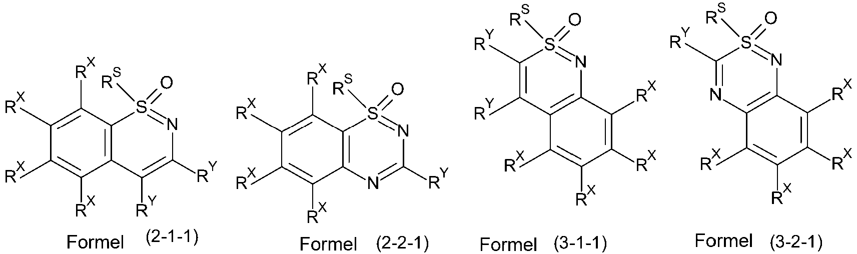 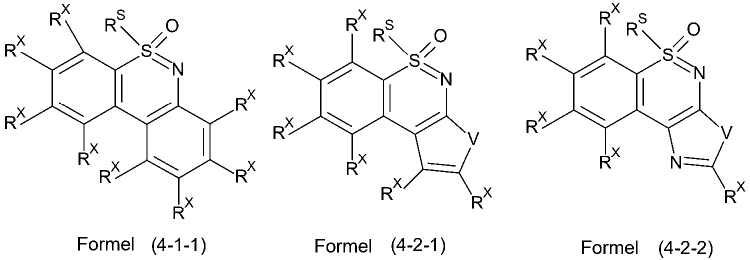 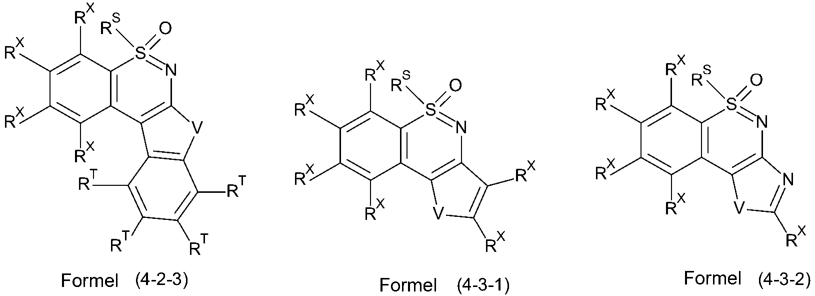 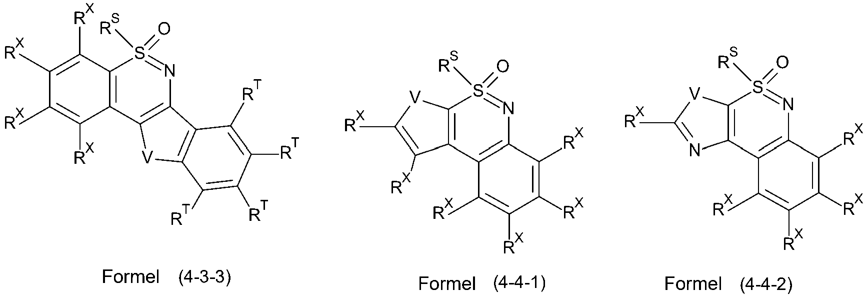 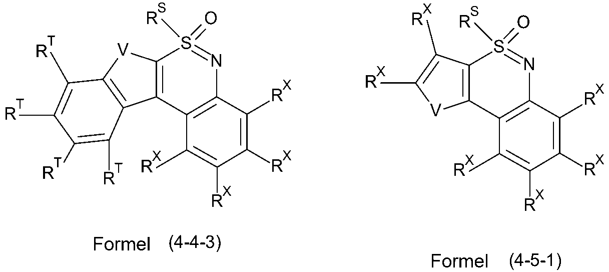 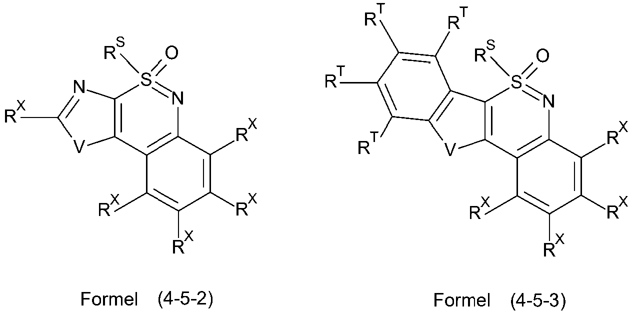 wobei die Symbole R^{Y} und R^{S} dieselbe Bedeutung wie in Anspruch 1 haben und die Symbole R^{X}, R^{T} und V dieselbe Bedeutung wie in Anspruch 3 haben.

6. Organische Elektrolumineszenzvorrichtung gemäß einem oder mehreren der vorstehenden Ansprüche, **dadurch gekennzeichnet, dass** die Verbindung der Formel (1) mindestens einen Rest umfasst, der aus den Gruppen der Formel (R-1) ausgewählt ist, 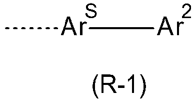 wobei
Ar^{S} für eine Einfachbindung oder für ein aromatisches oder heteroaromatisches Ringsystem mit 5 bis 30 aromatischen Ringatomen steht, das durch einen oder mehrere Reste R substituiert sein kann; und
Ar² für Phenyl, Biphenyl, Terphenyl, Quaterphenyl, Fluoren, Spirobifluoren, Naphthalin, Anthracen, Phenanthren, Triphenylen, Fluoranthen, Indol, Benzofuran, Benzothiophen, Dibenzofuran, Dibenzothiophen, Carbazol, Indenocarbazol, Indolocarbazol, Phenanthrolin, Pyridin, Pyrimidin, Pyrazin, Pyridazin, Triazin, Chinolon, Benzopyridin, Benzopyridazin, Benzopyrimidin, Chinazolin, Benzimidazol oder eine Kombination aus zwei oder drei dieser Gruppen, von denen jede durch einen oder mehrere Reste R substituiert sein kann .

7. Elektrolumineszenzvorrichtung nach einem oder mehreren der vorstehenden Ansprüche, **dadurch gekennzeichnet, dass** die Verbindung der Formel (1) mindestens einen Rest umfasst, der aus den Gruppen der Formel (R-2) ausgewählt ist, 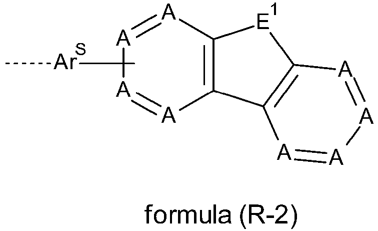 wobei die gestrichelte Bindung die Bindung an die Struktur der Formel (1) anzeigt, Ar^{S} dieselbe Bedeutung wie in Anspruch 6 hat und wobei:
A bei jedem Vorkommen gleich oder verschieden für CR^{A} oder N steht; oder zwei benachbarte Gruppen A für eine Gruppe der Formel (Ar-5) oder (Ar-6) stehen, 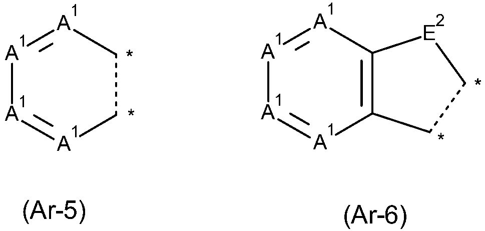
wobei die gestrichelten Bindungen zwischen den Zeichen "*" der gemeinsamen Bindung der Gruppe der Formel (Ar-5) oder (Ar-6) mit der Gruppe der Formel (R-2) entsprechen, und wobei:
E¹, E² für C(R⁰)₂, C=O, NR^{N}, O oder S stehen, wobei R⁰ und R^{N} dieselbe Bedeutung wie in Anspruch 3 haben;
A¹ steht bei jedem Vorkommen gleich oder verschieden für CR^{A1} oder N;
R^{A}, R^{A1} stehen bei jedem Vorkommen, gleich oder verschieden, für H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, von denen jede durch einen oder mehrere Reste R substituiert sein kann, wobei jeweils eine oder mehrere nicht benachbarte CH₂-Gruppen durch RC=CR, C=C, Si(R)₂, Ge(R)₂, Sn(R)₂, C=O, C=S, C=Se, P(=O)(R), SO, SO₂, O, S oder CONR ersetzt sein können und wobei ein oder mehrere H-Atome durch D, F, Cl, Br, I, CN oder NO₂ ersetzt sein können; ein aromatisches oder heteroaromatisches Ringsystem mit 5 bis 60 aromatischen Ringatomen, das jeweils durch einen oder mehrere Reste R substituiert sein kann, oder eine Aryloxygruppe mit 5 bis 60 aromatischen Ringatomen, die durch einen oder mehrere Reste R substituiert sein kann; wobei zwei benachbarte Substituenten R^{A} gemeinsam ein aliphatisches, aromatisches oder heteroaromatisches Ringsystem bilden können, das durch einen oder mehrere Reste R substituiert sein kann, und wobei zwei benachbarte Substituenten R^{A1} gemeinsam ein aliphatisches, aromatisches oder heteroaromatisches Ringsystem bilden können, das durch einen oder mehrere Reste R substituiert sein kann.

8. Organische Elektrolumineszenzvorrichtung nach einem oder mehreren der vorstehenden Ansprüche, **dadurch gekennzeichnet, dass** die Verbindung der Formel (1) als Matrixmaterial für fluoreszierende oder phosphoreszierende Emitter und/oder in einer Lochsperrschicht und/oder in einer Elektronentransportschicht und/oder in einer Elektronen- oder Exzitonensperrschicht und/oder in einer Lochtransportschicht eingesetzt wird.

9. Organische Elektrolumineszenzvorrichtung nach einem oder mehreren der vorstehenden Ansprüche, **dadurch gekennzeichnet, dass** die Verbindung der Formel (1) als Matrixmaterial für phosphoreszierende Emitter eingesetzt wird.

10. Verbindung der Formel (1'), 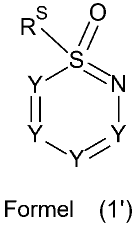 wobei für die verwendeten Symbole und Indizes Folgendes gilt:
Y steht für CR^{Y} oder N; mit der Maßgabe, dass mindestens zwei benachbarte Gruppen Y für ein kondensiertes aromatisches oder heteroaromatisches Ringsystem mit dem Benzothiazinring der Formel (1') stehen;
R^{S}, R^{Y} stehen bei jedem Vorkommen, gleich oder verschieden, für H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, von denen jede durch einen oder mehrere Reste R substituiert sein kann, wobei jeweils eine oder mehrere nicht benachbarte CH₂-Gruppen durch RC=CR, C=C, Si(R)₂, Ge(R)₂, Sn(R)₂, C=O, C=S, C=Se, P(=O)(R), SO, SO₂, O, S oder CONR ersetzt sein können und wobei ein oder mehrere H-Atome durch D, F, Cl, Br, I, CN oder NO₂ ersetzt sein können; ein aromatisches oder heteroaromatisches Ringsystem mit 5 bis 60 aromatischen Ringatomen, das jeweils durch einen oder mehrere Reste R substituiert sein kann, oder eine Aryloxygruppe mit 5 bis 60 aromatischen Ringatomen, die durch einen oder mehrere Reste R substituiert sein kann;
- wobei der Rest R^{S} mit einem Rest R^{Y} oder mit dem benachbarten, aus zwei Gruppen Y gebildeten Ring ein aliphatisches, aromatisches oder heteroaromatisches Ringsystem bilden kann, wobei diese Gruppen durch einen oder mehrere Reste R substituiert sein können; und
- wobei zwei benachbarte Reste R^{Y} gemeinsam ein aliphatisches, aromatisches oder heteroaromatisches Ringsystem bilden können, das durch einen oder mehrere Reste R substituiert sein kann;
R steht bei jedem Vorkommen, gleich oder verschieden, für H, D, F, Cl, Br, I, CHO, CN, C(=O)Ar, P(=O)(Ar)₂, S(=O)Ar, S(=O)₂Ar, N(R')₂, N(Ar)₂, NO₂, Si(R')₃, B(OR')₂, OSO₂R', eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, von denen jede durch einen oder mehrere Reste R' substituiert sein kann, wobei jeweils eine oder mehrere nicht benachbarte CH₂-Gruppen durch R'C=CR', C=C, Si(R')₂, Ge(R')₂, Sn(R')₂, C=O, C=S, C=Se, P(=O)( R'), SO, SO₂, O, S oder CONR' ersetzt sein können und wobei ein oder mehrere H-Atome durch D, F, Cl, Br, I, CN oder NO₂ ersetzt sein können, ein aromatisches oder heteroaromatisches Ringsystem mit 5 bis 60 aromatischen Ringatomen, das jeweils durch einen oder mehrere Reste R' substituiert sein können, oder eine Aryloxygruppe mit 5 bis 60 aromatischen Ringatomen, die durch einen oder mehrere Reste R' substituiert sein kann; wobei zwei benachbarte Substituenten R gemeinsam ein aliphatisches oder aromatisches Ringsystem bilden können, das durch einen oder mehrere Reste R' substituiert sein kann;
Ar jeweils gleich oder verschieden ein aromatisches oder heteroaromatisches Ringsystem mit 5 bis 60 aromatischen Ringatomen ist, das jeweils auch durch einen oder mehrere Reste R' substituiert sein kann; und
R' steht bei jedem Vorkommen, gleich oder verschieden, für H, D, F, Cl, Br, I, CN, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen, wobei jeweils eine oder mehrere nicht benachbarte CH₂-Gruppen durch SO, SO₂, O, S ersetzt sein können und wobei ein oder mehrere H-Atome durch D, F, Cl, Br oder I ersetzt sein können, oder ein aromatisches oder heteroaromatisches Ringsystem mit 5 bis 24 aromatischen Ringatomen;
**dadurch gekennzeichnet, dass** die Verbindungen der Formel (1') mindestens einen Rest enthalten, der für eine Gruppe der Formel (R-2) steht, 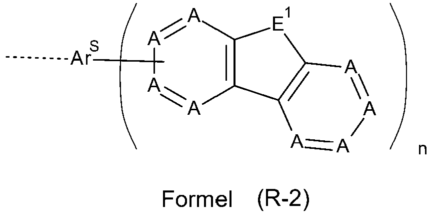
wobei die gestrichelte Bindung die Bindung an die Struktur anzeigt und wobei:
Ar^{S} für eine Einfachbindung oder für ein aromatisches oder heteroaromatisches Ringsystem mit 5 bis 30 aromatischen Ringatomen steht, das durch einen oder mehrere Reste R substituiert sein kann;
A steht, bei jedem Vorkommen gleich oder verschieden, für CR^{A} oder N; oder zwei benachbarte Gruppen A stehen für eine Gruppe der Formel (Ar-5) oder (Ar-6), 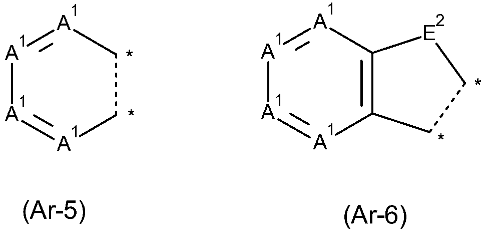
wobei die gestrichelten Bindungen zwischen den Zeichen "*" der gemeinsamen Bindung der Gruppe der Formel (Ar-5) oder (Ar-6) mit der Gruppe der Formel (R-2) entsprechen, und wobei:
E¹, E² für C(R⁰)₂ stehen, wobei C = O, NR^{N}, O oder S ist;
A¹ steht, bei jedem Vorkommen gleich oder verschieden, für CR^{A1} oder N;
R⁰, R^{N} stehen bei jedem Vorkommen, gleich oder verschieden, für H, D, F, Cl, Br, I, CN, Si(R)₃, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, von denen jede durch einen oder mehrere Reste R substituiert sein kann, wobei jeweils eine oder mehrere nicht benachbarte CH₂-Gruppen durch RC=CR, C=C, Si(R)₂, Ge(R)₂, Sn(R)₂, C=O, C=S, C=Se, P(=O)(R), SO, SO₂, O, S oder CONR ersetzt sein können und wobei ein oder mehrere H-Atome durch D, F, Cl, Br, I, CN oder NO₂ ersetzt sein können, ein aromatisches oder heteroaromatisches Ringsystem mit 5 bis 60 aromatischen Ringatomen, das jeweils durch einen oder mehrere Reste R substituiert sein kann, oder eine Aryloxygruppe mit 5 bis 60 aromatischen Ringatomen, die durch einen oder mehrere Reste R substituiert sein können, wobei zwei benachbarte Substituenten R⁰ gemeinsam ein aliphatisches, aromatisches oder heteroaromatisches Ringsystem bilden können, das durch einen oder mehrere Reste R substituiert sein kann;
R^{A}, R^{A1} stehen bei jedem Vorkommen gleich oder verschieden für H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, von denen jede durch einen oder mehrere Reste R substituiert sein kann, wobei jeweils eine oder mehrere nicht benachbarte CH₂-Gruppen durch RC=CR, C=C, Si(R)₂, Ge(R)₂, Sn(R)₂, C=O, C=S, C=Se, P(=O)(R), SO, SO₂, O, S oder CONR ersetzt sein können und wobei ein oder mehrere H-Atome durch D, F, Cl, Br, I, CN oder NO₂ ersetzt sein können; ein aromatisches oder heteroaromatisches Ringsystem mit 5 bis 60 aromatischen Ringatomen, das jeweils durch einen oder mehrere Reste R substituiert sein kann, oder eine Aryloxygruppe mit 5 bis 60 aromatischen Ringatomen, die durch einen oder mehrere Reste R substituiert sein kann; wobei zwei benachbarte Substituenten R^{A} gemeinsam ein aliphatisches, aromatisches oder heteroaromatisches Ringsystem bilden können, das durch einen oder mehrere Reste R substituiert sein kann; und wobei zwei benachbarte Substituenten R^{A1} gemeinsam ein aliphatisches, aromatisches oder heteroaromatisches Ringsystem bilden können, das durch einen oder mehrere Reste R substituiert sein kann; und
n eine ganze Zahl gleich 1, 2, 3 oder 4 ist.

11. Verbindung nach Anspruch 10, **dadurch gekennzeichnet, dass** sie aus Verbindungen der nachstehend dargestellten Formeln (2-1') bis (4-5') ausgewählt ist: 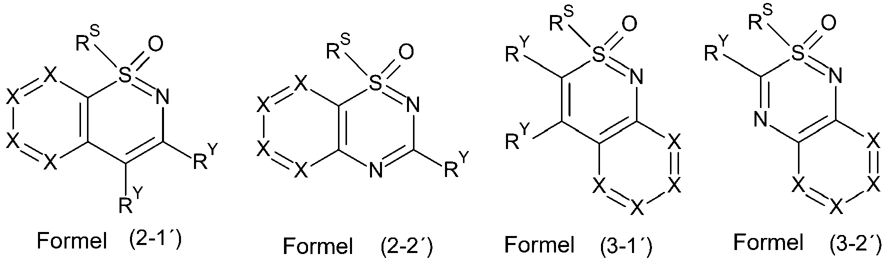 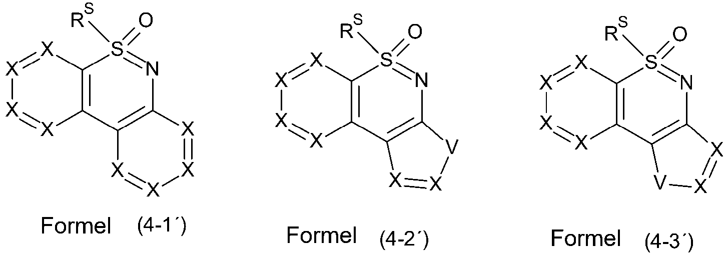 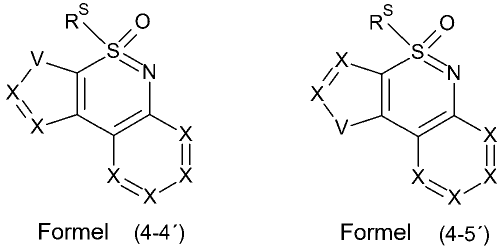 wobei die Symbole R^{S}, R^{Y} dieselbe Bedeutung haben wie in Anspruch 10, und wobei:
V für C(R⁰)₂, NR^{N}, O oder S steht;
X steht, bei jedem Vorkommen gleich oder verschieden, für CR^{X} oder N; oder zwei benachbarte Gruppen X stehen für eine Gruppe der Formel (Ar-3) oder (Ar-4), 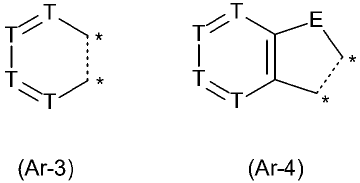
wobei die gestrichelten Bindungen zwischen den Zeichen "*" der gemeinsamen Bindung der Gruppe der Formel (Ar-3) oder (Ar-4) mit dem Rest der Struktur entsprechen, und ferner:
- E steht für C(R⁰)₂, C=O, NR^{N}, O oder S; wobei R^{N} und R⁰ dieselbe Bedeutung wie in Anspruch 10 haben;
- T steht bei jedem Vorkommen gleich oder verschieden für CR^{T} oder N;
- R^{T} und R^{X} stehen bei jedem Vorkommen, gleich oder verschieden, für H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, die jeweils durch einen oder mehrere Reste R substituiert sein können, wobei jeweils eine oder mehrere nicht benachbarte CH₂-Gruppen durch RC=CR, C=C, Si(R)₂, Ge(R)₂, Sn(R)₂, C=O, C=S, C=Se, P(=O)(R), SO, SO₂, O, S oder CONR ersetzt sein können und wobei ein oder mehrere H-Atome durch D, F, Cl, Br, I, CN oder NO₂ ersetzt sein können; ein aromatisches oder heteroaromatisches Ringsystem mit 5 bis 60 aromatischen Ringatomen, das jeweils durch einen oder mehrere Reste R substituiert sein kann, oder eine Aryloxygruppe mit 5 bis 60 aromatischen Ringatomen, die durch einen oder mehrere Reste R substituiert sein kann; wobei zwei benachbarte Substituenten R^{T} gemeinsam ein aliphatisches, aromatisches oder heteroaromatisches Ringsystem bilden können, das durch einen oder mehrere Reste R substituiert sein kann, und wobei zwei benachbarte Substituenten R^{X} gemeinsam ein aliphatisches, aromatisches oder heteroaromatisches Ringsystem bilden können, das durch einen oder mehrere Reste R substituiert sein kann;
**dadurch gekennzeichnet, dass** die Verbindungen der Formeln (2-1') bis (4-5') mindestens einen Rest R^{S}, R^{X}, R^{Y}, R^{T} oder R enthalten, der für eine Gruppe der Formel (R-2) gemäß der Definition in Anspruch 10 steht.

12. Formulierung, die mindestens eine Verbindung gemäß Anspruch 10 oder 11 und mindestens ein Lösungsmittel enthält.

13. Elektronisches Bauelement, das mindestens eine Verbindung gemäß Anspruch 10 oder 11 umfasst.

14. Elektronisches Bauelement gemäß Anspruch 13, **dadurch gekennzeichnet, dass** es ausgewählt ist aus der Gruppe bestehend aus organischen Elektrolumineszenzbauelementen, organischen integrierten Schaltungen, organischen Feldeffekttransistoren, organischen Dünnschichttransistoren, organischen Leuchttransistoren, organischen Solarzellen, farbstoffsensibilisierten organischen Solarzellen, organischen optischen Detektoren, organischen Fotorezeptoren, organischen Feldlöschbauelementen, lichtemittierenden elektrochemischen Zellen, organischen Laserdioden und organischen Plasmon-emittierenden Bauelementen.

15. Elektronisches Bauelement nach Anspruch 13 oder 14, bei dem es sich um ein organisches Elektrolumineszenzbauelement handelt, **dadurch gekennzeichnet, dass** die Verbindung gemäß nach Anspruch 10 oder 11 als Matrixmaterial für Emitter, als Lochtransportmaterial oder als Elektronentransportmaterial eingesetzt wird.

## Revendications

1. Dispositif électroluminescent organique comprenant une anode, une cathode et au moins une couche organique entre la cathode et l'anode, **caractérisé en ce que** ladite au moins une couche organique comprend un composé de formule (1), 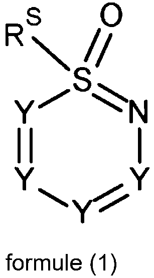
où les symboles utilisés ont les significations suivantes :
Y représente CR^{Y} ou N ; étant entendu qu'au moins deux groupes Y adjacents forment un système de cycle aromatique ou hétéroaromatique condensé avec le cycle benzothiazine de formule (1) ;
R^{S}, R^{Y} représentent à chaque occurrence, de manière identique ou différente, H, D, F, CI, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, un groupe alkyle, alcoxy ou thioalkyle à chaîne droite ayant 1 à 40 atomes de C ou un groupe alkyle, alcoxy ou thioalkyle ramifié ou cyclique ayant 3 à 40 atomes de C, chacun d'entre eux pouvant être substitué par un ou plusieurs radicaux R, où dans chaque cas un ou plusieurs groupes CH2 non adjacents peuvent être remplacés par RC=CR, C≡C, Si(R)₂, Ge(R)₂, Sn(R)₂, C=O, C=S, C=Se, P(=O)(R), SO, SO₂, O, S ou CONR et où un ou plusieurs atomes d'H peuvent être remplacés par D, F, Cl, Br, I, CN ou NO₂, un système de cycles aromatiques ou hétéroaromatiques ayant 5 à 60 atomes de cycles aromatiques, qui peut dans chaque cas être substitué par un ou plusieurs radicaux R, ou un groupe aryloxy ayant 5 à 60 atomes de cycles aromatiques, qui peut être substitué par un ou plusieurs radicaux R ;
où le radical R^{s} peut former un système de cycle aliphatique, aromatique ou hétéroaromatique avec un radical R^{y} ou avec le cycle adjacent formé par deux groupes Y, qui peut être substitué par un ou plusieurs radicaux R ; et où deux radicaux R^{y} adjacents peuvent former ensemble un système de cycle aliphatique, aromatique ou hétéroaromatique, qui peut être substitué par un ou plusieurs radicaux R ;
R représente à chaque occurrence, de manière identique ou différente, H, D, F, CI, Br, I, CHO, CN, C(=O)Ar, P(=O)(Ar)₂, S(=O)Ar, S(=O)₂Ar, N(R')₂, N(Ar)₂, NO₂, Si(R')₃, B(OR')₂, OSO₂R', un groupe alkyle, alcoxy ou thioalkyle à chaîne linéaire ayant 1 à 40 atomes de C ou un groupe alkyle, alcoxy ou thioalkyle ramifié ou cyclique ayant 3 à 40 atomes de C, chacun d'eux pouvant être substitué par un ou plusieurs radicaux R', où dans chaque cas un ou plusieurs groupes CH₂ non adjacents peuvent être remplacés par R'C=CR', C=C, Si(R')₂, Ge(R')₂, Sn(R')₂, C=O, C=S, C=Se, P(=O)(R'), SO, SO₂, O, S ou CONR' et où un ou plusieurs atomes d'H peuvent être remplacés par D, F, CI, Br, I, CN ou NO₂, un système de cycles aromatiques ou hétéroaromatiques ayant 5 à 60 atomes de cycle aromatiques, qui peut dans chaque cas être substitué par un ou plusieurs radicaux R', ou un groupe aryloxy ayant 5 à 60 atomes de cycle aromatiques, qui peut être substitué par un ou plusieurs radicaux R' ; où deux substituants R adjacents peuvent former ensemble un système de cycles aliphatiques ou aromatiques, qui peut être substitué par un ou plusieurs radicaux R' ;
Ar représente, à chaque occurrence, de manière identique ou différente, un système de cycle aromatique ou hétéroaromatique ayant 5 à 60 atomes de cycle aromatiques, qui peut dans chaque cas également être substitué par un ou plusieurs radicaux R' ;
R' représente à chaque occurrence, de manière identique ou différente, H, D, F, Cl, Br, I, CN, un groupe alkyle, alcoxy ou thioalkyle à chaîne droite ayant 1 à 20 atomes de C ou un groupe alkyle, alcoxy ou thioalkyle ramifié ou cyclique ayant 3 à 20 atomes de C, où dans chaque cas un ou plusieurs groupes CH₂ non adjacents peuvent être remplacés par SO, SO₂, O, S et où un ou plusieurs atomes d'H peuvent être remplacés par D, F, Cl, Br ou I, ou un système de cycle aromatique ou hétéroaromatique ayant 5 à 24 atomes de cycles aromatiques.

2. Dispositif électroluminescent organique selon la revendication 1, **caractérisé en ce que** le composé de formule (1) est choisi parmi les composés de formules (2), (3) ou (4) ; 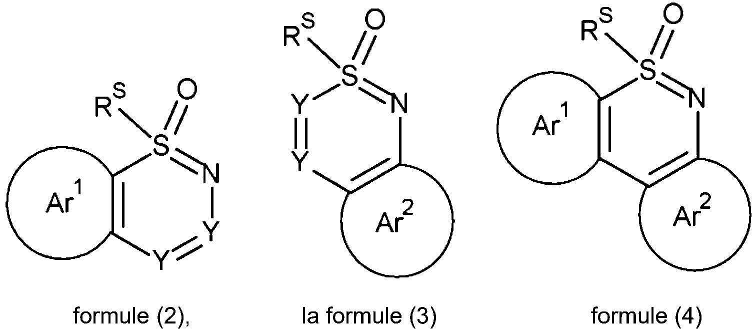 où les cycles Ar¹ et Ar² sont des systèmes de cycles aromatiques ou hétéroaromatiques condensés avec le cycle benzothiazine, qui possèdent de 5 à 30 atomes de cycle aromatique et qui peuvent être substitués par au moins un radical R ; et où les symboles Y et R^{S} ont la même signification que dans la revendication 1.

3. Dispositif électroluminescent organique selon la revendication 2, **caractérisé en ce que** les cycles Ar¹ et Ar² sont choisis parmi les cycles de formules (Ar-1) et (Ar-2), 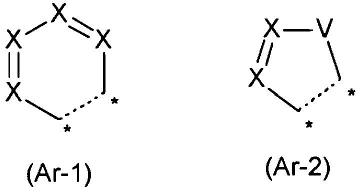 où les liaisons en pointillés entre les signes ^{« * »} correspondent à la liaison commune du groupe de formule (Ar-1) ou (Ar-2) avec le cycle benzothiazine, et où :
V représente C(R⁰)₂, NR^{N}, O ou S ;
X représente, de manière identique ou différente à chaque occurrence, CR^{x} ou N ; ou deux groupes X adjacents représentent un groupe de formule (Ar-3) ou (Ar-4), 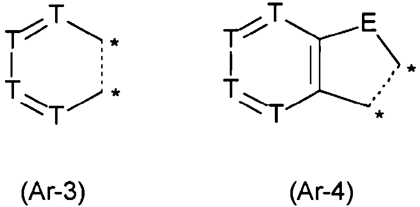
où les liaisons en pointillés entre les signes ^{« * »} correspondent à la liaison commune du groupe de formule (Ar-3) ou (Ar-4) avec un groupe de formule (Ar-1) ou (Ar-2), et en outre :
E représente C(R⁰)₂, C=O, NR^{N}, O ou S ; et
T représente, de manière identique ou différente à chaque occurrence, CR^{T} ou N ;
R^{T}, R^{X} représentent à chaque occurrence, de manière identique ou différente, H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, un groupe alkyle, alcoxy ou thioalkyle à chaîne droite ayant 1 à 40 atomes de C ou un groupe alkyle, alcoxy ou thioalkyle ramifié ou cyclique ayant 3 à 40 atomes de C, dont chacun peut être substitué par un ou plusieurs radicaux R, où dans chaque cas un ou plusieurs groupes CH₂ non adjacents peuvent être remplacés par RC=CR, C=C, Si(R)₂, Ge(R)₂, Sn(R)₂, C=O, C=S, C=Se, P(=O)(R), SO, SO₂, O, S ou CONR et où un ou plusieurs atomes d'H peuvent être remplacés par D, F, Cl, Br, I, CN ou NO₂, un système de cycles aromatiques ou hétéroaromatiques ayant 5 à 60 atomes de cycle aromatique, qui peut dans chaque cas être substitué par un ou plusieurs radicaux R, ou un groupe aryloxy ayant 5 à 60 atomes de cycle aromatique, qui peut être substitué par un ou plusieurs radicaux R ; où deux substituants adjacents R^{T} peuvent former ensemble un système de cycles aliphatique, aromatique ou hétéroaromatique, qui peut être substitué par un ou plusieurs radicaux R, et où deux substituants adjacents R^{X} peuvent former ensemble un système de cycles aliphatique, aromatique ou hétéroaromatique, qui peut être substitué par un ou plusieurs radicaux R ;
R^{o} représente à chaque occurrence, de manière identique ou différente, H, D, F, Cl, Br, I, CN, un groupe alkyle, alcoxy ou thioalkyle à chaîne droite ayant 1 à 40 atomes de C ou un groupe alkyle, alcoxy ou thioalkyle ramifié ou cyclique ayant 3 à 40 atomes de C, chacun d'eux pouvant être substitué par un ou plusieurs radicaux R, où dans chaque cas un ou plusieurs groupes CH₂ non adjacents peuvent être remplacés par RC=CR, C≡C, Si(R)₂, Ge(R)₂, Sn(R)₂, C=O, C=S, C=Se, P(=O)(R), SO, SO₂, O, S ou CONR et où un ou plusieurs atomes de H peuvent être remplacés par D, F, Cl, Br, I, CN ou NO₂, un système de cycles aromatiques ou hétéroaromatiques ayant 5 à 60 atomes de cycle aromatiques, qui peut dans chaque cas être substitué par un ou plusieurs radicaux R, ou un groupe aryloxy ayant 5 à 60 atomes de cycle aromatiques, qui peut être substitué par un ou plusieurs radicaux R, où deux substituants R^{o} adjacents peuvent former ensemble un système de cycles aliphatique, aromatique ou hétéroaromatique, qui peut être substitué par un ou plusieurs radicaux R ; et
R^{N} représente à chaque occurrence, de manière identique ou différente, H, D, F, Cl, Br, I, CN, Si(R)₃, un groupe alkyle, alcoxy ou thioalkyle à chaîne droite ayant 1 à 40 atomes de C ou un groupe alkyle, alcoxy ou thioalkyle ramifié ou cyclique ayant 3 à 40 atomes de C, chacun pouvant être substitué par un ou plusieurs radicaux R, où dans chaque cas un ou plusieurs groupes CH₂ non adjacents peuvent être remplacés par RC=CR, C=C, Si(R)₂, Ge(R)₂, Sn(R)₂, C=O, C=S, C=Se, P(=O)(R), SO, SO₂, O, S ou CONR et où un ou plusieurs atomes d'H peuvent être remplacés par D, F, CI, Br, I, CN ou NO₂, un système de cycle aromatique ou hétéroaromatique ayant 5 à 60 atomes de cycles aromatiques, qui peut dans chaque cas être substitué par un ou plusieurs radicaux R, ou un groupe aryloxy ayant 5 à 60 atomes de cycles aromatiques, qui peut être substitué par un ou plusieurs radicaux R.

4. Dispositif électroluminescent organique selon une ou plusieurs des revendications précédentes, **caractérisé en ce que** le composé de formule (1) est choisi parmi les composés de formules (2-1) à (4-5) tels que représentés ci-dessous : 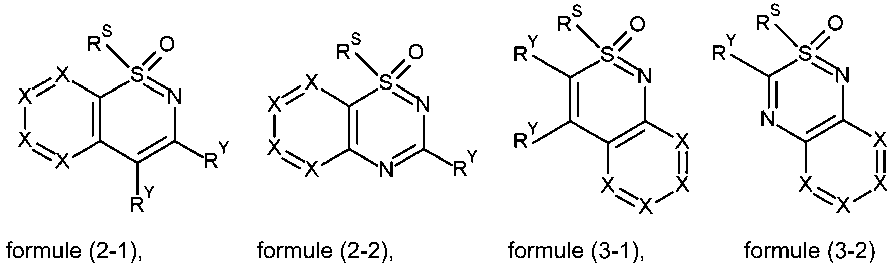 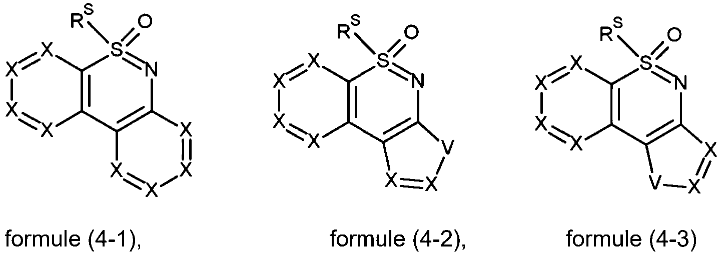 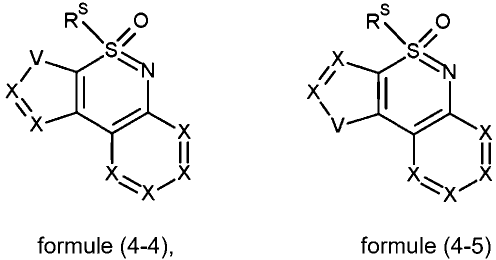 où les symboles X, R^{S}, R^{Y} ont la même signification que dans la revendication 1 et le symbole V a la même signification que dans la revendication 3.

5. Dispositif électroluminescent organique selon une ou plusieurs des revendications précédentes, **caractérisé en ce que** le composé de formule (1) est choisi parmi les composés de formules (2-1-1) à (4-5-3) tels que représentés ci-dessous : 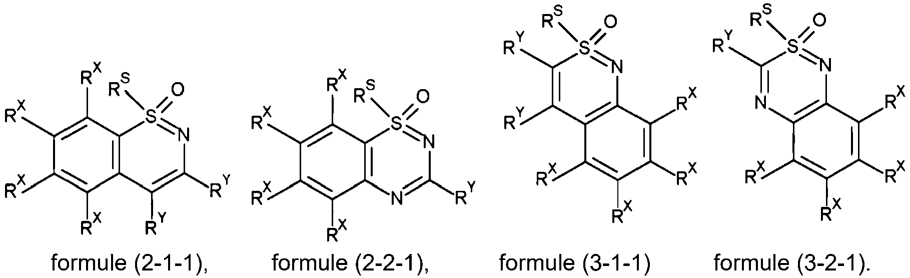 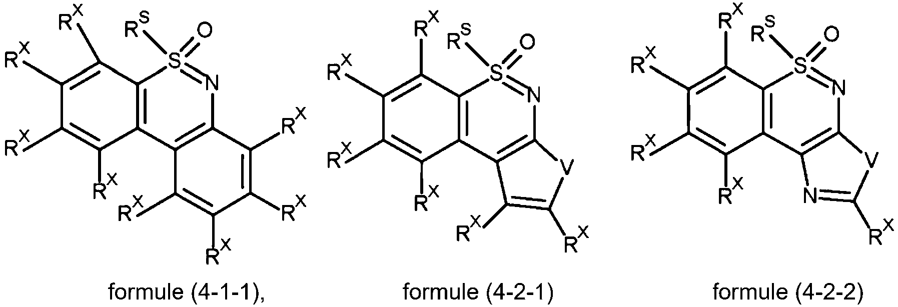 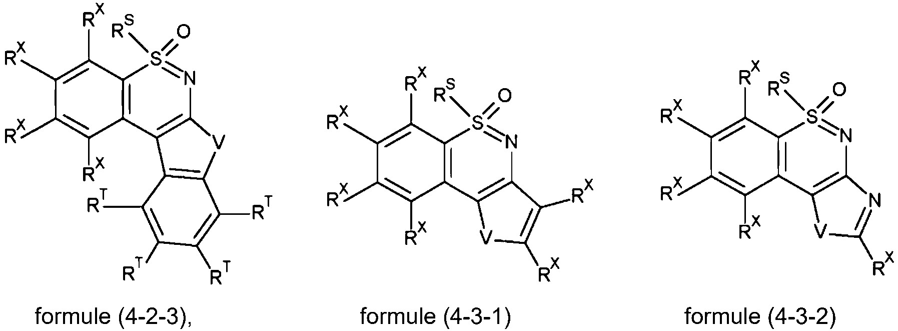 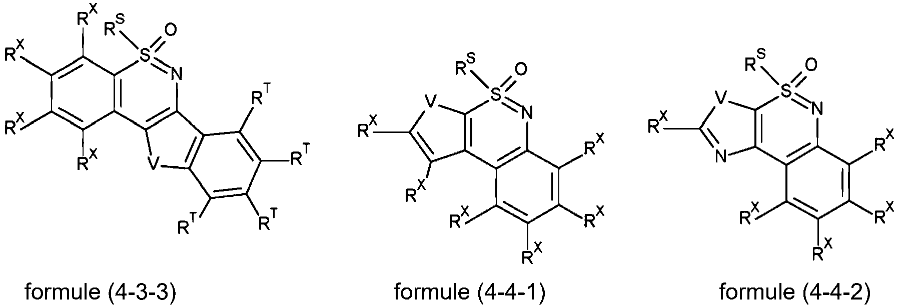 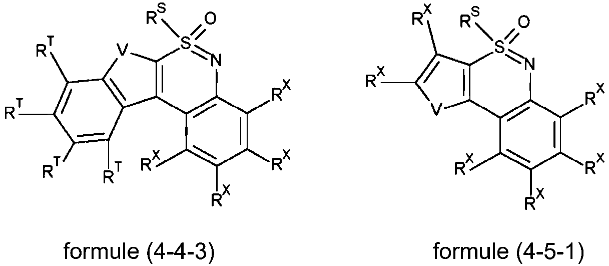 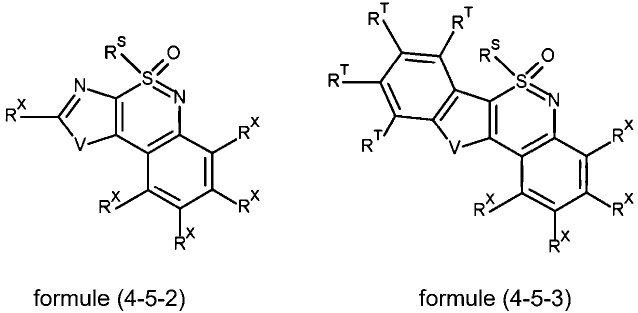 où les symboles R^{Y} et R^{S} ont la même signification que dans la revendication 1 et les symboles R^{X}, R^{T} et V ont la même signification que dans la revendication 3.

6. Dispositif électroluminescent organique selon une ou plusieurs des revendications précédentes, **caractérisé en ce que** le composé de formule (1) comprend au moins un radical choisi parmi les groupes de formule (R-1), 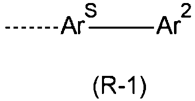 où
Ar^{S} représente une liaison simple ou un système de cycle aromatique ou hétéroaromatique ayant 5 à 30 atomes de cycle aromatique, qui peut être substitué par un ou plusieurs radicaux R ; et
Ar² représente un phényle, un biphényle, un terphényle, un quaterphényle, un fluorène, un spirobi-fluorène, un naphtalène, un anthracène,un phénanthrène, un triphénylène, un fluoranthène, un indole, un benzofurane, un benzothiophène, un dibenzofurane, un dibenzo-thiophène, un carbazole, un indénocarbazole, un indolocarbazole, une phénanthroline, une pyridine, une pyrimidine, une pyrazine, une pyridazine, une triazine, une quinolone, une benzopyridine, une benzopyridazine, une benzopyrimidine, une quinazoline, un benzimidazole, ou une combinaison de deux ou trois de ces groupes, chacun d'entre eux pouvant être substitué par un ou plusieurs radicaux R.

7. Dispositif électroluminescent selon une ou plusieurs des revendications précédentes, **caractérisé en ce que** le composé de formule (1) comprend au moins un radical choisi parmi les groupes de formule (R-2), 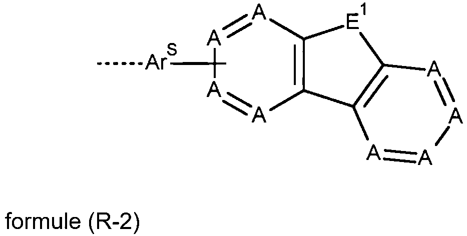 où la liaison en pointillé indique la liaison à la structure de formule (1), Ar^{S} a la même définition que dans la revendication 6 et où :
A représente, de manière identique ou différente à chaque occurrence, CR^{A} ou N ; ou deux groupes A adjacents représentent un groupe de formule (Ar-5) ou (Ar-6), 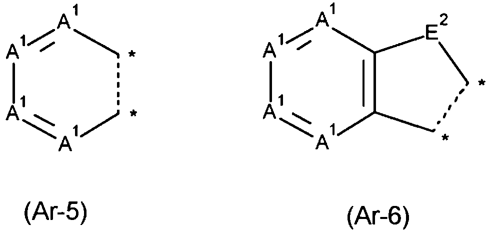
où les liaisons en pointillés entre les signes ^{« * »} correspondent à la liaison commune du groupe de formule (Ar-5) ou (Ar-6) avec le groupe de formule (R-2), et où :
E¹, E² représente C(R⁰)₂, C=O, NR^{N}, O ou S, où R⁰ et R^{N} ont la même définition que dans la revendication 3 ;
A¹ représente, de manière identique ou différente à chaque occurrence, CR^{A1} ou N ;
R^{A}, R^{A1} représentent à chaque occurrence, de manière identique ou différente, H, D, F, CI, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, un groupe alkyle, alcoxy ou thioalkyle à chaîne linéaire ayant 1 à 40 atomes de C ou un groupe alkyle, alcoxy ou thioalkyle ramifié ou cyclique ayant 3 à 40 atomes de C, chacun pouvant être substitué par un ou plusieurs radicaux R, où dans chaque cas un ou plusieurs groupes CH₂ non adjacents peuvent être remplacés par RC=CR, C≡C, Si(R)₂, Ge(R)₂, Sn(R)₂, C=O, C=S, C=Se, P(=O)(R), SO, SO₂, O, S ou CONR et où un ou plusieurs atomes d'H peuvent être remplacés par D, F, CI, Br, I, CN ou NO₂, un système de cycle aromatique ou hétéroaromatique ayant 5 à 60 atomes de cycles aromatiques, qui peut dans chaque cas être substitué par un ou plusieurs radicaux R, ou un groupe aryloxy ayant 5 à 60 atomes de cycles aromatiques, qui peut être substitué par un ou plusieurs radicaux R ; où deux substituants R^{A} adjacents peuvent former ensemble un système de cycle aliphatique, aromatique ou hétéroaromatique, qui peut être substitué par un ou plusieurs radicaux R, et où deux substituants R^{A1} adjacents peuvent former ensemble un système de cycle aliphatique, aromatique ou hétéroaromatique, qui peut être substitué par un ou plusieurs radicaux R.

8. Dispositif électroluminescent organique selon une ou plusieurs des revendications précédentes, **caractérisé en ce que** le composé de formule (1) est employé comme matériau de matrice pour émetteurs fluorescents ou phosphorescents, et/ou dans une couche de blocage de trous et/ou dans une couche de transport d'électrons et/ou dans une couche de blocage d'électrons ou de blocage d'excitons et/ou dans une couche de transport de trous.

9. Dispositif électroluminescent organique selon une ou plusieurs des revendications précédentes, **caractérisé en ce que** le composé de formule (1) est utilisé comme matériau de matrice pour des émetteurs phosphorescents.

10. Composé de formule (1'), 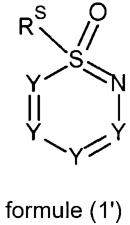 où les symboles et indices utilisés ont les significations suivantes:
Y représente CR^{Y} ou N ; étant entendu qu'au moins deux groupes Y adjacents représentent un système de cycle aromatique ou hétéroaromatique condensé avec le cycle benzothiazine de formule (1') ;
R^{S}, R^{Y} représentent à chaque occurrence, de manière identique ou différente, H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, un groupe alkyle, alcoxy ou thioalkyle à chaîne droite ayant 1 à 40 atomes de C ou un groupe alkyle, alcoxy ou thioalkyle ramifié ou cyclique ayant 3 à 40 atomes de C, dont chacun peut être substitué par un ou plusieurs radicaux R, où dans chaque cas un ou plusieurs groupes CH₂ non adjacents peuvent être remplacés par RC=CR, C=C, Si(R)₂, Ge(R)₂, Sn(R)₂, C=O, C=S, C=Se, P(=O)(R), SO, SO₂, O, S ou CONR et où un ou plusieurs atomes d'H peuvent être remplacés par D, F, Cl, Br, I, CN ou NO₂, un système de cycle aromatique ou hétéroaromatique ayant 5 à 60 atomes de cycle aromatique, qui peut dans chaque cas être substitué par un ou plusieurs radicaux R, ou un groupe aryloxy ayant 5 à 60 atomes de cycle aromatique, qui peut être substitué par un ou plusieurs radicaux R ;
- où le radical R^{S} peut former un système de cycle aliphatique, aromatique ou hétéroaromatique avec un radical R^{Y} ou avec le cycle adjacent formé par deux groupes Y, qui peut être substitué par un ou plusieurs radicaux R ; et
- où deux radicaux adjacents R^{Y} peuvent former ensemble un système de cycle aliphatique, aromatique ou hétéroaromatique, qui peut être substitué par un ou plusieurs radicaux R ;
R représente à chaque occurrence, de manière identique ou différente, H, D, F, CI, Br, I, CHO, CN, C(=O)Ar, P(=O)(Ar)₂, S(=O)Ar, S(=O)₂Ar, N(R')₂, N(Ar)₂, NO₂, Si(R')₃, B(OR')₂, OSO₂R', un groupe alkyle, alcoxy ou thioalkyle à chaîne droite ayant 1 à 40 atomes de C ou un groupe alkyle, alcoxy ou thioalkyle ramifié ou cyclique ayant 3 à 40 atomes de C, chacun d'eux pouvant être substitué par un ou plusieurs radicaux R', où dans chaque cas un ou plusieurs groupes CH₂ non adjacents peuvent être remplacés par R'C=CR', C≡C, Si(R')₂, Ge(R')₂, Sn(R')₂, C=O, C=S, C=Se, P(=O)(R'), SO, SO₂, O, S ou CONR' et où un ou plusieurs atomes d'H peuvent être remplacés par D, F, CI, Br, I, CN ou NO2, un système de cycle aromatique ou hétéroaromatique ayant 5 à 60 atomes de cycles aromatiques, qui peut dans chaque cas être substitué par un ou plusieurs radicaux R', ou un groupe aryloxy ayant 5 à 60 atomes de cycles aromatiques, qui peut être substitué par un ou plusieurs radicaux R'; où deux substituants adjacents R peuvent former ensemble un système de cycle aliphatique ou aromatique, qui peut être substitué par un ou plusieurs radicaux R' ;
Ar représente, à chaque occurrence, de manière identique ou différente, un système de cycle aromatique ou hétéroaromatique ayant 5 à 60 atomes de cycle aromatiques, qui peut dans chaque cas être également substitué par un ou plusieurs radicaux R' ; et
R' représente, à chaque occurrence, de manière identique ou différente, H, D, F, Cl, Br, I, CN, un groupe alkyle, alcoxy ou thioalkyle à chaîne linéaire ayant 1 à 20 atomes de C ou un groupe alkyle, alcoxy ou thioalkyle ramifié ou cyclique ayant 3 à 20 atomes de C, où dans chaque cas un ou plusieurs groupes CH₂ non adjacents peuvent être remplacés par SO, SO₂, O, S et où un ou plusieurs atomes de H peuvent être remplacés par D, F, Cl, Br ou I, ou un système de cycle aromatique ou hétéroaromatique ayant 5 à 24 atomes de cycle aromatique; **caractérisé en ce que** les composés de formule (1') comprennent au moins un radical qui représente un groupe de formule (R-2), 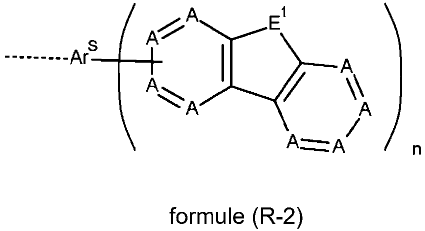
où la liaison en pointillés indique la liaison à la structure, et où :
Ar^{S} représente une liaison simple ou un système de cycles aromatiques ou hétéroaromatiques ayant 5 à 30 atomes de cycles aromatiques, qui peut être substitué par un ou plusieurs radicaux R',
A représente, de manière identique ou différente à chaque occurrence, CR^{A} ou N ; ou deux groupes A adjacents représentent un groupe de formule (Ar-5) ou (Ar-6), 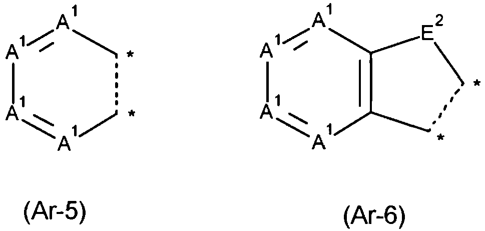
où les liaisons en pointillés entre les signes ^{« * »} correspondent à la liaison commune du groupe de formule (Ar-5) ou (Ar-6) avec le groupe de formule (R-2), et où :
E¹, E² représentent C(R⁰)₂, C=O, NR^{N}, O ou S ;
A¹ représente, de manière identique ou différente à chaque occurrence, CR^{A1} ou N ;
R^{o}, R^{N} représentent à chaque occurrence, de manière identique ou différente, H, D, F, CI, Br, I, CN, Si(R)₃, un groupe alkyle, alcoxy ou thioalkyle à chaîne droite ayant 1 à 40 atomes de C ou un groupe alkyle, alcoxy ou thioalkyle ramifié ou cyclique ayant 3 à 40 atomes de C, chacun d'entre eux pouvant être substitué par un ou plusieurs radicaux R, où dans chaque cas un ou plusieurs groupes CH₂ non adjacents peuvent être remplacés par RC=CR, C=C, Si(R)₂, Ge(R)₂, Sn(R)₂, C=O, C=S, C=Se, P(=O)(R), SO, SO₂, O, S ou CONR et où un ou plusieurs atomes d'H peuvent être remplacés par D, F, Cl, Br, I, CN ou NO₂, un système de cycle aromatique ou hétéroaromatique ayant 5 à 60 atomes de cycles aromatiques, qui peut dans chaque cas être substitué par un ou plusieurs radicaux R, ou un groupe aryloxy ayant 5 à 60 atomes de cycles aromatiques, qui peut être substitué par un ou plusieurs radicaux R, où deux substituants R^{o} adjacents peuvent former ensemble un système de cycle aliphatique, aromatique ou hétéroaromatique, qui peut être substitué par un ou plusieurs radicaux R ;
R^{A}, R^{A1} représentent à chaque occurrence, de manière identique ou différente, H, D, F, CI, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, un groupe alkyle, alcoxy ou thioalkyle à chaîne linéaire ayant 1 à 40 atomes de C ou un groupe alkyle, alcoxy ou thioalkyle ramifié ou cyclique ayant 3 à 40 atomes de C, chacun pouvant être substitué par un ou plusieurs radicaux R, où dans chaque cas un ou plusieurs groupes CH₂ non adjacents peuvent être remplacés par RC=CR, C=C, Si(R)₂, Ge(R)₂, Sn(R)₂, C=O, C=S, C=Se, P(=O)(R), SO, SO₂, O, S ou CONR et où un ou plusieurs atomes d'H peuvent être remplacés par D, F, Cl, Br, I, CN ou NO₂, des systèmes de cycles aromatiques ou hétéroaromatiques ayant 5 à 60 atomes de cycles aromatiques, qui peuvent dans chaque cas être substitués par un ou plusieurs radicaux R, ou un groupe aryloxy ayant de 5 à 60 atomes de carbone aromatiques, qui peut être substitué par un ou plusieurs radicaux R ; où deux substituants R^{A} adjacents peuvent former ensemble un système cyclique aliphatique, aromatique ou hétéroaromatique, qui peut être substitué par un ou plusieurs radicaux R ; et où deux substituants R^{A1} adjacents peuvent former ensemble un système de cycle aliphatique, aromatique ou hétéroaromatique, qui peut être substitué par un ou plusieurs radicaux R ; et
n est un nombre entier égal à 1, 2, 3 ou 4.

11. Composé selon la revendication 10, **caractérisé en ce qu'**il est choisi parmi les composés de formules (2-1') à (4-5') tels que représentés ci-dessous : 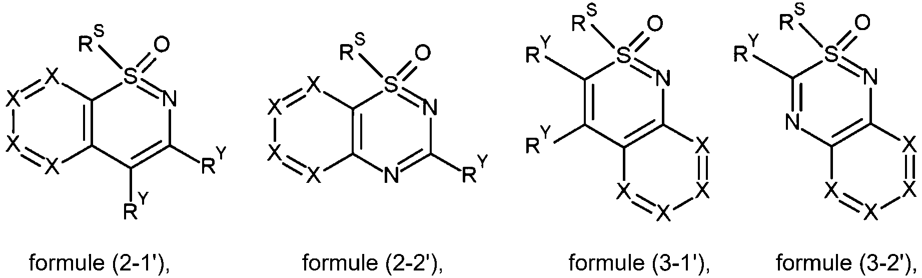 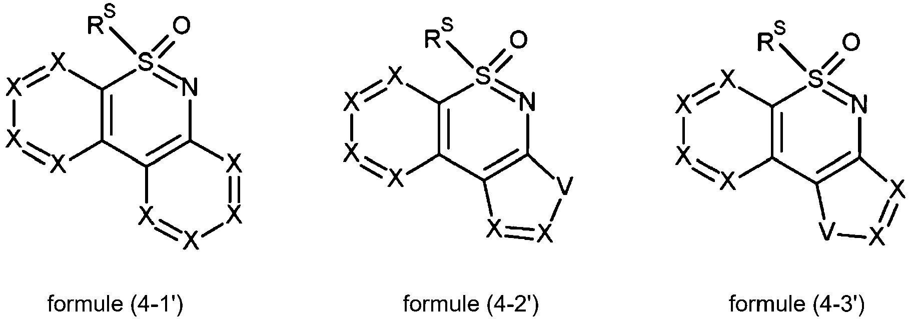 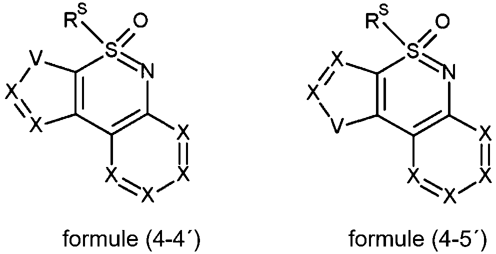 où les symboles R^{S}, R^{Y} ont la même signification que dans la revendication 10, et où :
V représente C(R⁰)₂, NR^{N}, O ou S ;
X représente, de manière identique ou différente à chaque occurrence, CR^{X} ou N ; ou deux groupes X adjacents représentent un groupe de formule (Ar-3) ou (Ar-4), 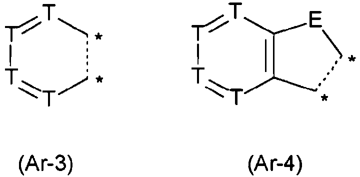
où les liaisons en pointillés entre les signes ^{«} * ^{»} correspondent à la liaison commune du groupe de formule (Ar-3) ou (Ar-4) avec le reste de la structure, et en outre :
- E représente C(R⁰)₂, C=O, NR^{N}, O ou S ; où R^{N} et R⁰ ont la même signification que dans la revendication 10 ;
- T représente, de manière identique ou différente à chaque occurrence, CR^{T} ou N ;
- R^{T}, R^{X} représentent à chaque occurrence, de manière identique ou différente, H, D, F, Cl, Br, I, CHO, CN, C(=O)Ar, P(=O)(Ar)₂, S(=O)Ar, S(=O)₂Ar, N(R)₂, N(Ar)₂, NO₂, Si(R)₃, B(OR)₂, OSO₂R, un groupe alkyle, alcoxy ou thioalkyle à chaîne droite ayant de 1 à 40 atomes de C ou un groupe alkyle, alcoxy ou thioalkyle ramifié ou cyclique ayant de 3 à 40 atomes de C, chacun d'entre eux pouvant être substitué par un ou plusieurs radicaux R, où dans chaque cas un ou plusieurs des groupes CH₂ non adjacents peuvent être remplacés par RC=CR, C=C, Si(R)₂, Ge(R)₂, Sn(R)₂, C=O, C=S, C=Se, P(=O)(R), SO, SO₂, O, S ou CONR et où un ou plusieurs atomes d'H peuvent être remplacés par D, F, Cl, Br, I, CN ou NO₂, un système de cycle aromatique ou hétéroaromatique ayant 5 à 60 atomes de cycle aromatique, qui peut dans chaque cas être substitué par un ou plusieurs radicaux R, ou un groupe aryloxy ayant 5 à 60 atomes de cycle aromatique, qui peut être substitué par un ou plusieurs radicaux R; où deux substituants adjacents R^{T} peuvent former ensemble un système de cycle aliphatique, aromatique ou hétéroaromatique, qui peut être substitué par un ou plusieurs radicaux R, et où deux substituants adjacents R^{X} peuvent former ensemble un système de cycle aliphatique, aromatique ou hétéroaromatique, qui peut être substitué par un ou plusieurs radicaux R ;
**caractérisé en ce que** les composés de formules (2-1') à (4-5') comprennent au moins un radical R^{s}, R^{x}, R^{y}, R^{T} ou R, qui représente un groupe de formule (R-2) tel que défini dans la revendication 10.

12. Formulation comprenant au moins un composé selon la revendication 10 ou 11 et au moins un solvant.

13. Dispositif électronique comprenant au moins un composé selon la revendication 10 ou 11.

14. Dispositif électronique selon la revendication 13, **caractérisé en ce qu'**il est choisi dans le groupe constitué par les dispositifs électroluminescents organiques, les circuits intégrés organiques, les transistors organiques à effet de champ, les transistors organiques en couches minces, les transistors organiques électroluminescents, les cellules solaires organiques, les cellules solaires organiques à colorant, les détecteurs optiques organiques, les photorécepteurs organiques, les dispositifs organiques à extinction de champ, les cellules électrochimiques électroluminescentes, les diodes laser organiques et les dispositifs organiques à émission de plasmons.

15. Dispositif électronique selon la revendication 13 ou 14, qui est un dispositif électroluminescent organique, **caractérisé en ce que** le composé selon la revendication 10 ou 11 est employé comme matériau matriciel pour émetteurs, un matériau de transport de trous ou un matériau de transport d'électrons.
